# EUROPEAN PATENT SPECIFICATION

(11) **EP 2 158 212 B1**
(45) Date of publication and mention of the grant of the patent: **14.05.2014**
(21) Application number: 08736917.9
(22) Date of filing: 09.04.2008
(51) Int. Cl.: C07K 14/415, C12N 15/82, A01H 5/00

(54) **RHD6 and its use in modulating plant root hair development**
RHD6 und dessen Verwendung bei der Modulierung der Entwicklung von Pflanzenwurzelhaaren
RHD6 et son utilisation pour la modulation du développement du chevelu racinaire de plantes

(30) Priority: 22.05.2007 GB 0709835
(43) Date of publication of application: 03.03.2010
(60) Divisional application: 12162135.3
(73) Proprietor: Plant Bioscience Limited, Norwich, Norfolk NR4 7UH (GB)
(72) Inventor: DOLAN, Liam, South Parks Road Oxford OX1 3RB (GB); MENAND, Benoit, 13006 Marseille (FR); YI, Keke, Hangzhou 31 0021 (CN)
(74) Representative: Fleck, Barbara
(86) International application number: PCT/GB2008/001246
(87) International publication number: WO 2008/142364

(56) References cited:
- EP-A- 0 803 572
- WO-A-2004/031349
- DATABASE EMBL [Online] 19 April 2003 (2003-04-19), SHINN P ET AL.: "Arabidopsis thaliana At1g66470 mRNA, complete cds." XP002488269 retrieved from EBI accession no. EMBL:BT006320 Database accession no. BT006320 cited in the application
- MASUCCI JAMES D ET AL: "The rhd6 Mutations of Arabidopsis thaliana Alters Root-Hair Initiation through an Auxin- and Ethylene-Associated Process" PLANT PHYSIOLOGY (ROCKVILLE), vol. 106, no. 4, 1994, pages 1335-1346, XP002488267 ISSN: 0032-0889 cited in the application
- MASUCCI J D ET AL: "HORMONES ACT DOWNSTREAM OF TTG AND GL2 TO PROMOTE ROOT HAIR OUTGROWTH DURING EPIDERMIS DEVELOPMENT IN THE ARABDOPSIS ROOT" PLANT CELL, AMERICAN SOCIETY OF PLANT PHYSIOLOGISTS, ROCKVILLE, MD, US, vol. 8, no. 9, 1 September 1996 (1996-09-01), pages 1505-1517, XP002042249 ISSN: 1040-4651 cited in the application
- MENAND BENOIT ET AL: "An ancient mechanism controls the development of cells with a rooting function in land plants" SCIENCE (WASHINGTON D C), vol. 316, no. 5830, June 2007 (2007-06), pages 1477-1480, XP002488268 ISSN: 0036-8075

## Description

### FIELD OF INVENTION

This invention relates to the modulation of root hair development in plants.

### BACKGROUND OF THE INVENTION

In 1990, Schiefelbein and Somerville²⁷ published a paper describing their work with *Arabidopsis thaliana* mutants in their efforts to understand genetic control of root hair development. They examined roots from 12,000 mutagenized *Arabidopsis* seedlings, leading to identification of more than 40 mutants impaired in root hair morphogenesis. Mutants were characterized as belonging to four phenotypic classes which genetically were produced from single nuclear recessive mutations in four different genes designated *RHD1, RHDP, RHD3,* and *RHD4.* As a result of the phenotypic analysis of the mutants and homozygous double mutants, a model for root hair development was proposed, including the stages at which the genes are normally required. The *RHD1* gene product appears to be necessary for proper initiation of root hairs, whereas the *RHDS, RHD3,* and *RHD4* gene products are required for normal hair elongation. These authors concluded that the results they obtained demonstrate that root hair development in *Arabidopsis* is amenable to genetic dissection and should prove to be a useful model system to study the molecular mechanisms governing cell differentiation in plants.

In 1994, Masucci and Schiefelbein⁷ extended those results by identifying another mutant, the *rhd6* mutant, concluding that root-hair initiation in *Arabidopsis thaliana* provides a model for studying cell polarity and its role in plant morphogenesis. They observed that root hairs normally emerge at the apical end of root epidermal cells, implying that these cells are polarized. The *rhd6* mutant was characterized as displaying three defects: (a) a reduction in the number of root hairs, (b) an overall basal shift in the site of root hair emergence, and (c) a relatively high frequency of epidermal cells with multiple root hairs. They concluded that these defects implicate the *RHD6* gene in root-hair initiation and indicate that *RHD6* is normally associated with the establishment of, or response to, root epidermal cell polarity. Similar alterations in the site of root-hair emergence, although less extreme, were also discovered in roots of the auxin-, ethylene-, abscisic acid-resistant mutant *axr2* and the ethylene-resistant mutant *etrl*. All three *rhd6* mutant phenotypes were rescued when either auxin (indoleacetic acid) or an ethylene precursor (1 -aminocyclopropane-1-carboxylic acid) was included in the growth medium. The *rhd6* root phenotypes could be phenocopied by treating wild-type seedlings with an inhibitor of the ethylene pathway (aminoethoxyvinylglycine). These results indicate that *RHD6* is normally involved in directing the selection or assembly of the root-hair initiation site through a process involving auxin and ethylene.

Root hairs play important roles in plant nutrition and water uptake. In most soils they are important for phosphate and iron uptake. In drought conditions they are important in the uptake of other nutrients such as nitrate. Therefore the manipulation of root hair traits will be important in developing crops that can effectively extract nutrients from the soil. Until now this has been difficult since no gene with a function limited to the root hair has been identified.

EP0803572B1 discloses the identification, isolation, cloning, and characterization of the *CPC* gene of *Arabidopsis thaliana,* for regulating initiation of root hair formation, as well as transgenic plants over-expressing the *CPC* gene. The *CPC* gene is not responsible for the *rhd* mutant phenotypes described above. This is confirmed, for example, in US661749, as well as EP0803572B1 itself.

### SUMMARY OF INVENTION

The present invention relates to a method for increasing the length and/or longevity of root hairs and/or the number of root hairs in a plant comprising:
increasing expression of an RHD6-related polypeptide by incorporating a heterologous nucleic acid which encodes a RHD6-related polypeptide comprising an amino acid sequence having at least 90% sequence identity to SEQ ID NO: 1
into a plant cell by means of transformation, expressing said heterologous nucleic acid within cells of said plant and regenerating the plant from one or more transformed cells wherein the length and/or longevity of root hairs and/or the number of root hairs is increased relative to control plants in which expression of the RHD6-related polypeptide is not increased. We found that the over-expression of ROOT HAIR DEFECTIVE 6 (*RHD6*) genes in plants alters root hair development, for example leading to plants with an increased number, length and/or longevity of root hairs.
Furthermore, over-expression of a different gene family (*ROOT HAIR DEFECTIVE SIX LIKE1 (RSL)* genes) produces a similar effect. Modulation of the expression of these genes (collectively termed 'RHD6-related genes') in plants may be useful, for example, in manipulating root hair traits in diverse groups of plant species (including crops) to improve their ability to extract nutrients from the soil.

We also describe an isolated ROOT HAIR DEFECTIVE 6 (RHD6)-related gene.

*RHD6*-related genes include both *ROOT HAIR DEFECTIVE 6 (RHD6)* genes and *ROOT HAIR DEFECTIVE SIX LIKE1* (*RSL1*) genes, and functional homologues thereof, as described herein. RHD6-related genes include genes capable of complementing the *rhd6* mutation in plants.

We also describe an isolated gene encoding an amino acid sequence encoded by the *RHD6*-related gene or a gene product that is sufficiently homologous thereto to permit, on production thereof in an *rhd6* mutant cell a functional complementation of said mutation. We further describe an isolated product of the expression of an isolated *RHD6*-related gene.

We further describe an isolated polynucleotide which encodes a gene product comprising an amino acid sequence shown in SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 and 114.

We further describe an isolated polynucleotide which has at least 40% nucleic acid sequence identity with one or more of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115.

We also describe expression constructs, plant cells, and plants or plant progeny, including seeds, which comprise an isolated *RHD6-*related gene or polynucleotide described herein.

We also describe a method of modulating root hair development in a plant comprising;
increasing the expression of an RHD6-related polypeptide within cells of said plant relative to control plants.

An RHD6-related polypeptide may, for example, comprise an amino acid sequence shown in SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114.

We also describe a method of improving the tolerance of a plant to nutrient-deficient conditions comprising;
increasing the expression of an RHD6-related polypeptide within cells of said plant relative to control plants.

We further describe a method of increasing the production of a root-secreted phytochemical in a plant comprising;
increasing the expression of an *RHD6*-related polypeptide within cells of a plant which produces the root-secreted phytochemical.

In some embodiments, expression of an RHD6-related polypeptide may be increased in a plant by expressing a heterologous nucleic acid encoding said RHD6-related polypeptide within cells of said plant.

In some embodiments of the disclosure, expression of an RHD6-related polypeptide may be increased in a plant by;
crossing a first and a second plant to produce a population of progeny plants;
determining the expression of the RHD6-related polypeptide in the progeny plants in the population, and
identifying a progeny plant in the population in which expression of the RHD6-related polypeptide is increased relative to controls.

In some embodiments of the disclosure, expression of an *RHD6*-related polypeptide may be increased in a plant by;
exposing a population of plants to a mutagen,
determining the expression of the *RHD6*-related polypeptide in one or more plants in said population, and;
identifying a plant with increased expression of the *RHD6-*related polypeptide.

Plants identified as having increased expression of the *RHD6*-related polypeptide may be sexually or asexually propagated or grown to produce off-spring or descendants showing increased expression of the *RHD6*-related polypeptide.

We also describe a method of producing a plant with altered root-hair development comprising:
incorporating a heterologous nucleic acid which alters the expression of a *RHD6*-related polypeptide into a plant cell by means of transformation, and;
regenerating the plant from one or more transformed cells.

Also described herein is a plant produced by a method described herein which displays altered root-hair development relative to controls.

### BRIEF DESCRIPTION OF THE DRAWINGS

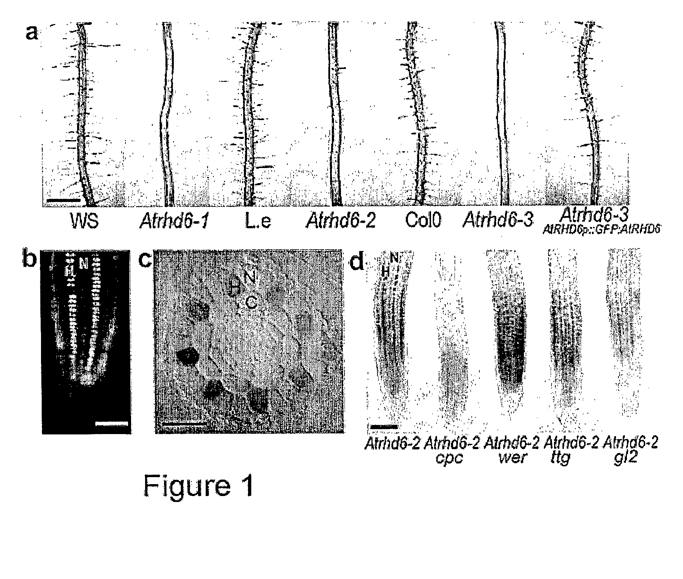

Figure 1 shows that *AtRHD6* is a positive regulator of root hairs development in Arabidopsis. Figure 1a shows roots of *Atrhd6-1, Atrhd6-2* and *Atrhd6-3* mutants with their respective wild type and complementation of the *Atrhd6-3* mutant with a genomic *AtRHD6p::GFP:AtRHD6* fusion. Figure 1b shows a fluorescent image of the genomic *AtRHD6p::GFP:AtRHD6* fusion in the *Atrhd6-3* background showing AtRHD6 protein in hair cells nuclei. Figure 1c shows the expression of the *Atrhd6-2* enhancer trap *GUS* gene in root cross section. Figure 1d shows a whole mount longitudinal view of the expression of the enhancer trap *GUS* gene in *Atrhd6-2* and in different backgrounds (*cpc, wer, ttg1* and *gl2*). H, hair cell; N, non hair cells; C, cortex. Scales bars, 500 µm (a), 50 µm (b), 25 µm (c) and 100 µm (d).

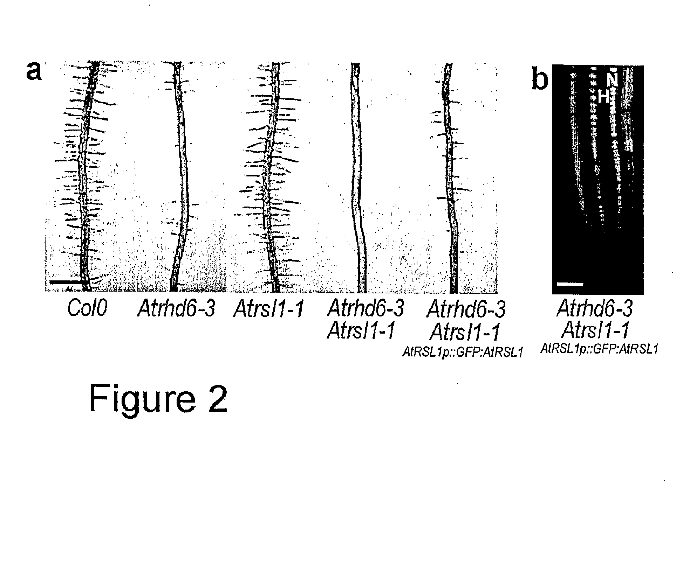

Figure 2 shows that *AtRSL1* positively regulates root hairs development in Arabidopsis. Figure 2a shows roots of WT, *Atrhd6-3* single mutant, *Atrsl1-1* single mutant, *Atrhd6-3 Atrsl1-1* double mutant and *Atrhd6-3 Atrsl1-1* double mutant bearing the *AtRSL1p::GFP:AtRSL1* transgene. Plants were grown on MS media with sucrose overlaid with a cellophane disc to increase root hairs production in the *Atrhd6-3* mutant. Figure 2b shows a fluorescent image of the genomic *AtRSL1p::GFP:AtRSL1* fusion in the *Atrhd6-3 Atrsl1-1* background showing AtRSL1 protein in hair cells nuclei. H, hair cell; N, non hair cells. Scale bars, 500 µm (a) and 50 µm (b).

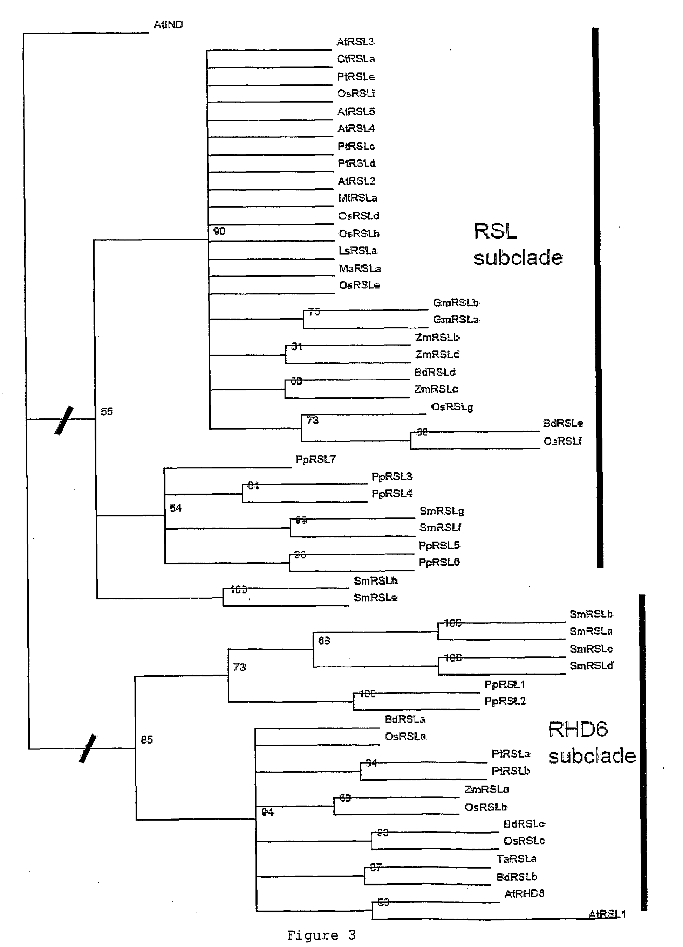

Figure 3 shows the relationship between RHD6-LIKE proteins from Arabidopsis and Physcomitrella. The tree is a strict consensus tree of 12 most parsimonious tree generated using the alignment of bHLH domains amino acids sequences shown in Tables 1 and 2. The Arabidopsis genes used are the members of bHLH subfamily VIIIc, except AtIND (INDEHISCENT)/At4g00120 which was used as out-group and belongs to the bHLH subfamily VIIIb ^{8, 10, 26}. Physcomitrella PpRSL 1 to 7 sequences were obtained by BLAST of the Physcomitrella genomic sequence. PpIND1 is a Physcomitrella sequence similar to AtIND and a putative member of family VIIIb in Physcomitrella. Numbers are bootstrap values and indicates an 82 % level of confidence for the occurrence of the AtRHD6 clade. The brackets indicates the AtRHD6 clade and the sister clade.

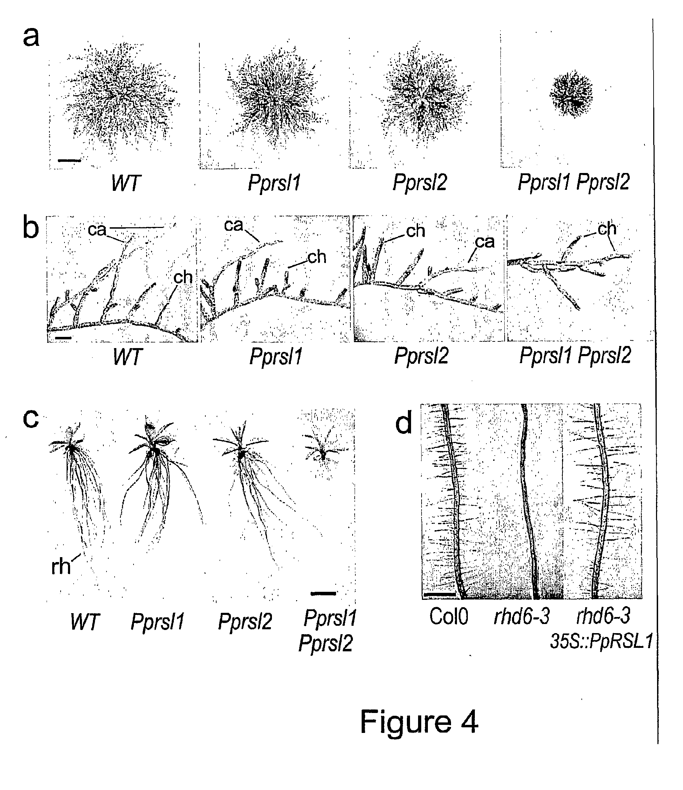

Figure 4 shows that *PpRSL1* and *PpRSL2* positively control the development of caulonemal cells and rhizoids in Physcomitrella and PpRSL1 and AtRHD6 have a conserved molecular function. Figure 4a and b show eighteen day old protonema from WT, *Pprsl1* and *Pprsl2* single mutants, and *Pprsl1 Pprsl2* double mutant, grown from spores on 0.8% agar. Figure 4a shows whole protonema growing from a single spore. Figure 4b shows dissected filaments from protonema shown in Figure 4a. Figure 4c shows isolated one month old gametophores. Figure 4d shows roots of the Arabidopsis *Atrhd6-3* mutant carrying the *35S::PpRSL1* transgene compared to WT and *Atrhd6-3* roots. ca, caulonemal cell; ch, chloronemal cell; rh, rhizoid. Scale bars, 1 mm (a), 100 µm (b), 1 mm (c), and 500 µm (d).

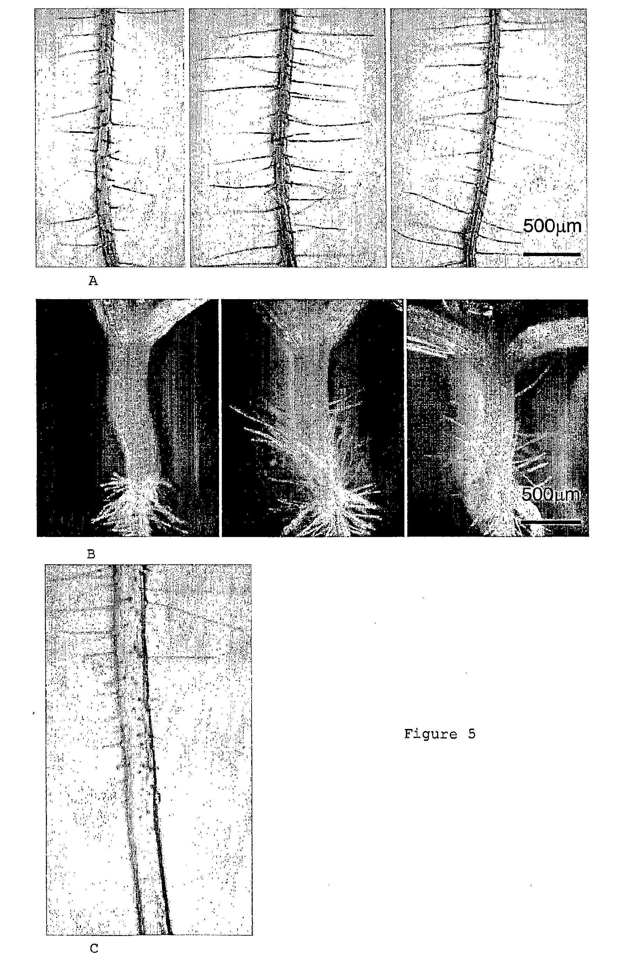

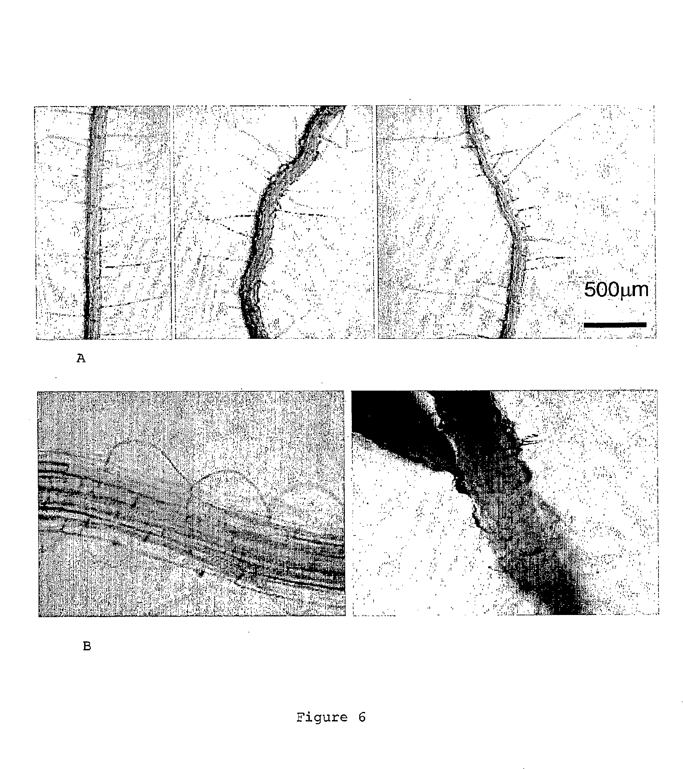

Figure 5 shows the phenotype for the transformants:35S::RHD6 Figure 5A shows col-0 *rhd6*/*rsl1* with 35S::RHD6; Figure 5B shows col-*0 rhd6*/*rsl1* with 35S::RHD6; Figure 5C. *rhd6*/*rsl1* with 35S::RHD6 Figure 6 shows the phenotype for the transformants:35S::RSL2 and 35S::RSL3 Figure 6A shows col-0 *rhd6*/*rsl1* with 35S::RSL2/3; Figure 6B shows root hypocotyls

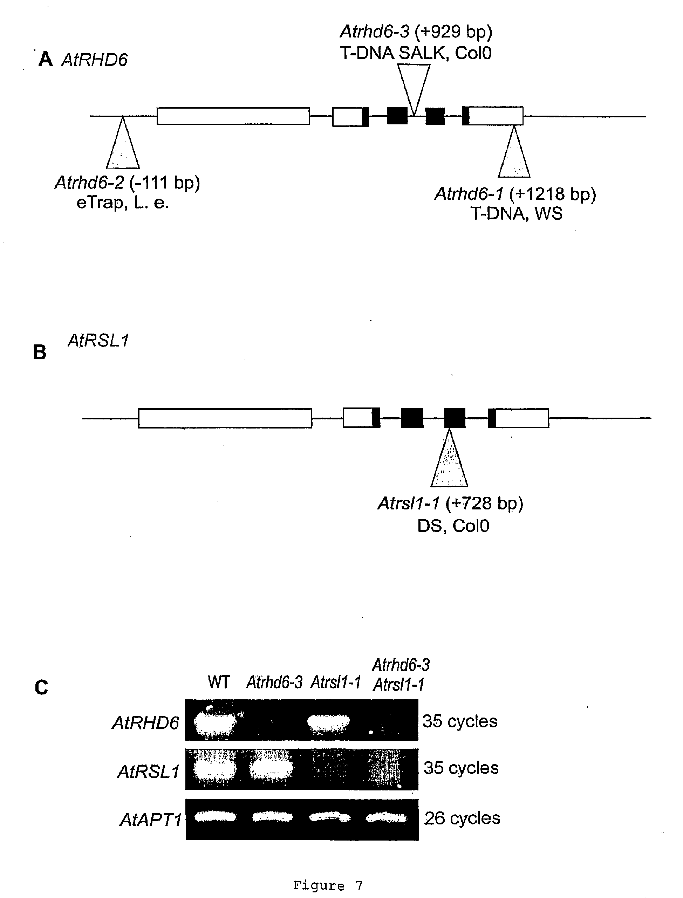

Figure 7 shows the molecular basis of mutations in A. *thaliana AtRHD6* (A) and *AtRSL1* (B) genes. White boxes correspond to coding regions (black boxes for the bHLH domain encoding region). Grey triangles indicate the position of each insertion. Numbers in brackets indicate the distance between each T-DNA insertion and the start codon. (C) RT-PCR showing that *Atrhd6-3, Atrsl1-1* and *Atrhd6-3 Atrsl1-1* are RNA null mutants. AtAPT1, Adenine phosphoribosyltransferase 1.

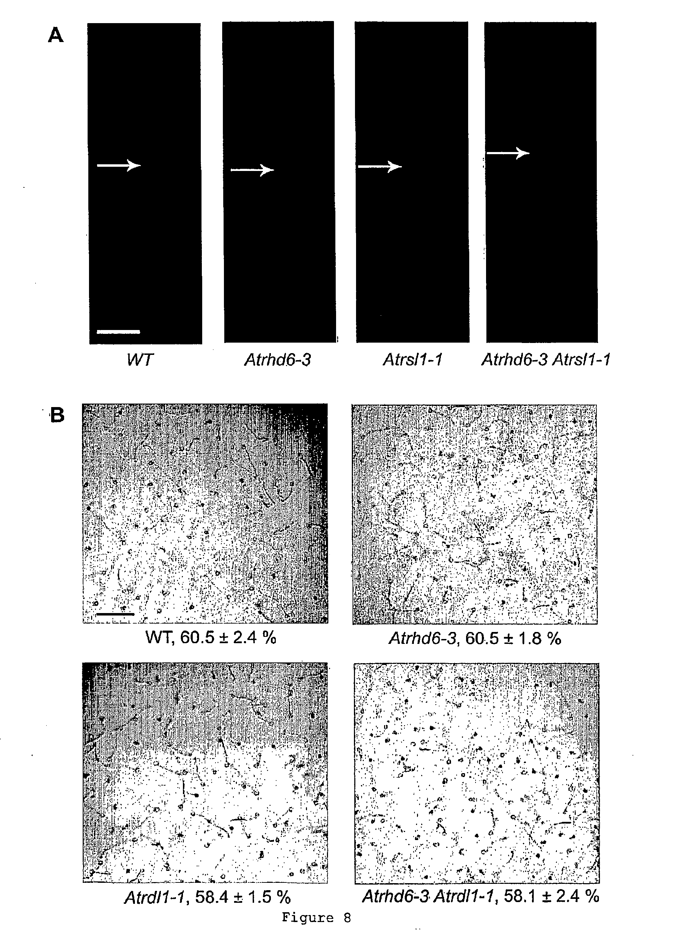

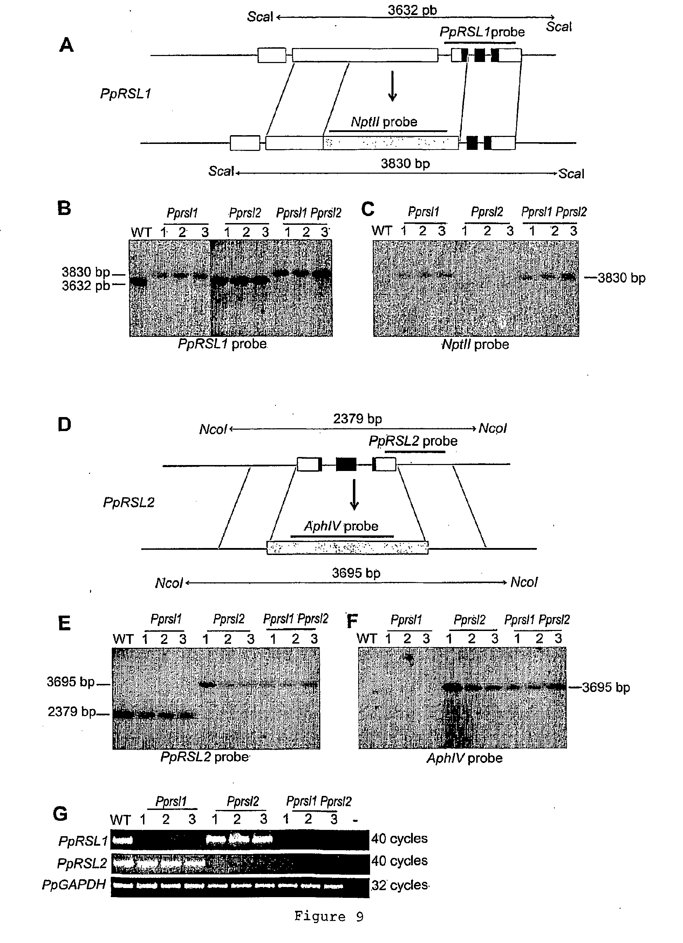

Figure 8 shows that the *Atrhd6-3* and *Atrsl1-1* single mutants and the *Atrhd6 Atrsll* double mutant have no detectable pollen tube growth defects. Ratios of resistance to antibiotic of F2 plants from *Atrhd6-3 Atrsl1-1* double mutant backcrossed to WT are 76.7 % (n=1404; χ²(3/1) = 2,19; P > 0,05) for Sulfadiazin (resistance carried by the *Atrhd6-3* allele) and 74.9 % (n=1289; χ²(3/1) = 0.0023; P > 0,05) for phosphinothricin (resistance carried by the *Atrsl1-1* allele) showing normal segregation of the single mutants and double mutant gametes. (Figure 8A shows pollen of the genotype indicated below each picture was used to pollinate WT stigma. Carpels were stained with aniline blue 4 hours after pollination. The growth of each mutant pollen tubes in the WT carpel is revealed by callose staining in blue (white arrows). Similar pollen tube growth is observed in WT and mutant pollen tubes. Figure 9B shows *in vitro* pollen tube growth experiment. WT and mutants pollens were germinated on agar plates. Representative plates are shown with the germination ratio (mean of 600 pollen grains per line, with standard error). Similar germination ratios are observed between WT and mutants pollen (Student's-t-test p values are 0.554 for *Atrhd6-3* versus WT, 0.904 for *Atrsl1-1* versus WT and 0.87 for *Atrhd6-3 Atrsl1-1* versus WT). Scale bars, 200 µm (Figure 8A and B).
Figure 9 shows the molecular basis of *P. patens Pprsl1, Pprsl2* and *Pprsl1 Pprsl2* mutations (three independent mutants, named 1 to 3, are shown in each case). Figure 9A and D show the structure of the *PpRSL1* (A) and *PpRSL2* (D) genes (up), and the expected result of the homologous recombination (down). White boxes correspond to coding regions (black boxes for the bHLH domain encoding region) and the grey boxes correspond to the resistance gene cassette (*NptII* and *AphIV*). The regions of homology used for gene replacement are delimited by grey lines. The distance between the restrictions sites used for Southern blots and the position of the probes used are also shown. Figure 9B, C, E and F show southern blots of WT and mutants DNA digested with *Sca*I (B and C) or *Nco*I (E and F) and hybridized with the probe indicated below the picture. Blots C and F are hybridization of the same membrane used for blot B and E respectively, after stripping of the gene specific probe. The replacement of the WT band by a larger band of expected size (see A and D) in mutants lines when hybridization is performed with the gene specific probe (B and E), and the hybridization of only the mutant band with the resistance gene probe (C and F), demonstrate the presence of single insertions in the *PpRSL1* and *PpRSL2* loci. (G) RT-PCR showing that the mutants are RNA null mutants. PpGAPDH, glyceraldehyde 3-phosphate dehydrogenase. In each case, the three independent single insertion mutants presented have the same phenotype and only the mutant 1 is shown in Fig. 4.

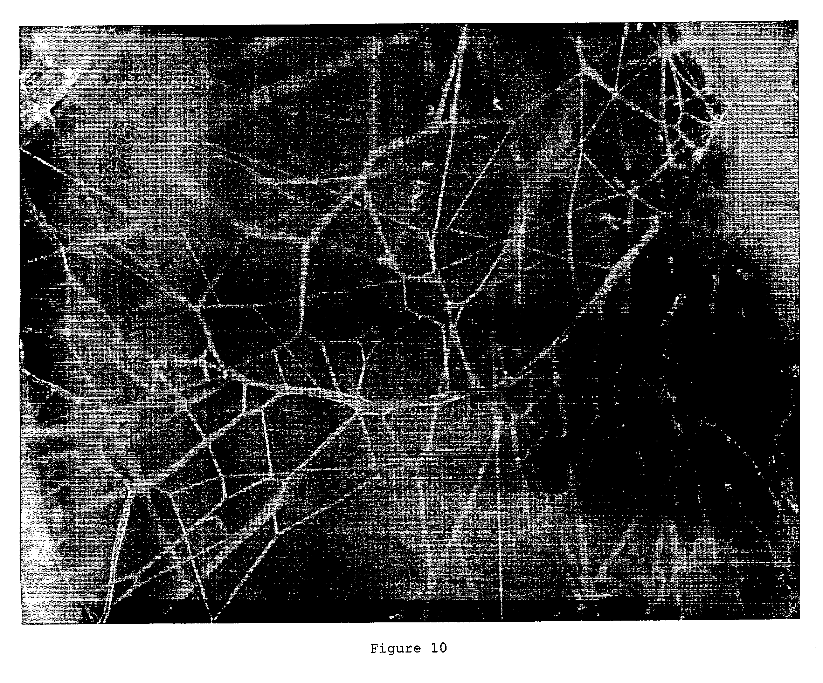

Figure 10 shows the root hair system of an Arabidopsis plant over-expressing RSL4 and displaying a root morphology resembling a fungal symbiont, such as *Mycorrhizae.*

### DETAILED DESCRIPTION OF THE PREFERRED EMBODIMENTS OF THE INVENTION

The present disclosure demonstrates the identification, isolation, cloning and expression of the *ROOT HAIR DEFECTIVE 6* (RHD6) and *ROOT HAIR DEFECTIVE SIX LIKE (RSL)* genes (collectively termed *'RHD6-*related genes' herein) in plants. It shows complementation of mutations by distantly related genes, providing the function of root hair development in plants in which the distantly related gene has been inactivated. Accordingly, those skilled in the art will appreciate that the gene responsible for previously identified mutant phenotypes has been isolated and cloned according to this invention. It will also be appreciated that from this disclosure functional benefits may be conferred on plants by means of introduction into plants and expression of these genes in such plants. Methods known in the art may be utilized for this purpose. Thus, for example, those skilled in the art will appreciate that the methods, for example, for achieving the expression of the *RHD6* and *RSL* genes described herein may be achieved according methods disclosed herein, and by methods, for example, disclosed in, but not limited to, EP0803572B1, which discloses the cloning and expression of the cpc gene, which, like the *RHD6* and *RSL* genes described herein, is also related to the control of root hair development in plants, albeit at a different stage of plant and root hair development.

We describe ROOT HAIR DEFECTIVE 6 (RHD6)-related polypeptides encoded by *ROOT HAIR DEFECTIVE 6* (RHD6)-related genes and nucleic acid sequences described herein.

ROOT HAIR *DEFECTIVE* 6 (RHD6)-related polypeptides include both ROOT HAIR DEFECTIVE 6 (RHD6) polypeptides and ROOT HAIR DEFECTIVE 6-LIKE 1 (RSL1) polypeptides, and functional homologues thereof, as described herein. RHD6-related polypeptides include may be capable of complementing the *rhd6* mutation upon expression in plants.

A ROOT HAIR DEFECTIVE 6 (RHD6)-related polypeptide may fall within the RHD6 clade comprising AtRHD6, AtRSL1, PpRSL1, PpRSL2, BdRSLb, TaRSLa, OsRSLc, BdRSLc, OsRSLb, ZmRSLa, PtRSLa, PrRSLb, OsRSLa, BdRSLa, SmRSLa, SmRSLb, SmRSLc and SmRSLd (the ROOT HAIR DEFECTIVE 6 (RHD6) clade) in a cladogram of protein sequences, for example using the sequences of AtIND and PpINDa as an outgroup (see figure 3). Alternatively, ROOT HAIR DEFECTIVE 6 (RHD6)-related polypeptide may fall within the RSL clade comprising AtRSL3, CtRSLa, PtRSLe, OsRSLi, AtRSL5, AtRSL4, PtRSLc, PtRSLd, AtRSL2, MtRSLa, OsRSLd, OsRSLh, LsRSLa, MaRSLa, OsRSLe, GmRSLb, GmRSLa, ZmRSLb, ZmRSLd, BdRSLd, ZmRSLc, OsRSLg, BdRSLe, OsRSLf, PpRSL3, PpRSL4, PpRSL5, PpRSL6, PpRSL7, SmRSLg, SmRSLf, SmRSLh and SmRSLe (the *ROOT HAIR DEFECTIVE SIX LIKE (RSL)* clade) in a cladogram of protein sequences, for example using the sequences of AtIND and PpINDa as an outgroup (see figure 3).

A cladogram may be produced using conventional techniques. For example, a cladogram may be calculated using ClustalW to align the protein sequences, Phylip format for tree output, with 1000 bootstrap replicates and TreeViewX (version 0.5.0) for visualisation.

A ROOT HAIR DEFECTIVE 6 (RHD6)-related polypeptide may comprise the amino acid sequence shown of SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114 or may be a fragment or variant of one of these sequences which retains RHD6 activity.

In some preferred embodiments of the invention, the ROOT HAIR DEFECTIVE 6 (RHD6)-RELATED polypeptide may be a ROOT HAIR DEFECTIVE 6 (RHD6) polypeptide having the amino acid sequence of SEQ ID NO:1 (At1g66470; NP_176820.1 GI: 15219658).

In other embodiments of the disclosure, the ROOT HAIR DEFECTIVE 6 (RHD6)-RELATED polypeptide may be a *ROOT HAIR DEFECTIVE SIX LIKE (RSL)* polypeptide having the amino acid sequence of any one of SEQ ID NOS: 5, 7, 9, and 11 or may be a fragment or variant of any of these sequences which retains RHD6 activity.

A ROOT HAIR DEFECTIVE 6 (RHD6)-RELATED polypeptide which is a variant a reference sequence set out herein, such as SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114, may comprise an amino acid sequence which shares greater than 20% sequence identity with the reference amino acid sequence, preferably greater than 30%, greater than 40%, greater than 50%, greater than 60%, greater than 65%, greater than 70%, greater than 80%, greater than 90% or greater than 95%.

Particular amino acid sequence variants may differ from a RHD6-related polypeptide sequence as described herein by insertion, addition, substitution or deletion of 1 amino acid, 2, 3, 4, 5-10, 10-20 20-30, 30-50, or more than 50 amino acids.

Sequence identity is commonly defined with reference to the algorithm GAP (Wisconsin Package, Accelerys, San Diego USA). GAP uses the Needleman and Wunsch algorithm to align two complete sequences that maximizes the number of matches and minimizes the number of gaps. Generally, default parameters are used, with a gap creation penalty = 12 and gap extension penalty = 4.

Use of GAP may be preferred but other algorithms may be used, e.g. BLAST (which uses the method of Altschul et al. (1990) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), or the TBLASTN program, of Altschul et al. (1990) supra, generally employing default parameters. In particular, the psi-Blast algorithm (Nucl. Acids Res. (1997) 25 3389-3402) may be used.

Sequence comparison may be made over the full-length of the relevant sequence described herein.

Certain domains of a RHD6-related polypeptide may show an increased level of identity with domains of a reference sequence, such as SEQ ID NO: 1, 3, 5, 7, 9, 11, 13 to 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 or 114, relative to the RHD6-related polypeptide sequence as a whole. For example, a RHD6-related polypeptide may comprise one or more domains or motifs consisting of an amino acid sequence which has at least 70%, at least 75%, at least 80%, at least 90%, at least 95%, or at least 98% sequence identity or similarity, with an amino acid sequence selected from the group consisting of SEQ ID NOS: 13 to 25 or other RHD6-related polypeptide domain shown in tables 1 and 2.

In some preferred embodiments of the disclosure, a RHD6-related polypeptide may comprise one or more domains or motifs consisting of an amino acid sequence which is selected from the group consisting of SEQ ID NOS: 13 to 25 or other RHD6-related polypeptide domain shown in tables 1 and 2.

We describe ROOT HAIR DEFECTIVE 6 (RHD6)-related genes and nucleic acid sequences which encode ROOT HAIR DEFECTIVE 6 (RHD6)-related polypeptides.

A nucleic acid encoding a RHD6-related polypeptide may comprise or consist of the nucleotide sequence of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115 or may be a variant or fragment of any one of these sequences which encodes a polypeptide which retains RHD6 activity.

In some preferred embodiments of the invention, a nucleic acid encoding a RHD6-related polypeptide may comprise or consist of the nucleotide sequence of SEQ ID NO: 2.

A variant sequence may be a mutant, homologue, or allele of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115 and may differ from one of these sequences by one or more of addition, insertion, deletion or substitution of one or more nucleotides in the nucleic acid, leading to the addition, insertion, deletion or substitution of one or more amino acids in the encoded polypeptide. Of course, changes to the nucleic acid that make no difference to the encoded amino acid sequence are included. A nucleic acid encoding a *RHD6*-related polypeptide, which has a nucleotide sequence which is a variant of an *RHD6*-related nucleic acid sequence set out herein may comprise a sequence having at least 30% sequence identity with the nucleic acid sequence of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115, for example, preferably greater than 40%, greater than 50%, greater than 60%, greater than 65%, greater than 70%, greater than 80%, greater than 90% or greater than 95%. Sequence identity is described above.

A fragment or variant may comprise a sequence which encodes a functional RHD6-related polypeptide i.e. a polypeptide which retains one or more functional characteristics of the polypeptide encoded by the wild-type RHD6 gene, for example, the ability to stimulate or increase root hair number, growth or longevity in a plant or to complement the *rhd6* mutation.

In other embodiments, a nucleic acid encoding a RHD6 polypeptide, which has a nucleotide sequence which is a variant of the sequence of any one of SEQ ID NOS: 2, 4, 6, 8, 10, 12, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, and 115 may selectively hybridise under stringent conditions with this nucleic acid sequence or the complement thereof.

Stringent conditions include, e.g. for hybridization of sequences that are about 80-90% identical, hybridization overnight at 42°C in 0.25M Na₂HPO₄, pH 7.2, 6.5% SDS, 10% dextran sulfate and a final wash at 55 °C in 0.1X SSC, 0.1% SDS. For detection of sequences that are greater than about 90% identical, suitable conditions include hybridization overnight at 65°C in 0.25M Na₂HPO₄, pH 7.2, 6.5% SDS, 10% dextran sulfate and a final wash at 60 °C in 0.1X SSC, 0.1% SDS.

An alternative, which may be particularly appropriate with plant nucleic acid preparations, is a solution of 5x SSPE (final 0.9 M NaCl, 0.05M sodium phosphate, 0.005M EDTA pH 7.7), 5X Denhardt's solution, 0.5% SDS, at 50°C or 65°C overnight. Washes may be performed in 0.2x SSC/0.1% SDS at 65°C or at 50-60°C in 1x SSC/0.1% SDS, as required.

Nucleic acids as described herein may be wholly or partially synthetic. In particular, they may be recombinant in that nucleic acid sequences which are not found together in nature (do not run contiguously) have been ligated or otherwise combined artificially. Alternatively, they may have been synthesised directly e.g. using an automated synthesiser.

The nucleic acid may of course be double- or single-stranded, cDNA or genomic DNA, or RNA. The nucleic acid may be wholly or partially synthetic, depending on design. Naturally, the skilled person will understand that where the nucleic acid includes RNA, reference to the sequence shown should be construed as reference to the RNA equivalent, with U substituted for T.

ROOT HAIR DEFECTIVE 6 (RHD6)-related polypeptides and nucleic acids may be readily identified by routine techniques of sequence analysis in a range of plants, including agricultural plants selected from the group consisting of *Lithospermum erythrorhizon, Taxus* spp, tobacco, cucurbits, carrot, vegetable brassica, melons, capsicums, grape vines, lettuce, strawberry, oilseed brassica, sugar beet, wheat, barley, maize, rice, soyabeans, peas, sorghum, sunflower, tomato, potato, pepper, chrysanthemum, carnation, linseed, hemp and rye.

A RHD6-related nucleic acid as described herein may be operably linked to a heterologous regulatory sequence, such as a promoter, for example a constitutive, inducible, root-specific or developmental specific promoter.

"Heterologous" indicates that the gene/sequence of nucleotides in question or a sequence regulating the gene/sequence in question, has been linked to the RHD6 related nucleic acid using genetic engineering or recombinant means, i.e. by human intervention. Regulatory sequences which are heterologous to an RHD6 related nucleic acid may be regulatory sequences which do not regulate the RHD6 related nucleic acid in nature or are not naturally associated with the RHD6 related nucleic acid. "Isolated" indicate that the isolated molecule (e.g. polypeptide or nucleic acid) exists in an environment which is distinct from the environment in which it occurs in nature. For example, an isolated nucleic acid may be substantially isolated with respect to the genomic environment in which it naturally occurs.

Many suitable regulatory sequences are known in the art and may be used in accordance with the invention. Examples of suitable regulatory sequences may be derived from a plant virus, for example the Cauliflower Mosaic Virus 35S (CaMV 35S) gene promoter that is expressed at a high level in virtually all plant tissues (Benfey et al, (1990) EMBO J 9: 1677-1684). Other suitable constitutive regulatory elements include the cauliflower mosaic virus 19S promoter; the Figwort mosaic virus promoter; and the nopaline synthase (nos) gene promoter (Singer et al., Plant Mol. Biol. 14:433 (1990); An, Plant Physiol. 81:86 (1986)). For example, RHD6-related genes such as AtRHD6, AtRSL1 and AtRSL4 may be expressed using constitutive promoters.

Constructs for expression of *RHD6* and RSL genes under the control of a strong constitutive promoter (the 35S promoter) are exemplified below. Expression of AtRHD6, AtRSL1 and AtRSL4 from the 35S promoter is shown to modulate root hair development in plants without causing additional phenotypic changes.

However, those skilled in the art will appreciate that a wide variety of other promoters may be employed to advantage in particular contexts. Thus, for example, one might select an epidermal or root-specific promoter to ensure expression of these constructs only in roots. Suitable root-specific promoters are described for example in Qi et al PNAS (2006) 103(49) 18848-18853. For example, RHD6-related genes such as AtRSL2 and ATRSL3, may be expressed using root-specific promoters.

Alternatively, or in addition, one might select an inducible promoter. In this way, for example, in a cell culture setting, production of a particular gene product of interest may be enhanced or suppressed by induction of the promoter driving expression of the genes described herein. Inducible promoters include the alcohol inducible alc gene-expression system (Roslan et al., Plant Journal; 2001 Oct; 28(2):225-35) may be employed.

*RHD6*-related nucleic acid may be contained on a nucleic acid construct or vector. The construct or vector is preferably suitable for transformation into and/or expression within a plant cell.

A vector is, *inter alia,* any plasmid, cosmid, phage or *Agrobacterium* binary vector in double or single stranded linear or circular form, which may or may not be self transmissible or mobilizable, and which can transform prokaryotic or eukaryotic host, in particular a plant host, either by integration into the cellular genome or exist extrachromasomally (e.g. autonomous replicating plasmid with an origin of replication).

Specifically included are shuttle vectors by which is meant a DNA vehicle capable, naturally or by design, of replication in two different organisms, which may be selected from actinomyces and related species, bacteria and eukaryotic (e.g. higher plant, mammalia, yeast or fungal) cells.

A construct or vector comprising nucleic acid as described above need not include a promoter or other regulatory sequence, particularly if the vector is to be used to introduce the nucleic acid into cells for recombination into the genome.

Constructs and vectors may further comprise selectable genetic markers consisting of genes that confer selectable phenotypes such as resistance to antibiotics such as kanamycin, hygromycin, phosphinotricin, chlorsulfuron, methotrexate, gentamycin, spectinomycin, imidazolinones, glyphosate and d-amino acids.

Those skilled in the art are well able to construct vectors and design protocols for recombinant gene expression, in particular in a plant cell. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 3rd edition, Sambrook & Russell, 2001, Cold Spring Harbor Laboratory Press.

Those skilled in the art can construct vectors and design protocols for recombinant gene expression, for example in a microbial or plant cell. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 3rd edition, Sambrook et al, 2001, Cold Spring Harbor Laboratory Press and Protocols in Molecular Biology, Second Edition, Ausubel et al. eds. John Wiley & Sons, 1992. Specific procedures and vectors previously used with wide success upon plants are described by Bevan, Nucl. Acids Res. (1984) 12, 8711-8721), and Guerineau and Mullineaux, (1993) Plant transformation and expression vectors. In: Plant Molecular Biology Labfax (Croy RRD ed) Oxford, BIOS Scientific Publishers, pp 121-148.

When introducing a chosen gene construct into a cell, certain considerations must be taken into account, well known to those skilled in the art. The nucleic acid to be inserted should be assembled within a construct that contains effective regulatory elements that will drive transcription. There must be available a method of transporting the construct into the cell. Once the construct is within the cell membrane, integration into the endogenous chromosomal material either will or will not occur. Finally, the target cell type is preferably such that cells can be regenerated into whole plants.

Those skilled in the art will also appreciate that in producing constructs for achieving expression of the genes according to this invention, it is desirable to use a construct and transformation method which enhances expression of the *RHD6* gene, the *RSL* gene or a functional homolog thereof. Integration of a single copy of the gene into the genome of the plant cell may be beneficial to minimize gene silencing effects. Likewise, control of the complexity of integration may be beneficial in this regard. Of particular interest in this regard is transformation of plant cells utilizing a minimal gene expression construct according to, for example, EP

Patent No. EP 1 407 000 B1.

Techniques well known to those skilled in the art may be used to introduce nucleic acid constructs and vectors into plant cells to produce transgenic plants with the properties described herein.

Agrobacterium transformation is one method widely used by those skilled in the art to transform woody plant species, in particular hardwood species such as poplar. Production of stable, fertile transgenic plants is now routine in the art:(Toriyama, et al. (1988) Bio/Technology 6, 1072-1074; Zhang, et al. (1988) Plant Cell Rep. 7, 379-384; Zhang, et al. (1988) Theor Appl Genet 76, 835-840; Shimamoto, et al. (1989) Nature 338, 274-276; Datta, et al. (1990) Bio/Technology 8, 736-740; Christou, et al. (1991) Bio/Technology 9, 957-962; Peng, et al. (1991) International Rice Research Institute, Manila, Philippines 563-574; Cao, et al. (1992) Plant Cell Rep. 11, 585-591; Li, et al. (1993) Plant Cell Rep. 12, 250-255; Rathore, et al. (1993) Plant Molecular Biology 21, 871-884; Fromm, et al. (1990) Bio/Technology 8, 833-839; Gordon-Kamm, et al. (1990) Plant Cell 2, 603-618; D'Halluin, et al. (1992) Plant Cell 4, 1495-1505; Walters, et al. (1992) Plant Molecular Biology 18, 189-200; Koziel, et al. (1993) Biotechnology 11, 194-200; Vasil, I. K., (1994) Plant Molecular Biology 25, 925-937; Weeks, et al. (1993) Plant Physiology 102, 1077-1084; Somers, et al. (1992) Bio/Technology 10, 1589-1594; WO92/14828; Nilsson, O. et al (1992) Transgenic Research 1, 209-220).

Other methods, such as microprojectile or particle bombardment (US 5100792, EP-A-444882, EP-A-434616), electroporation (EP 290395, WO 8706614), microinjection (WO 92/09696, WO 94/00583, EP 331083, EP 175966, Green et al. (1987) Plant Tissue and Cell Culture, Academic Press), direct DNA uptake (DE 4005152, WO 9012096, US 4684611), liposome mediated DNA uptake (e.g. Freeman et al. Plant Cell Physiol. 29: 1353 (1984)), or the vortexing method (e.g. Kindle, PNAS U.S.A. 87: 1228 (1990d)) may be preferred where Agrobacterium transformation is inefficient or ineffective, for example in some gymnosperm species.

Physical methods for the transformation of plant cells are reviewed in Oard, 1991, Biotech. Adv. 9: 1-11.

Alternatively, a combination of different techniques may be employed to enhance the efficiency of the transformation process, e.g. bombardment with Agrobacterium coated microparticles (EP-A-486234) or microprojectile bombardment to induce wounding followed by cocultivation with Agrobacterium (EP-A-486233).

Following transformation, a plant may be regenerated, e.g. from single cells, callus tissue or leaf discs, as is standard in the art. Almost any plant can be entirely regenerated from cells, tissues and organs of the plant. Available techniques are reviewed in Vasil et al., Cell Culture and Somatic Cell Genetics of Plants, Vol I, II and III, Laboratory Procedures and Their Applications, Academic Press, 1984, and Weissbach and Weissbach, Methods for Plant Molecular Biology, Academic Press, 1989.

The particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

Other aspects of the disclosure relate to the modulation of plant root hair development using *RHD6* related polypeptides and nucleic acids as described herein.

A method of modulating root hair development or altering the root hair phenotype in a plant may comprise;
increasing the expression of a RHD6-related polypeptide within cells of said plant relative to control plants.

Modulation of root hair development in a plant may include increasing one or more of: root-hair growth, number of root-hairs, length of root-hairs, rate of growth of root-hairs, and longevity of individual root-hairs on the plant.

RHD6-related polypeptides are described in more detail above.

Expression of an RHD6-related polypeptide may be increased by any suitable method. In some embodiments, the expression of a RHD6-related polypeptide may be increased by expressing a heterologous nucleic acid encoding the RHD6-related polypeptide within cells of said plant.

Suitable controls will be readily apparent to the skilled person and may include plants in which the expression of the RHD6-related polypeptide is not increased.

A method of producing a plant with altered root hair phenotype may comprise:
incorporating a heterologous nucleic acid which alters the expression of a RHD6-related polypeptide into a plant cell by means of transformation, and;
regenerating the plant from one or more transformed cells.

Suitable RHD6-related polypeptides are described in more detail above.

In some embodiments, the plant may be a plant whose roots are not naturally colonised by symbiotic fungi, such as Mycorrhizae. Plants whose roots are not naturally colonised by fungi include non-mycorrhizal plants such as *Brassicas.*

Plants for use in the methods described herein preferably lack mutations in RHD6-related genes. For example the plant may be a wild-type plant.

A plant with altered root hair phenotype produced as described above may show improved tolerance to nutrient-deficient growth conditions, increased production of phytochemicals and/or increased phytoremediation properties, such as absorption of heavy metals.

Nucleic acid encoding *RHD6*-related polypeptides and their expression in plants is described in more detail above.

In other embodiments, the expression of an RHD6-related polypeptide may be increased by increasing the expression of an endogenous nucleic acid encoding the RHD6-related polypeptide within cells of said plant.

The expression of an endogenous nucleic acid encoding the RHD6-related polypeptide within cells of said plant may be increased by recombinant means, such as the targeted insertion of regulatory factors,

The expression of an endogenous nucleic acid encoding the *RHD6-*related polypeptide within cells of said plant may be increased by non-recombinant means. For example, expression of the RHD6-related polypeptide may be increased in a plant by selective plant breeding methods which employ the RHD6-related amino acid or nucleic acid sequence as a molecular marker in order to produce a plant having an altered root hair phenotype, for example increased size, number or longevity of root hairs relative to controls.

A method of producing a plant having altered root hair phenotype may comprise:
providing a population of plants,
determining the amount of expression of a *RHD6*-related polypeptide as described herein in one or more plants in the population, and
identifying one or more plants in the population with increased expression of the RHD6-related polypeptide relative to other members of said population.

The identified plants may be further propagated or crossed, for example, with other plants having increased RHD6-related polypeptide expression or self-crossed to produce inbred lines. The expression of an RHD6-related polypeptide in populations of progeny plants may be determined and one or more progeny plants with reduced expression of the RHD6-related polypeptide identified.

The expression of an RHD6-related polypeptide in a plant may be determined by any convenient method. In some embodiments, the amount of expression of the RHD6-related polypeptide may be determined at the protein level. A method of producing a plant with altered root hair development may comprise:
providing a population of plants,
determining the amount of RHD6-related polypeptide in one or more plants of said population, and
identifying one or more plants in the population with increased amounts of an RHD6-related polypeptide relative to other members of said population.

The amount of RHD6-related polypeptide may be determined in one or more cells of the plant, preferably cells from a below-ground portion or tissue of the plant, such as the root.

The amount of RHD6-related polypeptide may be determined using any suitable technique. Conveniently, immunological techniques, such as Western blotting may be employed, using antibodies which bind to the RHD6-related polypeptide and show little or no binding to other antigens in the plant. For example, the amount of an RHD6-related polypeptide in a plant cell may be determined by contacting a sample comprising the plant cell with an antibody or other specific binding member directed against the RHD6-related polypeptide, and determining binding of the RHD6-related polypeptide to the sample. The amount of binding of the specific binding member is indicative of the amount of RHD6-related polypeptide which is expressed in the cell.

The expression of the RHD6-related polypeptide may be determined at the nucleic acid level. For example, the amount of nucleic acid encoding an RHD6-related polypeptide may be determined. A method of producing a plant having altered root hair development may comprise:
providing a population of plants,
determining the level or amount of nucleic acid, for example mRNA, encoding the RHD6-related polypeptide in a cell of one or more plants of said population, and,
identifying one or more plants in the population with increased amount of nucleic acid encoding an RHD6-related polypeptide relative to other members of said population.

The level or amount of encoding nucleic acid in a plant cell may be determined for example by detecting the amount of transcribed encoding nucleic acid in the cell. Numerous suitable methods for determining the amount of a nucleic acid encoding an RHD6-related polypeptide in a plant cell are available in the art, including, for example, Northern blotting or RT-PCR (see for example Molecular Cloning: a Laboratory Manual: 3rd edition, Sambrook & Russell (2001) Cold Spring Harbor Laboratory Press NY; Current Protocols in Molecular Biology, Ausubel et al. eds. John Wiley & Sons (1992); DNA Cloning, The Practical Approach Series (1995), series eds. D. Rickwood and B.D. Hames, IRL Press, Oxford, UK and PCR Protocols: A Guide to Methods and Applications (Innis, et al. 1990. Academic Press, San Diego, Calif.).

A suitable cell may be from a below-ground portion or tissue of the plant, such as the root.

A progeny plant identified as having increased RHD6-related polypeptide expression may be tested for altered root hair development relative to controls, for example increased growth, number or longevity of root hairs, or may be tested for other properties, such as increased resistance to nutrient deficient conditions, increased phytochemical production, increased phytoremediation properties or a consitutive low phosphate response.

A method of producing a plant having an altered root hair phenotype may comprise:
crossing a first and a second plant to produce a population of progeny plants;
determining the expression of a RHD6-related polypeptide in the progeny plants in the population, and
identifying a progeny plant in the population in which expression of the RHD6-related polypeptide is increased relative to controls.

A progeny plant having an altered root hair phenotype may show increased growth, number or longevity of root hairs relative to controls (e.g. other members of the population with a wild-type phenotype).

The identified progeny plant may be further propagated or crossed, for example with the first or second plant (i.e. backcrossing) or self-crossed to produce inbred lines.

The identified progeny plant may be tested for increased tolerance to nutrient-deficient conditions relative to controls.

Other aspects of the disclosure provide the use of an RHD6-related polypeptide or encoding nucleic acid as described herein as a marker for the selective breeding of a plant which has an altered root hair phenotype relative to control plants, and a method of selective breeding of a plant which has an altered root hair phenotype relative to control plants, which employs the RHD6-related amino acid or encoding nucleic acid sequence.

In some embodiments, plants having reduced expression of the RHD6-related polypeptide may be produced by random mutagenesis, followed by screening of mutants for reduced RHD6-related polypeptide expression. Suitable techniques are well known in the art and include Targeting Induced Local Lesions IN Genomes (TILLING). TILLING is a high-throughput screening technique that results in the systematic identification of non-GMO-derived mutations in specific target genes (Comai and Henikoff, The Plant Journal (2006) 45, 684-694 Till et al_BMC Plant Biol. 2007 Apr 7, 19.

Those skilled in the art will also appreciate that, based on the genetic information disclosed herein, Targeted Induced Local Lesions IN Genomes ("TILLING", e.g. utilizing PCR-based screening of plants generated through chemical mutagenesis (generally via ethyl methane sulfonate (EMS) treatment), often resulting in the isolation of missense and nonsense mutant alleles of the targeted gene(s); TILLING permits the high-throughput identification of mutations in target genes without production of genetically modified organisms and it can be an efficient way to identify mutants in a specific gene that might not confer a strong phenotype by itself), may be carried out to produce plants and offspring thereof with a change in the *RHD6* or *RSL* gene, thereby permitting identification of plants with specific phenotypes relevant to plant root hair production.

A method of producing a plant having an altered root hair phenotype may comprise:
exposing a population of plants to a mutagen,
determining the expression of a RHD6-related polypeptide or nucleic acid in one or more plants in said population, and
identifying a plant with increased expression of the RHD6-related polypeptide relative to other members of said population.

Suitable mutagens include ethane methyl sulfonate (EMS).

Methods for determining the expression of RHD6-related polypeptide or nucleic acid in plants is described in more detail above.

The identified plant may be further tested for increased tolerance or resistance to low-nutrient conditions relative to controls, increased production of phytochemicals or increased phytoremediation.

A plant identified as having increased expression of the RHD6-related polypeptide relative to controls (e.g. other members of the population) may display increased growth, number or longevity of root hairs relative to the controls.

A plant produced or identified as described above may be sexually or asexually propagated or grown to produce off-spring or descendants. Off-spring or descendants of the plant regenerated from the one or more cells may be sexually or asexually propagated or grown. The plant or its off-spring or descendents may be crossed with other plants or with itself.

Expression of RHD6-related genes such as RSL4 is shown herein to produce a phenotype in which the root hairs display a fungus-like morphology. This morphology is characterised by extensive indeterminate masses of growing cells which resemble fungal like colonies (figure 10) and results in a greatly increased surface area of root hairs. This phenotype may confer significantly enhanced root uptake of phosphate and iron, which is largely limited by the length and surface area of the root hair.

By means of expression of the genes described herein in a plant, a plant may be made to exhibit enhanced absorption of otherwise less efficiently absorbed nutrients. Thus, for example, it is known in the art that phosphate and iron absorption from the soil is achieved primarily by plant root hairs. By enhancing the number, length, time of production or duration of survival of plant root hairs, by expression, overexpression, or targeted misexpression (expression in cells that otherwise may not produce root hairs) of RHD6-related genes, absorption of iron or phosphate or both, as well as other nutrients, may be enhanced. In particular, absorption may be increased by the fungus-like root hair morphology produced by overexpression of RHD6-related genes such as AtRSL4 (see figure 10). Thus, it has been shown in the literature that *rhd6* mutants are compromised in their ability to absorb phosphate, see Plant Growth and Phosphorus Accumulation of Wild Type and Two Root Hair Mutants of Arabidopsis thaliana (Brassicaceae)", Terence R. Bates and Jonathan P. Lynch, American Journal of Botany 87(7): 958-963. 2000. This effect may be reversed by supplementation with the functional gene described herein. Aspects of the invention would be of particular benefit, for example, in low-iron or low-phosphate containing soils, such as those found in China, sub-Saharan Africa and Australia

A method of improving the tolerance or resistance of a plant to nutrient deficient conditions may comprise;
increasing the expression of an RHD6-related polypeptide within cells of said plant relative to control plants.

Nutrient deficient conditions include conditions which contain levels of one or more nutrients such as nitrate, phosphate and/or iron, which are insufficient to fulfil the nutritional requirements of the wild-type plant. A wild-type plant subjected to nutrient deficient conditions may adopt a nutrient deficient phenotype, such as reduced growth resulting in greatly reduced yield and crop quality.

For example, the plant may show improved growth in soil which contains low levels of one or more nutrients such as nitrate, phosphate and/or iron, relative to control plants (i.e. plants in which RHD6-related polypeptide expression is unaltered).

Furthermore, phosphate deficiency increases the expression of RHD6-related polypeptides such as AtRHD6 and AtRSL1 in the root epidermis of a plant. Expression of a RHD6-related polypeptide in the root epidermis may therefore be useful in producing a constitutive "low phosphate" response in a plant.

The genes described herein may be utilized to achieve enhanced production of compounds of interest, including medicinally relevant compounds. Thus, for example, it is known that plant root hairs are responsible for production of antibiotic compounds. In nature, these compounds are secreted by plant roots and especially plant root hairs, to thereby modify or otherwise control the microflora and microfauna surrounding the plant roots. Production of these phytochemicals is enhanced in plants in which the number, length, duration of production, time of production and other characteristics of root hair development and growth may be modified at will according to the methods of this invention.

A method of increasing the production or secretion of a root-secreted phytochemical in a plant may comprise;
increasing the expression of a RHD6-related polypeptide within cells of a plant which secretes the phytochemical through its roots.

Root-secreted phytochemicals include shikonin (Brigham LA, et al Plant Physiol. 1999 Feb;119(2):417-28) which may be produced by *Lithospermum erythrorhizon,* and paclitaxel, which may be produced by *Taxus* spp.

Heavy metals are an important environmental pollutant and may be removed by growing plants on contaminated soils. In phytoremediation or phytoextraction, plants absorb contaminating substances such as heavy metals from the soil and the plants are harvested at maturity, thereby removing these contaminants from the area. The long root hair phenotypes conferred by increased expression of RHD6-related polypeptides may enhance the phytoremediation properties of plant species.

A method of reducing the amount of a contaminating substance in soil comprising;
increasing the expression of a RHD6-related polypeptide within cells of a plant which absorbs the contaminating substance through its roots,
growing the plant or a descendent thereof in soil which comprises the contaminating substance such that the plant or descendent absorbs the contaminating substance from the soil, and
harvesting said plant or descendent thereof.

Contaminating substances include uranium, polychlorinated biphenyls, salt, arsenic and heavy metals such as cadmium, zinc and lead.

A plant suitable for use in the present methods is preferably a higher plant, for example an agricultural plant selected from the group consisting of *Lithospermum erythrorhizon, Taxus* spp, tobacco, cucurbits, carrot, vegetable brassica, melons, capsicums, grape vines, lettuce, strawberry, oilseed brassica, sugar beet, wheat, barley, maize, rice, soyabeans, peas, sorghum, sunflower, tomato, potato, pepper, chrysanthemum, carnation, linseed, hemp and rye.

In methods relating to phytochemical production, *Lithospermum erythrorhizon* and *Taxus* spp may be preferred.

In methods relating to phytoremediation, sunflower (*Helianthus annuus*), Chinese Brake fern, alpine pennycress *(Thlaspi caerulescens),* Indian mustard *(Brassica juncea*), Ragweed (Ambrosia *artemisiifolia)* Hemp Dogbane *(Apocymun cannabinum)* and Poplar may be preferred.

Another aspect of the disclosure provides a plant which is produced by a method described herein, wherein said plant shows altered root hair phenotype relative to controls.

For example, a plant may display increased growth, number or longevity of root hairs relative to controls (e.g. other members of the population with a wild-type phenotype).

A plant may display increased tolerance to nutrient deficient conditions and/or increased production of root-secreted phytochemicals.

Also provided is any part or propagule of such a plant, for example seeds, selfed or hybrid progeny and descendants.

A plant according to the present disclosure may be one which does not breed true in one or more properties. Plant varieties may be excluded, particularly registrable plant varieties according to Plant Breeders Rights.

In addition to a plant produced by a method described herein, the disclosure encompasses any clone of such a plant, seed, selfed or hybrid progeny and descendants, and any part or propagule of any of these, such as cuttings and seed, which may be used in reproduction or propagation, sexual or asexual. Also disclosed is a plant which is a sexually or asexually propagated off-spring, clone or descendant of such a plant, or any part or propagule of said plant, off-spring, clone or descendant.

While the foregoing disclosure provides a general description of the subject matter encompassed within the scope of the present invention, including methods of making and using this invention, the following examples are provided to further enable those skilled in the art to practice this invention and to provide a complete written description thereof. However, those skilled in the art will appreciate that the specifics of these examples should not be read as limiting on the invention, the scope of which should be apprehended from the claims.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above and table described below.
Tables 1 and 2 show a sequence alignment of bHLH amino acid sequences (Heim et al. Mol. Biol. Evol. 2003) generated by ClustalW (http://www.ebi.ac.uk).
Table 3 shows % identities of RHD6-related proteins as determined by DNA Strider (Christain Mark, Center. d'Etudes de Saclay).
Table 4 shows relative identities of the bHLH domains of RHD6-related proteins to RHD6.
Table 5 shoes the correspondence between the names on the tree of figure 3 and the alignments of tables 1 and 2 with respective species and locus or GI accession number.

### EXAMPLES

Root hairs are highly polarised cells that increase the surface area of the plant that is in contact with the soil. They play important roles in nutrient acquisition and anchorage in those land plants that have roots^{1, 2}. Other tip growing cells such as rhizoids and caulonemal cells have a similar function in more basal groups of land plants that lack roots^{3, 4}. Here we identify and characterise two basic helix loop helix transcription factors that control the development of root hair cells in Arabidopsis sporophyte and show that their closest homologs in *Physcomitrella patens* are required for the development of both rhizoids and caulonemal cells in the gametophyte of this moss. This indicates that an ancient mechanism controls the development of functionally and morphologically similar but non-homologous cell types in these divergent groups of land plants. This suggests that the evolution of the land plant body over the past 475 million years^{5,6} has resulted at least in part from the independent recruitment of genes from the gametophyte to the sporophyte.

Unless, stated otherwise, standard techniques were as follows:

### RT-PCR

Total RNA (5 µg for A. thaliana and 1 µg for P. patens) was reverse transcribed with the Superscript First Strand synthesis system (Invitrogen, Carlsbad, USA) in a 20 µl reaction containing oligo d

(T)12-18 primer. One µl of this product was used for PCR in 20 µl reactions containing primers described herein.

### Pollen growth experiments

In vivo and in vitro pollen tube growth experiments were done as described previously (44, 45).

### Southern analysis of Inserts

Southern blots were performed with the DIG System for PCR labelling of DNA probes (Roche Diagnostics, Penzberg, Germany) according to manufacturer protocol Hybridization was done at 42°C in DIG Easy Hyb hybridization buffer.

### Arabidopsis growth conditions.

*Arabidopsis thaliana* (L.) Heyn. lines were grown vertically for 4 days on MS medium + 2% sucrose solidified with 0.5% Phytagel at 24 °C under continuous illumination. For the cellophane disc experiment, the agar was overlaid with a cellophane disk (AA packaging, Preston, UK) before application of the seeds.

### Enhancer trapping and cloning of the AtRHD6 gene

*Atrhd6-2* is an enhancer trap line (1261) of Arabidopsis (ecotype Lansberg erecta) generated with the *DsE* element ¹⁸ that was screened for root hairless phenotype and reporter gene expression in hair cells. Failure to complement *Atrhd6-1* ⁷ indicated that line 1261 carries a mutation that is allelic to *Atrhd6-1.* The DNA sequence flanking the *DsE* element insertion was identified by inverse-PCR ¹⁹. Genomic DNA of the 1261 line was digested by Sau3A I and subsequently ligated using T4 DNA ligase. The ligated DNA was used for PCR with Ds element-specific primers. This showed that the *DsE* is inserted 111 bp upstream the ATG site of *At1g66470* gene (Fig. 7).

### GUS staining of Arabidopsis thaliana roots and embedding

Four-days-old seedlings were stained for 12 hr at 37°C in 1 mM 5-bromo-4-chloro-3-indolyl-glucuronide, 0.5 mM potassium ferricyanide, 0.5 mM potassium ferrocyanide, and 10 mM sodium phosphate buffer (p_{H} 7). Seedlings were embedding in Technovit 7100® resin (Kulzer GmbH, Germany) according to the manufacturer instructions and 10 µm transverse sections were taken from roots.

### Identification of A. thaliana mutants and generation of transgenic plants

Verification of the T-DNA insertion sites in mutants used in this work (Fig. 7) was carried out by sequencing PCR fragments amplified with primers described herein. Atrhd6-3 (ecotype Columbia 0) correspond to the GABI-Kat line 475E09 (10). *Atrsl1-1* (ecotype Columbia 0) corresponding to line WiscDsLox356A02 comes from the Biotechnology centre of the University of Wisconsin. cpc, wer, ttg1 and gl2 mutants have been described previously (32-35).

The genomic constructs AtRHD6p::GFP:AtRHD6 and AtRSL1p::GFP:AtRSL1 contain the promoter and 5'UTR of AtRHD6 or AtRSL1 upstream of the GFP coding sequence fused in N-terminal to the AtRHD6 or AtRSL1 coding region including introns and the AtRHD6 or AtRSL1 3'UTR with terminator. These constructs were generated using the Gateway system (Invitrogen, Carlsbad, USA). The AtRHD6 or AtRSL1 promoter + 5'UTR and the AtRHD6 or AtRSL1 coding region + 3'UTR + terminator were amplified with PCR primers containing recombination sequences and cloned into pDONR P4-P1R and pDONR P2R-P3. A GATEWAY multisite reaction was then performed with the two resulting pDONR plasmids, the plasmid p207-GFP2.5 and the binary vector pGWBmultisite (destination vector). The binary vector pGWBmultisite was generated by replacing the R1-CmR-ccdB-R2 cassette of pGWB1 into R4-CmR-ccdB-R3 (pGWB1 is from Tsuyoshi Nakagawa, Shimane University, Japan). AtRHD6p::GFP:AtRHD6 and AtRSL1p::GFP:AtRSL1 were transformed respectively in Atrhd6-3 and in Atrhd6-3 Atrsl1-1 double mutant by floral dip (36) and transformants were selected on kanamycin (50 µg/ml) and hygromycin (50 µl/ml). Nine independent transgenic lines containing the AtRHD6p::GFP:AtRHD6 construct in the Atrhd6-3 background were obtained. They show different levels of complementation of the AtRhd6- hairless phenotype but all lines express the GFP in hair cells before the emergence of root hair. Five independent lines having the same GFP expression pattern and restore the Atrhd6-3 phenotype were obtained for AtRSL1p::GFP:AtRSL1 transformation in Atrhd6 Atrsll.

For the p35S::PpRSL1 construct, the PpRSL1 coding sequence was amplified from protonema cDNA. This fragment was cloned between the BamHI and SalI sites of a modified pCAMBIA1300 plasmid containing the CaMV 35S promoter and the terminator of pea Rubisco small subunit E9 from 35S-pCAMBIA1301 cloned into its EcoRI and PstI sites (37). The p35S::PpRSL1 construct was transformed by floral dip in Atrhd6-3 and transformants were selected on hygromycin (50 µg/ml). Ten independent transgenic lines that complement the Atrhd6-3 hairless phenotype were obtained.

### Physcomitrella genes isolation and phylogenetic analyses

Physcomitrella *RSLs* and the *PpIND1* genome sequences where obtained from BLAST of the available genome sequence assembled into contigs (http://moss.nibb.ac.jp/). The splice sites were predicted with NetPlantGene 20 and the bHLH coding sequences were confirmed by RTPCR and sequencing. The full length coding sequence of PpRSL1 was obtained by sequencing EST clone pdp31414, provided by the RIKEN BioResource Center ²¹ and the full length coding sequence of *PpRSL2* was obtained by RT-PCR. Sequences have been deposited to GenBank as follows: PpRSL1 (EF156393), PpRSL2 (EF156394), PpRSL3 (EF156395), PpRSL4 (EF156396), PpRSL5 (EF156397) PpRSL6 (EF156398), PpRSL7 (EF156399) and PpIND1 (EF156400). The phylogenetic analysis was performed with PAUP* software as described previously ²².

### Physcomitrella growth conditions

The Gransden wild type strain of *Physcomitrella patens* (Hedw.) Bruch and Schimp 23 was used in this study. Cultures were grown at 25°C and illuminated with a light regime of 16 h light /8h darkness and a quantum irradiance of 40 µE m-2s-1. For the analysis of protonema phenotype, spores kept at 4°C for at least 1 month were germinated in a 5 ml top agar (0.8%) plated on 9 cm Petri dish containing 25 ml of 0.8% agar overlaid with a cellophane disk (AA packaging, Preston, UK). Leafy gametophores were grown on 100 times diluted minimal media ²⁴ supplemented with 5 mg/L NH4 tartrate and 50 mg/L Glucose.

### Constructing mutants in Physcomitrella genes

The constructs for Physcomitrella transformation were made in plasmids pBNRF and pBHSNR. pBNRF carries a *NptII* gene driven by a 35S promoter cloned in the *Eco*RI site of pBilox, a derivative of pMCS5 (MoBiTec, Goettingen Germany) carrying two direct repeats of the loxP sites cloned in the *Xho*I*-Kpn*I and *Bgl*II*-Spe*I sites. pBHSNR contains a *AphIV* gene driven by a 35S promoter clone between the 2 loxP sites of pBilox using *SacI* and *Not*I*.* pPpRSL1-KO was made by cloning *PpRSL1* genomic fragment 1 in pBNRF digested with *Xba*I and *Xho*I and then cloning *PpRSL1* genomic fragment 2 in the resulting plasmid digested with *HpaI* and *Asc*I. pPpRSL2-KO was made by cloning *PpRSL2* genomic fragment 1 in pBHSNR digested with *Mlu*I and *SpeI* and then cloning *PpRSL2* genomic fragment 2 in the resulting plasmid digested with *BamH*I and *HindIII.*

### PEG transformation of protoplasts

PEG transformation of protoplasts was done as described previously ²⁵. pPpRSL1-KO was linearised with *Sca*I and *Ssp*I before protoplast transformation and transformants were selected on G418 (50 µl/ml). pPpRSL2-KO was linearised with *BclI* and SspI before protoplast transformation and transformants were selected on Hygromycin B (25 µl/ml). The *Pprsl1 Pprsl2* double mutants were obtained by transformation of *Pprsl1* line 1 with the pPpRSL2-KO construct. Stable transformants were first selected by PCR using primers flanking the recombination sites and then analysed by Southern blot and RT-PCR. For each transformation, three independent lines having the expected single insertion pattern and being RNA null mutants were selected (Fig. 9). In each case, the 3 transformants selected had the same phenotype.

### Plants overexpressing RHD6 or RSL Genes

For the 35S::RHD6 and RSL2, 3, and 4 constructs, the coding sequence of each gene was amplified from root cDNA with primers as listed below. This fragment was subcloned into a modified pCAMBIA1300 plasmid containing the CaMV 35S promoter and the terminator of pea Rubisco small subunit E9. All these overexpression constructs were transformed by floral dip in *rhd6*/*rsl1* and transformants were selected on hygromycin (50 µl/ml)
35S::RHD6
   CCAGGATCCATGGCACTCGTTAATGACCAT
   CCAGTCGACTTAATTGGTGATCAGATTCGAA
35S::RSL2 CCAGGATCCATGGGAGAATGGAGCAACAA
   CCAGTCGACTCATCTCGGTGAGCTGAGA
35S::RSL3
   CGGGGTACC ATGGAAGCCATGGGAGAAT
   CGCGGATCC TCATCTGGTCAGTGCATTGAG
35S::RSL4
   CGGGGTACCATGGACGTTTTTGTTGATGGT
   CGCGGATCCTCACATAAGCCGAGACAAAAG

### Results

The Arabidopsis root epidermis is organised in alternate rows of hair forming cells (H cells) that produce a tip growing protuberance (root hairs) and rows of non-hair cells (N cells) that remain hairless. *AtRHD6* (*ROOT HAIR DEFECTIVE 6*) positively regulates the development of H cells - *Atrhd6* mutants develop few root hairs (Fig. 1a)⁷.

We cloned *AtRHD6* using an enhancer trap line (*Atrhd6-2*) in which the *GUS* reporter gene is expressed in H cells but not in N cells (Fig.1 c, d, Fig. 7). *AtRHD6* encodes the basic-Helix-loop-helix (bHLH) transcription factor At1g66470⁸. The identification of another independent allele (*Atrhd6-3*) with a similar phenotype and the complementation of the *Atrhd6-3* mutation with a whole gene *AtRHD6p::GFP:AtRHD6* fusion confirmed that the defect in root hair development observed in this mutant is due to mutation of *At1g66470* (Fig. 1a). This complementing *AtRHD6p::GFP:AtRHD6* fusion indicates that AtRHD6 protein accumulates in H cell nuclei in the meristem and elongation zones (Fig. 1b) but disappears before the emergence of the root hair. The spatial pattern of N cells and H cells in the Arabidopsis root epidermis is controlled by a transcriptional network including the positive regulator of H cell identity *CPC* and the negative regulators of H cell identity *WER, TTG* and *GL2*⁹.

To determine if *AtRHD6* is regulated by these genes we analysed the promoter activity of the *Atrhd6-2* enhancer trap in different mutant backgrounds. While the *Atrhd6-2* enhancer trap expresses GUS in cells in the H position this expression spreads to the cells in the N position in the *wer,* ttg and *gl2* mutant backgrounds indicating that *WER, TTG* and *GL2* negatively regulate transcription of *AtRHD6* in the N position (Fig. 1d). No expression was observed in the *cpc* mutant indicating that *CPC* positively regulates *AtRHD6* expression (Fig. 1d). Thus, *AtRHD6* controls the development of root hair cells and acts downstream of the genes involved in epidermal pattern formation.

AtRHD6 is a member of sub family VIIIc of bHLH transcription factors that comprises five other members ^{8, 10}. One of these genes, *At5g37800,* hereafter named *RHD SIX-LIKE1* (*AtRSL1*), is very similar to *AtRHD6* and these two genes may derive from a relatively recent duplication event ⁸. This provides indication that *AtRHD6* and *AtRSL1* might have redundant functions. To determine if AtRSL1 is also required for root hair development we identified a line (*Atrsl1-1*) carrying a complete loss of function mutation in the *AtRSL1* gene and created the *Atrhd6-3 Atrsl1-1* double mutant (Fig. 7).

Because no new phenotypes were observed when these mutants were grown in our standard growth conditions, we grew them on the surface of cellophane discs, where small numbers of root hairs develop in the *Atrhd6-3* single mutant (Fig. 2a). Plants homozygous for the *Atrsl1-1* mutation had wild type root hair morphology when grown on cellophane discs (Fig. 2a). However, the *Atrhd6-3 Atrsl1-1* double mutant did not develop root hairs, indicating that *AtRHD6* and *AtRSL1* have partially redundant functions in root hair development (Fig. 2a). *Atrhd6-3 Atrsl1-1* double mutant plants carrying the genomic construct *AtRSL1p::GFP:AtRSL1* displayed the AtRhd6-3- mutant phenotype, confirming that the extreme hairless phenotype of the *Atrhd6-3 Atrsl1-1* double mutant is the result of a loss of function of both *AtRHD6* and *ATRSL1* genes (Fig. 2a). The complementing GFP:AtRSL1 fusion protein accumulates in hair cells nuclei in the meristem and elongation zones, indicating that *AtRHD6* and *AtRSL1* have similar expression patterns (Fig. 2b). These data indicate that *AtRSL1* and *AtRHD6* act together to positively regulate root hair development.

To determine if *AtRHD6* and *AtRSL1* are required for the development of the only other tip growing cell in flowering plants, the pollen tube, we characterised the phenotypes of pollen tubes in *Atrhd6-3, Atrsl1-1* and *Atrhd6-3 Atrsl1-1* mutants both *in vitro* and *in vivo.* We detected neither a defect in pollen tube growth nor in the segregation of mutant alleles in the F2 progeny of backcrosses to wild type (Fig. 8). No other defective phenotype was detected in any other part of *Atrhd6-3, Atrsl1-1* or *Atrhd6-3 Atrsl1-1* mutants.

Together these data indicate that AtRHD6 and AtRSL1 are bHLH transcription factors that are specifically required for the development of root hairs and act downstream of the genes that regulate epidermal pattern formation in the flowering plant Arabidopsis.

The most ancestral grade of land plants are the bryophytes - the earliest micro fossils of land plants from the middle Ordovician circa 475 Ma have bryophyte characteristics ⁶. Bryophytes do not have roots but possess tip-growing cells that are morphologically similar to root hairs and fulfil rooting functions. In mosses, caulonemal cells increase the surface area of the filamentous protonema tissue in contact with the substrate and rhizoids anchor the leafy gametophore to their growth substrate ^{3, 4} and both cell types are hypothesised to be involved in nutrient acquisition ³. However, rhizoids and caulonema develop from the gametophyte of mosses whereas root hairs develop from the sporophyte of modern vascular plants. Thus, according to the current view that land plants evolved by the intercalation of a sporophytic generation from a haplontic algal ancestor followed by the progressive increase of size and complexity of the sporophyte in parallel to a reduction of the gametophyte ^{11, 12}, neither rhizoids nor caulonema are homologous to root hairs.

To determine if the developmental mechanism that controls the development of root hairs in angiosperms also controls the development of non-homologous tip growing cells with a rooting function in bryophytes, we identified *RHD6-LIKE* genes from the moss *Physcomitrella patens.* We identified seven members of the *AtRHD6* subfamily of bHLH genes from the publicly available Physcomitrella genomic sequence (http://moss.nibb.ac.jp/) providing indication that these genes have been conserved through the land plant evolution. These were designated *Physcomitrella patens RHD6-LIKE 1* to 7 (*PpRSL1* to *PpRSL7).* To analyse the relationship between Physcomitrella and Arabidopsis *RSL* genes we constructed phylogenetic trees by maximum parsimony. A strict consensus tree is presented in Fig. 3. This shows that *AtRHD6, AtRSL1* and the two *Physcomitrella PpRSL1* and *PpRSL2* genes are closely related and together form a monophyletic clade (AtRHD6 clade) that is sister to the clade comprising all the other members of the subfamily (sister clade) (Fig. 3). This indicates that the AtRHD6 clade evolved before the separation of the bryophytes and the vascular plants from a common ancestor.

To characterize the function of the *RHD6-LIKE* genes in moss we constructed deletion mutants that lacked the function of *PpRSL1* and *PpRSL2* genes and determined if they develop morphological defects. Three independent RNA null mutants with single insertions in *PpRSL1* and in *PpRSL2* were made. Double mutants with single insertions into both genes were also generated (Fig. 9). The phenotypes of each of these mutants were then analysed. A haploid protonema develops upon germination of a wild type Physcomitrella spore 3. This filamentous tissue comprises two cell types, the chloronema and the caulonema (Fig. 4 a, b). Chloronemal cells contain large chloroplasts and grow by a slow tip growth mechanism. Caulonemal cells are more elongated, contain few smaller chloroplasts, grow by rapid tip growth and are involved in the colonization of the substrate. Leafy gametophores usually develop from caulonema and are anchored to their substrate by tip growing multicellular rhizoids that are morphologically similar to caulonema (Fig. 4c). The *Pprs11* and *Pprs12* single mutants have slightly smaller and greener protonema cultures than WT and this phenotype is much stronger in the *Pprs11 Pprs12* double mutant which produces small dark green protonema (Fig. 4a). *Pprs11* and *Pprs12* single mutants produce fewer caulonemal cells than the WT indicating that the greener protonema phenotype is the result of a defect in the development of caulonemal cells (Fig. 4b). No caulonemal cells develop in the *Pprs11 Pprs12* double mutant and the protonema of this mutant comprises chloronemal cells only (Fig. 4b). In wild type plants gametophores develop from caulonema but in the *Pprs11 Pprs12* double mutants the gametophores develop from chloronema, as previously observed in another caulonema defective mutant ¹³. The gametophores of the *Pprs11 Pprs12* double mutant develop few very short rhizoids (Fig. 4c). No other defective phenotypes were detected in the chloronema, in the leafy part of the gametophore or in the sporophyte in the single or double mutants. This indicates that *PpRSL1* and *PpRSL2* together regulate the development of caulonemal cells and rhizoids in the moss gametophyte. To determine if protein function is conserved across the land plants we performed a cross-species complementation experiment. Expression of *PpRSL1* under the CaMV35S promoter in the *Atrhd6-3* mutant resulted in the formation of wild type root hairs (Fig. 4d). Thus, the moss *PpRSL1* gene can substitute for loss of *AtRHD6* function in Arabidopsis. This indicates that the molecular function of *PpRSL1* and *AtRHD6* has been conserved since the divergence of seed plants and mosses from a common ancestor and suggests that the same molecular mechanism controls the development of Arabidopsis root hairs and Physcomitrella caulonema and rhizoids.

Plants were engineered to over-express RHD6 or RSL Genes using a constitutive promoter, as described above. The phenotype of these transformants is described below:

### 35S::RHD6

The deficient of root hair phenotype of *rhd6*/*rs11* can be rescued by the over-expression of RHD6. The transformants get longer root hair and higher percent of ectopic root hair (root hairs developed on the non hair cells) than col-0. A few root hairs can be observed on the hypocotyls.

Phosphate deficiency alters *AtRHD6* and *AtRSL1* gene expression. When grown in the presence of sufficient phosphate these genes are expressed in the meristem and elongation zone and transcription is down regulated in the regions where hairs form. When growing in conditions where phosphate is limiting, *AtRHD6* and *AtRSL1* are expressed in the root hair forming zone where they positively regulate the development of root hairs. This shows that phosphate deficiency promotes expression. Therefore, expressing high levels of *AtRSL1* and *AtRHD6* in the root epidermis results in a constitutive "low phosphate" response.

### 35S::RSL2 and 35S::RSL3

The *rhd6*/*rs11* plants harbouring *35S::RSL2* or *35S::RSL3* constructs also develop some root hair. The root hairs are longer than the col-0. Some transgenic lines showed swollen epidermal cells on the roots and hypocotyls. There are also some root hairs on the hypocotyls.

Over-expression of *AtRSL2* and *AtRSL3* using CaMV35S promoter in wild type plants results in the development of long root hairs. These hairs are not as long as those that form fungal like colonies upon over expression of AtRSL4. Plants over-expressing AtRSL2 and AtRSL3 develop stunted phenotypes. This may be due to expression in non-root hair cells.

### 35S::RSL4

The deficient of root hair phenotype of *rhd6*/*rs11* can also be partially complemented by introducing the over expression of RSL4. The transformants show longer root hair than col-0. A few root hairs were also detected on the hypocotyls, which is quite similar with that of RHD6 overexpression transformants.

Over-expression of *AtRSL4* using CaMV35S promoter in wild type plants, causes the formation of long root hairs which can form extensive indeterminate growing masses of cells resembling fungal like colonies (figure 10). The root hair system of a plant overexpressing RSL4 is shown in figure 10. The root is surrounded by a mass of fungus-like cells, which resemble mycorrhizae, the nutrient scavanging fungi that form associations with roots. Furthermore, when the RSL4 is expressed by the 35S promoter, this phenotypic effect (long root hairs) was found to be restricted to the root hair cells. No defective phenotypes resulting from the RSL4 expression were observed elsewhere in the plant.

Plants overexpressing RHD6-related genes may therefore have increased nutrient uptake ability because of their increased surface area resulting from enhanced root hair growth. This effect may be marked in plants, such as Brassicas, which are devoid of mycorrhizae throughout their entire life cycle.

Here we show how closely related transcription factors control the development of tip growing cells that have a rooting function in the seed plant sporophyte and the bryophyte gametophyte. These data indicate that we have identified an ancient developmental mechanism that was present in the common ancestor of the mosses and vascular plants (tracheophytes). These genes will have been important for the invasion of land by plants when nutrient acquisition and anchorage to the solid substrate of the continental surface was necessary. The observation that rhizoids have been found on some of the oldest macro fossils of land plants is consistent with this view ¹⁴⁻¹⁶.

Our results provide indication that *RHD6-LIKE* genes functioned in the haploid generation (gametophyte) of the early land plant life cycle which may have been bryophyte-like ¹⁴, where they controlled the formation of cells with a rooting function. We propose that during the subsequent radiation of the land plants these genes were deployed in the development of the diploid generation (sporophyte) of vascular plants where they control the development of root hairs in angiosperms and we predict they control the development of root hairs and rhizoids in lycophytes (clubmosses and allies) and monilophytes (ferns and horse tails). It is likely that such independent recruitment of genes from haploid to diploid phases of the life cycle was in part responsible for the explosion in morphological diversity of the diploid stage of the life cycle (sporophyte) that occurred in the middle Palaeozoic when green plants colonised the continental surfaces of the planet ¹⁷.

### REFERENCES

1. Carol, R. J. & Dolan, L. Philos. Trans. R. Soc. Lond. B Biol. Sci. 357, 815-21 (2002).
2. Gahoonia, T. S., Care, D. & Nielsen, N. E. Plant Soil 191, 181-188 (1997).
3. Duckett, J. G. et al. Protonemal morphogenesis. In Bryology for the twenty-first century (eds. Bates, J. W., Ashton, N. W. & Duckett, J. G.) 223-245 (British Bryological Society, 1998).
4. Sakakibara, K. et al. Development 130, 4835-4846 (2003).
5. Kenrick, P. & Crane, P. R. Nature 389, 33-39 (1997).
6. Wellman, C. H. et al Nature 425, 282-285 (2003).
7. Masucci, J. D. et al Plant Physiol. 106, 1335-1346 (1994).
8. Heim, M. A. et al. Mol. Biol. Evol. 20, 735-747 (2003).
9. Schiefelbein, J. Curr. Opin. Plant Biol. 6, 74-78 (2003).
10. Bailey, P. C. et al. Plant Cell 15, 2497-2501 (2003).
11. Blackwell, W. H. Bot. Rev. 69, 125-148 (2003).
12. Graham, L. E. et al Proc. Natl Acad. Sci. USA 97, 4535-4540 (2000).
13. Thelander, M. et al J. Exp. Bot. 56, 653-662 (2005).
14. Edwards, D. et al Nature 374, 635-636 (1995).
15. Kerp, H. et al New Data on Nothia aphylla Lyon 1964 ex El-Saadawy et Lacey 1979, a Poorly Known Plant from the Lower Devonian Rynie Chert. In Plants Invade the Land (eds. Gensel, P. & Edwards, D.) 52-82 (Columbia University Press, New York, 2001).
16. Kerp, H. et al T. Roy. Soc. Edin-Earth 94, 411-428 (2004).
17. Davis, P. & Kenrick, P. Fossil Plants (The Natural History Museum, London, 2004).
18. Sundaresan, V. et al. Genes Dev. 9, 1797-1810 (1995).
19. Long, D. et al. Mol. Gen. Genet. 241, 627-636 (1993).
20. Hebsgaard, S. M. et al. Nucleic Acids Res. 24, 3439-3452 (1996).
21. Nishiyama, T. et al. PNAS. USA 100, 8007-8012 (2003).
22. Harrison, C. J. & Langdale, J. A. Plant J. 45, 561-572 (2006).
23. Ashton, N. W. & Cove, D. J. Mol. Gen. Genet. 154, 87-95 (1977).
24. Ashton, N. W. et al Planta 144, 427-435 (1979).
25. Schaefer, D. G. & Zryd, J. P. Plant J. 11, 1195-1206 (1997).
26. Liljegren, S. J. et al. Cell 116, 843-853 (2004).
27. Schiefelbein, J. W. et al The Plant Cell, 2, 235-243 (1990).
28. V. Sundaresan et al., Genes Dev. 9, 1797 (1995).
29. J. D. Masucci et al Plant Physiol. 106, 1335 (1994).
30. D. Long et al., Mol. & Gen. Genet. 241, 627 (1993).
31. M. G. Rosso et al., Plant Mol. Biol. 53, 247 (2003).
32. M. E. Galway et al., Dev. Biol. 166, 740 (1994).
33. M. M. Lee, J. Schiefelbein, Cell 99, 473 (1999).
34. J. D. Masucci, J. W. Schiefelbein, Plant Cell 8, 1505 (1996).
35. T. Wada et al Science 277, 1113 (1997).
36. S. J. Clough, A. F. Bent, Plant J. 16, 735 (1998).
37. K. K. Yi et al., Plant Physiol. 138, 2087 (2005).
38. S. M. Hebsgaard et al., Nucleic Acids Res. 24, (Sep 1, 1996).
39. T. Nishiyama et al. PNAS USA 100, 8007 (2003).
40. C. J. Harrison, J. A. Langdale, Plant J. 45, 561 (2006).
41. N. W. Ashton, D. J. Cove, Mol. & Gen. Genet. 154, 87 (1977).
42. N. W. Ashton, N. H. Grimsley, D. J. Cove, Planta 144, 427 (1979).
43. D. G. Schaefer, J. P. Zryd, Plant J. 11, 1195 (1997).
44. L. M. Fan et al J. Exp. Bot. 52, 1603 (2001).
45. E. Ryan, et al New Phytol. 138, 49 (1998).

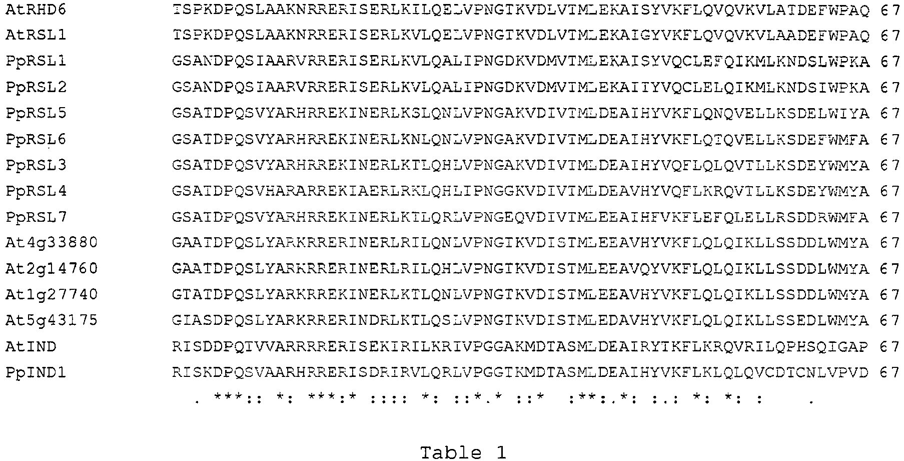

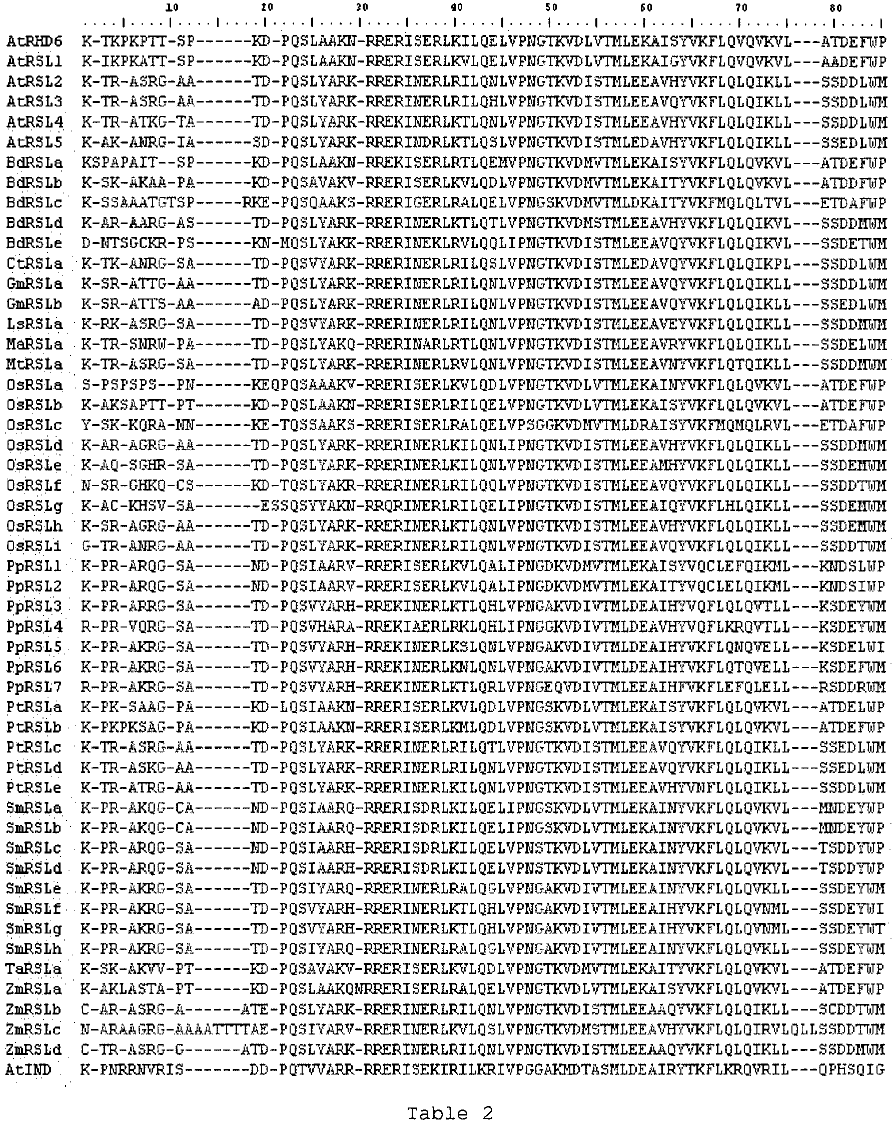

**Table 3**

| **Gene** | **Database reference** | **% Identity with RHD6** | |
|---|---|---|---|
| | | **over full length** | **over bHLH domain** |
| RHD6 | At1g66470 | 100 | 100 |
| RSL1 | At5g37800 | 58.8 | 95.5 |
| RSL2 | At4g33880 | 19.1 | 75 |
| RSL3 | At2g14760 | 22.7 | 75 |
| RSL4 | At1g27740 | 23.2 | 73.1 |
| RSL5 | At5g43175 | 22.3 | 73.1 |

**Table 4**

| Protein | **Sequence Identity AtRHD6** | Protein | **Sequence Identity AtRHD6** |
|---|---|---|---|
| AtRHD6 | 1 | PpRSL1 | 0.625 |
| AtRSL1 | 0.93 | PpRSL2 | 0.611 |
| AtRSL2 | 0.611 | PpRSL3 | 0.583 |
| AtRSL3 | 0.611 | PpRSL4 | 0.513 |
| AtRSL4 | 0.597 | PpRSL5 | 0.597 |
| AtRSL5 | 0.583 | PpRSL6 | 0.611 |
| BdRSLa | 0.835 | PpRSL7 | 0.527 |
| BdRSLb | 0.763 | PtRSLa | 0.791 |
| BdRSLc | 0.675 | PtRSLb | 0.847 |
| BdRSLd | 0.611 | PtRSLc | 0.597 |
| BdRSLe | 0.569 | PtRSLd | 0.597 |
| CtRSLa | 0.625 | PtRSLe | 0.611 |
| GmRSLa | 0.611 | SmRSLa | 0.708 |
| GmRSLb | 0.597 | SmRSLb | 0.708 |
| LsRSLa | 0.611 | SmRSLc | 0.722 |
| MaRSLa | 0.611 | SmRSLd | 0.722 |
| MtRSLa | 0.611 | SmRSLe | 0.625 |
| OsRSLa | 0.78 | SmRSLf | 0.625 |
| OsRSLb | 0.902 | SmRSLg | 0.625 |
| OsRSLc | 0.652 | SmRSLh | 0.625 |
| OsRSLd | 0.597 | TaRSLa | 0.777 |
| OsRSLe | 0.638 | ZmRSLa | 0.835 |
| OsRSLf | 0.597 | ZmRSLb | 0.561 |
| OsRSLg | 0.602 | ZmRSLc | 0.493 |
| OsRSLh | 0.611 | ZmRSLd | 0.589 |
| OsRSLi | 0.597 | *At*/*ND* | *0.424* |

**Table 5**

| **Name** | **Species** | **Locus name** | **GI accession** |
|---|---|---|---|
| AtRHD6 | *Arabidopsis thaliana* | AT1G66470 | |
| AtRSL1 | | AT5G37800 | |
| AtRSL2 | | AT4G33880 | |
| AtRSL3 | | AT2G14760 | |
| AtRSL4 | | AT1G27740 | |
| AtRSL5 | | AT5G43175 | |
| BdRSLa | | | |
| BdRSLb | *Brachypodium distachyon* | | |
| BdRSLc | | N/A | |
| BdRSLd | | | |
| BdRSLe | | | |
| CtRSLa | *Carthamus tinctorius* | | 125399878 |
| GmRSLa | *Glycine max* | | 26056905 |
| GmRSLb | | | 15663066 |
| LsRSLa | *Lactuca saligna* | | 83790803 |
| MaRSLa | *Musa acuminata* | | 102139852 |
| MtRSLa | *Medicago truncatula* | | 92870204 |
| OsRSLa | *Oryza sativa ssp japonica* | Os01g02110 | |
| OsRSLb | | Os02g48060 | |
| OsRSLc | | Os06g30090 | |
| OsRSLd | | Os03g10770 | |
| OsRSLe | | Os03g42100 | |
| OsRSLi | | Os07g39940 | |
| OsRSLf | | Os11g41640 | |
| OsRSLg | | Os12g32400 | |
| OsRSLh | | Os12g39850 | |
| PpRSL1 | | | 140084326 |
| PpRSL2 | | | 140084333 |
| PpRSL3 | | | |
| PpRSL4 | *Physcomitrella patens* | | |
| PpRSL5 | | | |
| PpRSL6 | | | |
| PpRSL7 | | | |
| PtRSLa | | | |
| PtRSLb | | | |
| PtRSLc | *Populus trichocarpa* | | |
| PtRSLd | | | |
| PtRSLe | | N/A | |
| SmRSLa | | | |
| SmRSLh | | | |
| SmRSLb | | | |
| SmRSLc | *Selaginella moelendorfii* | | |
| SmRSLd | | | |
| SmRSLe | | | |
| SmRSLf | | | |
| SmRSLg | | | |
| TaRSLa | *Triticum aestivum* | | |
| ZmRSLa | *Zea mays* | AZM4_60871 | |
| ZmRSLb | | AZM4_70092 | |
| ZmRSLc | | AZM4_91750 | |
| ZmRSLd | | AZM4_86104 | |

### PRIMERS

*A, Amplification and sequencing of* the *insertion* sites *of A. thaliana mutants*
*rhd6-1* insertion site
   AtRHD6-A: 5'-GGATTGATTTAATTACCATATTTAT-3'
   LB2: 5'-CAAGTATCAAACGATGTG-3'
*rhd6-2* insertion site (inverse PCR)
   DL3: 5'-CACCG GTACCGACCGTTACCGACCG-3'
   Ds3I2: 5'-TACCGGTACCGAAAACGAACGGGA-3'
*rhd6-3* insertion site
   AtRHD6-B: 5'-GTTCCCAATGGCACCAAGGTACA-3'
   GABI-LB: 5'-CCCATTTGGACGTGAATGTAGACAC-3'
*rsl1-1* insertion site
   AtRSL1-A: 5'-CGTGTGGACCGACGTCTGA
   JL-202: 5'-CATTTTATAATAACGCTGCGGACATCTAC-3'

*B, Construction of the pRHD6::GFP-RHD6::RHD6t plasmid* Amplification of the *AtRHD6* promoter + 5'UTR fragment from BAC F28G11

RHD6prom-attB4F: 5'-GGGACAACTTTGTATAGAAAAGTTGTTCTCAAAGAGGG
ACAAGACCAAAGCCCATGAC-3'

RHD6prom-attB1R: 5'-GGGGACTGCTTTTTTGTACAAACTTGCTAGACACTAATA
AGTTTGATAAGTGATTTTTTGT-3'

Amplification of the *AtRHD6* coding region + 3'UTR + terminator fragment from BAC F28G11

RHD6term-attB2F: 5'-GGGGACAGCTTTCTTGTACAAAGTGGCCATGGCACTCG
TTAATGACCATCCCAACGAGA-3'

RHD6term-attB3R: 5'-GGGGACAACTTTGTATAATAAAGTTGCTGATAAATCGAG
ATCTTAGGTATGTCGTCC-3'

*C, Construction of the pRSL1::GFP-RSL1::RSL1t plasmid*

Amplification of the *AtRSL1* promoter + 5'UTR fragment from Col0 genomic DNA

RSL1prom-attB4F: 5'- GGGGACAACTTTGTATAGAAAAGTTGTGATAATGGATTG
GAGAAAAATTAAAG-3'

RSL1prom-attB1R: 5'- GGGGACTGCTTTTTTGTACAAACTTGTATTGCAATGTTC
GTTAATGAGTGAC -3'

Amplification of the *AtRSL1* coding region + 3'UTR + terminator fragment from Col0 genomic DNA

RSL1term-attB2F: 5'-GGGGACAGCTTTCTTGTACAAAGTGGGTAATTACAT
CTCAACCCCAAATTCTT-3'

RSL1term-attB3R: 5'-_ACAACTTTGTATAATAAAGTTGATGTATAATTTCCGAA
GATGCTTAAAA -3'

*D, Construction of the p35S::PpRSL1 plasmid*

Amplification of *RSL1* coding sequence from Col0 cDNA
35SPpRDL1 F: 5'-CCAGGATCCATGGCAGGTCCAGCAGGA-3'
35SPpRDL2 R: 5'-CCAGTCGACTTAGTCAGCAGAAGGCTGATT-3'

### E, Construction of the pPpRSL1-KO plasmid

Amplification of *PpRSL1* fragment 1 from *P. patens* WT DNA
PpRSL1KO 1 F: 5'-CCTCTAGAAGTACTTGTGATCCACAGCCTA-3'
PpRSL1KO 1 R: 5'-GGCTCGAGCCGTACTGGGTGGTTTG-3'

Amplification of *PpRSL1* fragment 2 from *P. patens* WT DNA
PpRSL1KO 2 F: 5'-CCGTTAACTTCTACATGTTGCGTTATTTATGGT-3'
PpRSL1KO 2 R: 5'-CCGGCGCGCCAATATTTATATAAATAAGCATAATACACTTCGA-3'

*F, Construction of the pPpRSL2-KO plasmid*
Amplification of *PpRSL2* fragment 1 from *P. patens* WT DNA
PpRSL2KO 1 F: 5'-GCAACGCGTGGGTTTGATCAAAGACGGAA-3'
PpRSL2KO 1 R: 5'-GCTACTAGTCGTCAACCTAACCCAAACAT-3'

Amplification of *PpRSL2* fragment 2 from *P. patens* WT DNA
PpRSL2KO 2 F: 5'-GAGGGATCCGTGAGGTGAAAGCAGTGAAA-3'
PpRSL2KO 2 R: 5'-CCAAAGCTTAGGCCTGTGAACTCGGACA-3'

*G, Amplification of Dig labelled probes for Southern blots*
Amplification of the *PpRSL1* probe from *P. patens* WT DNA
PpRSL1 probe F: 5'-GCTGCTAGGGTAACATAAACATTCTT-3'
PpRSL1 probe R: 5'-CTGGACACTGGAATGAACCTA-3'

Amplification of *NptII (NEOMYCIN PHOSPHOTRANSFERASE* II) probe from pBNRF
NptII probe F: 5'-CCCATGGAGTCAAAGATTCA-3'
NptII probe R: 5'-CCGCGAATTCGAGCTCGGT-3'

Amplification of the *PpRSL2* probe from *P. patens* WT DNA
PpRSL2 probe F: 5'-CCCAAATATGCATTTTTAATCTTT-3'
PpRSL2 probe R: 5'-GCGACAATCCAGCAGCCTCTAT-3'

Amplification of *AphIV (AMINOGLYCOSIDE PHOSPHOTRANSFERASE IV*) probe from pBHSNR
AphIV probe F: 5'-GTAGGAGGGCGTGGATATGT-3'
AphIV probe R: 5'-CGAGTGCTGGGGCGT-3'

*H, amplification of PpRSL2 coding sequence from protonema cDNA*
PpRSL2 RT-PCR F: 5'-GGGACCTCAAGGATGCAGCA-3'
PpRSL2 RT-PCR R: 5'-CGAACTCAATAACGTCAGGA-3'

*I, RT-PCRs*

RT-PCR of At*APT1 (ADENINE PHOSPHORIBOSYLTRANSFERASE 1*)
AtAPT1 RT-PCR F: 5'-TCCCAGAATCGCTAAGATTGCC-3'
AtAPT1 RT-PCR R: 5'-CCTTTCCCTTAAGCTCTG-3'

RT-PCR of *AtRHD6*
AtRHD6 RT-PCR F: 5'-CTCACACGGGAGAGAGCA-3'
AtRHD6 RT-PCR R: 5'-CTTCGATTCTTGGCTGCTA-3'

RT-PCR of *AtRSL1*
AtRSL1 RT-PCR F: 5'-GCCTAGCAGCCAAGAACCGAA-3'
AtRSL1 RT-PCR R: 5'-CTCATCGGCTGCAAGTACCTTA-3'

RT-PCR of *PpRSL1*
PpRSL1 RT-PCR F: 5'-CTGGTTGGTTAGGAGATCTTGCAT-3'
PpRSL1 RT-PCR R: 5'-GTTGTAATTTGGTCCATTTCTGCT-3'

RT-PCR of *PpRSL2*
PpRSL2 RT-PCR F: 5'-GGGACCTCAAGGATGCAGCA-3'
PpRSL2 RT-PCR R: 5'-CGAACTCAATAACGTCAGGA-3'

RT-PCR of *PpGAPDH (GLYCERALDEHYDE 3-PHOSPHATE DEHYDROGENASE)*
PpGAPDH RT-PCR F: 5'-GAGATAGGAGCATCTGTACCGCTTGT-3'
PpGAPDH RT-PCR R: 5'-CGCATGGTGGGATCGGCT-3'

### SEQUENCES

MALVNDHPNETNYLSKQNSSSSEDLSSPGLDQPDAAYAGGGGGGSASSSSTMNSDHQQH
QGFVFYPSGEDHHNSLMDFNGSSFLNFDHHESFPPPAISCGGSSGGGGFSFLEGNNMSYG
FTNWNHQHHMDIISPRSTETPQGQKDWLYSDSTVVTTGSRNESLSPKSAGNKRSHTGEST
QPSKKLSSGVTGKTKPKPTTSPKDPQSLAAKNRRERISERLKILQELVPNGTKVDLVTML
EKAISYVKFLQVQVKVLATDEFWPAQGGKAPDISQVKDAIDAILSSSQRDRNSNLITN

RHD6 amino acid sequence (At1g66470; NP_176820.1 GI: 15219658 SEQ ID NO: 1) 
atggcactcgttaatgaccatcccaacgagaccaattacttgtcaaaacaaaattcctcc
tcttccgaagatctctcctcgccgggactggatcagccagatgcagcttatgccggtgga
ggaggaggaggaggctcggcttcgagcagtagcacgatgaattcagatcatcaacaacat
caggggtttgtattttacccatccggtgaagatcatcacaactctttgatggatttcaac
ggatcatcatttcttaactttgatcatcacgagagctttcctcctccagccataagctgt
ggtggtagtagcggtgggggcggcttctccttcttggagggcaacaacatgagctacggc
ttcacaaactggaatcatcaacatcatatggatattattagccctagatccaccgaaact
ccccaaggccagaaagactggttatattctgattcaactgttgtaaccactggttctaga
aacgagtctctttcgcctaaatccgctggaaacaaacgttctcacacgggagagagcact
caaccgtcgaagaaactgagtagcggtgtgaccggaaagaccaagcctaagccaacaact
tcacctaaagatccacaaagcctagcagccaagaatcgaagagaaaggataagtgaacgt
ctcaagatattgcaagaacttgttcccaatggcaccaaggttgatttggtgacaatgctt
gaaaaggctattagttatgtcaagttccttcaagtacaagttaaggtattagcgaccgat
gagttttggccggctcaaggaggaaaagctcctgacatttctcaagttaaagacgccatt
gatgccattctctcctcatcacaacgagacaggaattcgaatctgatcaccaattaa
RHD6 nucleotide sequence (NM_105318.2 GI: 30697352 SEQ ID NO: 2) 
MSLINEHCNE RNYISTPNSS EDLSSPQNCG LDEGASASSS STINSDHQNN QGFVFYPSGE
TIEDHNSLMD FNASSFFTFD NHRSLISPVT NGGAFPVVDG NMSYSYDGWS HHQVDSISPR
VIKTPNSFET TSSFGLTSNS MSKPATNHGN GDWLYSGSTI VNIGSRHEST SPKLAGNKRP
FTGENTQLSK KPSSGTNGKI KPKATTSPKD PQSLAAKNRR ERISERLKVL QELVPNGTKV
DLVTMLEKAI GYVKFLQVQV KVLAADEFWP AQGGKAPDIS QVKEAIDAIL SSSQRDSNST
RETSIAE
RSL1 amino acid sequence (At5g37800 SEQ ID NO: 3) 
atgtcactcattaacgaacattgcaatgagcgtaattacatctcaaccccaaattcttca
gaagatctctcttcaccacagaattgcggattagacgaaggagcttcagcttcaagcagt
agcaccataaattctgatcatcaaaataatcaaggtttgtgttttacccttccggggaa
accattgaagatcataattctttgatggatttcaatgcttcatcattcttcacctttgat
aatcaccgaagccttatctctcccgtgaccaacggtggtgccttcccggtcgtggacggg
aacatgagttacagctatgatggctggagtcatcatcaagtggatagtattagccctaga
gtcatcaaaactccaaatagctttgaaacaacgagcagttttggattgacttcaaactcc
atgagtaaaccggccacaaaccatggaaatggagactggttatactctggttcaactatt
gtaaacatcggttcaaggcacgagtccacgtcccctaaactggctggcaataaacggcct
ttcacgggagagaacacacaactttcaaagaagccgagtagcggtacgaatggaaagatc
aagcctaaggcaacaacttcacctaaagatccacaaagcctagcagccaagaaccgaaga
gaaaggataagcgaacgcctcaaggtattgcaagaacttgtaccgaatggtaccaaggtg
gatttggtaactatgcttgagaaagcaattggctatgtaaagtttcttcaagtacaagtt
aaggtacttgcagccgatgagttttggccggcacaaggagggaaagctccggacatttct
caagttaaagaagctattgacgcaatcctctcatcatcacaacgagatagtaactcaact
agagaaacaagtatagcagaataa
RSL1 nucleotide sequence (SEQ ID NO: 4) 
MEAMGEWSNN LGGMYTYATE EADFMNQLLA SYDHPGTGSS SGAAASGDHQ GLYWNLGSHH
NHLSLVSEAG SFCFSQESSS YSAGNSGYYT VVPPTVEENQ NETMDFGMED VTINTNSYLV
GEETSECDVE KYSSGKTLMP LETVVENHDD EESLLQSEIS VTTTKSLTGS KKRSRATSTD
KNKRARVNKR AQKNVEMSGD NNEGEEEEGE TKLKKRKNGA MMSRQNSSTT FCTEEESNCA
DQDGGGEDSS SKEDDPSKAL NLNGKTRASR GAATDPQSLY ARKRRERINE RLRILQNLVP
NGTKVDISTM LEEAVHYVKF LQLQIKLLSS DDLWMYAPIA FNGMDIGLSS PR
RSL2 amino acid sequence (At4g33880; SEQ ID NO: 5) 
atggaagccatgggagaatggagcaacaacctcggaggaatgtacacttatgcaaccgag
gaagccgatttcatgaaccagcttctcgcctcttatgatcatcctggcaccggctcatcc
tccggcgcagcagccagtggtgaccaccaaggcttgtattggaaccttggttctcatcac
aaccaccttagcctcgtgtctgaagccggtagcttctgtttctctcaagagagcagcagc
tacagcgctgggaacagcggatattacaccgttgttccacccacggttgaagagaaccaa
aatgagacaatggactttgggatggaagatgtgaccatcaatacaaactcataccttgtt
ggtgaggagacaagtgagtgtgacgttgagaaatactcttctggaaagactcttatgcct
ttggaaaccgtagtggagaaccacgatgacgaggaaagcttgttgcaatctgagatctct
gtgactactacaaaatctctcaccggctccaaaaagagatcccgtgccacatctactgat
aaaaacaagagagcaagagtgaataagaggcccagaagaacgtagagatgagtggggat
aacaatgaaggagaagaggaagaaggagagacgaagttgaagaaaagaaagaatgggca
 
atgatgagtagacagaactcaagcaccactttctgtacggaggaagaatcaaactgcgct
gatcaagacggtggaggagaagactcatcctctaaggaagatgatccctcaaaggccctc
aacctcaatggtaaaacaagagccagtcgtggtgcagccaccgatcctcaaagcctctat
gcaaggaaaagaagagaaaggattaacgagagactaaggattttacaaaatctcgtcccc
aatggaacaaaggtcgatattagtacaatgcttgaggaagcagttcattacgtcaaattt
ttgcagctccaaattaagttattgagctctgatgatctatggatgtatgcgccgattgct
ttcaatgggatggacattggtctcagctcaccgagatga
RSL2 nucleotide sequence (SEQ ID NO: 6) 
MEAMGEWSTG LGGIYTEEAD FMNQLLASYE QPCGGSSSET TATLTAYHHQ GSQWNGGFCF
SQESSSYSGY CAAMPRQEED NNGMEDATIN TNLYLVGEET SECDATEYSG KSLLPLETVA
ENHDHSMLQP ENSLTTTTDE KMFNQCESSK KRTRATTTDK NKRANKARRS QKCVEMSGEN
ENSGEEEYTE KAAGKRKTKP LKPQKTCCSD DESNGGDTFL SKEDGEDSKA LNLNGKTRAS
RGAATDPQSL YARVDISTML EEAVQYVKFL QLQIKRLLAI GTNHRNRSIP LWTARNRQIS
KAHSRKRLRL RAVAKIIWSD EMTRFLLELI TLEKQAGNYR GKSLIEKGKE NVLVKFKKRF
PITLNWNKVK NRLDTLKKQY EIYPAKLRSH PLRFIPLLDV VFRDETVVVE ESWQPRRGVH
RRAPVLDLSD SECPNNNGDE REDLMQNRER DHMRPPTPDW MSQTPMENSP TSANSDPPFA
SQERSSTHTQ VKNVSRNRKR KQNPADSTLD RIAATMKKI
RSL3 amino acid sequence (At2g14760; SEQ ID NO: 7) 
atggaagccatgggagaatggagcaccggcctaggcggaatatatacagaggaagctgac
tttatgaatcagctccttgcctcctatgagcaaccttgtggcggttcatcttcagagaca
accgccacactcacggcctaccaccaccagggttctcaatggaatggtggcttttgcttc
tctcaggagagcagtagttatagtggttactgcgcggcgatgccacggcaagaagaagat
aacaatgggatggaggacgcgacaatcaacacgaacttgtaccttgttggtgaagagaca
agtgaatgtgatgcgacggaatactccggtaaaagcctcttgcctttggagactgtcgca
gaaaaccacgaccatagtatgctacagcctgagaactccttgaccacgaccactgatgag
aaaatgttcaaccaatgtgagagttcaaagaagaggacgcgtgccacaacaactgataag
aacaagagagccaacaaggcacgaaggagccagaaatgcgtagagatgagtggcgaaaat
gaaaatagcggcgaagaagaatatacggagaaggctgcggggaagagaaagaccaaacca
cttaagccgcaaaagacttgttgttcggatgacgaatcaaacggtggagacactttcttg
tccaaagaagatggcgaggactctaaggctctcaacctcaacggcaagactagggccagc
cgcggcgcggccacagatcctcaaagcctttacgcaaggaaaagaagagagaggataaac
gagaggctaaggatttgcaacatctcgtccctaatggaacaaaggttgatattagcacg
atgttggaagaagcagtacaatacgtcaaatttctacagctccaaattaagttattgagc
tctgatgatctatggatgtatgcgcctattgcttacaacggaatggacattggccttgac
ctaaaactcaatgcactgaccagatga
RSL3 nucleotide sequence (SEQ ID NO: 8) 
MDVFVDGELE SLLGMFNFDQ CSSSKEERPR DELLGLSSLY NGHLHQHQHH NNVLSSDHHA
FLLPDMFPFG AMPGGNLPAM LDSWDQSHHL QETSSLKRKL LDVENLCKTN SNCDVTRQEL
AKSKKKQRVS SESNTVDESN TNWVDGQSLS NSSDDEKASV TSVKGKTRAT KGTATDPQSL
YARKRREKIN ERLKTLQNLV PNGTKVDIST MLEEAVHYVK FLQLQIKLLS SDDLWMYAPL
AYNGLDMGFH HNLLSRLM
RSL4 amino acid sequence (At1g27740; SEQ ID NO: 9) 
atggacgtttttgttgatggtgaattggagtctctcttggggatgttcaactttgatcaa
tgttcatcatctaaagaggagagaccgcgagacgagttgcttggcctctctagcctttac
aatggtcatcttcatcaacatcaacaccataacaatgtcttatcttctgatcatcatgct
ttcttgctccctgatatgttcccatttggtgcaatgccgggaggaaatcttccggccatg
cttgattcttgggatcaaagtcatcacctccaagaaacgtcttctcttaagaggaaacta
cttgacgtggagaatctatgcaaaactaactctaactgtgacgtcacaagacaagagctt
gcgaaatccaagaaaaaacagagggtaagctcggaaagcaatacagttgacgagagcaac
actaattgggtagatggtcagagtttaagcaacagttcagatgatgagaaagcttcggtc
acaagtgttaaaggcaaaactagagccaccaaagggacagccactgatcctcaaagcctt
tatgctcggaaacgaagagagaagattaacgaaaggctcaagacactacaaaaccttgtg
ccaaacgggacaaaagtcgatataagcacgatgcttgaagaagcggtccattacgtgaag
ttcttgcagcttcagattaagttgttgagctcggatgatctatggatgtacgcaccattg
gcttacaacggcctggacatggggttccatcacaaccttttgtctcggcttatgtga
RSL4 nucleotide sequence (SEQ ID NO: 10) 
MENEAFVDGELESLLGMFNFDQCSSNESSFCNAPNETDVFSSDDFFPFGTILQSNYAAVL
DGSNHQTNRNVDSRQDLLKPRKKQKLSSESNLVTEPKTAWRDGQSLSSYNSSDDEKALGL
VSNTSKSLKRKAKANRGIASDPQSLYARKRRERINDRLKTLQSLVPNGTKVDISTMLEDA
VHYVKFLQLQIKLLSSEDLWMYAPLAHNGLNMGLHHNLLSRLI
RSL5 amino acid sequence (At5g43175; SEQ ID NO: 11) 
atggagaatgaagcttttgtagatggtgaattggagtctcttttggggatgttcaacttt
gatcaatgttcatctaacgaatcgagcttttgcaatgctccaaatgagactgatgttttc
tcttctgatgatttcttcccatttggtacaattctgcaaagtaactatgcggccgttctt
gatggttccaaccaccaaacgaaccgaaatgtcgactcaagacaagatctgttgaaacca
aggaagaagcaaaagttaagctcggaaagcaatttggttaccgagcctaagactgcttgg
 
agagatggtcaaagcctaagcagttataatagttcagatgatgaaaaggctttaggttta
gtgtctaatacatcaaaaagcctaaaacgcaaagcgaaagccaacagaggatagcttcc
gatcctcagagcctatacgctaggaaacgaagagaaaggataaacgataggctaaagaca
ttgcagagcctagttcctaatgggacaaaggtcgatataagcacaatgctggaagatgct
gtccattacgtgaagttcctgcagcttcaaatcaagctcttgagttcagaagatctatgg
atgtatgcacctcttgctcacaatggtctgaatatgggactacatcacaatcttttgtct
cggcttatttaa
RSL5 nucleotide sequence (SEQ ID NO: 12) 
TSPKDPQSLAAKNRRERISERLKILQELVPNGTKVDLVTMLEKAISYVKFLQVQVKVLATDEFWPAQ
AtRHD6 bHLH amino acid sequence (SEQ ID NO: 13)
 
TSPKDPQSLAAKNRRERISERLKVLQELVPNGTKVDLVTMLEKAIGYVKFLQVQVKVLAADEFWPAQ
AtRLD1 bHLH amino acid sequence (SEQ ID NO: 14)
 
GSANDPQSIAARVRRERISERLKVLQALIPNGDKVDMVTMLEKAISYVQCLEFQIKMLKNDSLWPKA
PpRSL1 bHLH amino acid sequence (SEQ ID NO: 15)
 
GSANDPQSIAARVRRERISERLKVLQALIPNGDKVDMVTMLEKAITYVQCLELQIKMLKNDSIWPKA
PpRSL2 bHLH amino acid sequence (SEQ ID NO: 16)
 
GSATDPQSVYARHRREKINERLKSLQNLVPNGAKVDIVTMLDEAIHYVKFLQNQVELLKSDELWIYA
PpRSL5 bHLH amino acid sequence (SEQ ID NO: 17)
 
GSATDPQSVYARHRREKINERLKNLQNLVPNGAKVDIVTMLDEAIHYVKFLQTQVELLKSDEFWMFA
PpRSL6 bHLH amino acid sequence (SEQ ID NO: 18)
 
GSATDPQSVYARHRREKINERLKTLQHLVPNGAKVDIVTMLDEAIHYVQFLQLQVTLLKSDEYWMYA
PpRSL3 bHLH amino acid sequence (SEQ ID NO: 19)
 
GSATDPQSVHARARREKIAERLRKLQHLIPNGGKVDIVTMLDEAVHYVQFLKRQVTLLKSDEYWMYA
PpRSL4 bHLH amino acid sequence (SEQ ID NO: 20)
 
GSATDPQSVYARHRREKINERLKTLQRLVPNGEQVDIVTMLEEAIHFVKFLEFQLELLRSDDRWMFA
PpRSL7 bHLH amino acid sequence (SEQ ID NO: 21)
 
GAATDPQSLYARKRRERINERLRILQNLVPNGTKVDISTMLEEAVHYVKFLQLQIKLLSSDDLWMYA
At4g33880 bHLH amino acid sequence (SEQ ID NO: 22)
 
GAATDPQSLYARKRRERINERLRILQHLVPNGTKVDISTMLEEAVQYVKFLQLQIKLLSSDDLWMYA
At2g14760 bHLH amino acid sequence (SEQ ID NO: 23)
 
GTATDPQSLYARKRREKINERLKTLQNLVPNGTKVDISTMLEEAVHYVKFLQLQIKLLSSDDLWMYA
At1g27740 bHLH amino acid sequence (SEQ ID NO: 24)
 
GIASDPQSLYARKRRERINDRLKTLQSLVPNGTKVDISTMLEDAVHYVKFLQLQIKLLSSEDLWMYA
At5g43175 bHLH amino acid sequence (SEQ ID NO: 25)
 
MAGPAGALWSTCDPQPIQQAEIFSGPDNQAGLMSFHVDTPFHWGSEPWALHSRSDDIALMSPSLVHDISPYDSVL
HLSGVSGDVQDLVCGNPKFRQSGQWGQSEFSYSVQDNMQDLLTNQFIPYNTSSLGLNHLSPNFTDLDCAPVYNDT
KAFGTVTHNRAVPSTNTQSAQHGSSSMVSSNRPITSTASPTTQYGGPRTPSQTTQYGGSSMVTNSMEMFASAAPQ
GIMTTSGLSGGCNSDLMHLPKRQHAHSLPPTTGRDLTASEVVSGNSISNISGVGSFNSSQKSSASVMMSPLAASS
 
HMHKAAAVSEELKMASFNPGPFVPTQKKQHEQQDTMTSNRIWADKNNLGKISSSPIPIMGFEQSQQQSMSNSSP
VTSLGFEQRQKMSMGSSPSITIIGFEQRQKQPMSSSSPISNMVFEPRQKQPMSSSSPISNIVFEQRQLPTVGSSP
PISISGFEPKKQPSLSNSPPLSNLGFEQRLQPMSNASPISNLPFEQQRQQATMSNTRSAEPDSVESTTKWPLRMD
GAIGGCAGLPSSQKAPVIMQPETGTMKCPIPRTMPSNAKACPAVQNANSVNKRPLTVDDKDQTGSMNKKSMQKFL
GPQGCSRLESISALAHQKVSQSTTSGRALGPALNTNLKPRARQGSANDPQSIAARVRRERISERLKVLQALIPNG
DKVDMVTMLEKAISYVQCLEFQIKMLKNDSLWPKALGPLPNTLQELLELAGPEFAGIDGKNTEESSEKPKKSALE
VIELDGNQPSAD*
Physcomitrella RHD SIX LIKE 1 (PpRSL1) amino acid sequence (SEQ ID NO: 26; ABO84930.1 GI: 140084327) 
atggcaggtccagcaggagctttatggagtacttgtgatccacagcctattcaacaggcagagatatttagtggt
cctgacaaccaagctggtttgatgtcttttcatgtggataccccgttccattggggatctgaaccatggctctc
cactctcggtcagatgacatcgccttgatgtcccctcgcttgttcacgacatatcaccttatgattctgtcttg
catctttccggagtgtctggggatgtgcaagatttagtttgcgggaatcccaaatttcgccaaagtgggcaatgg
gggcagagcgagttttcatactctgttcaggacaacatgcaagatctcctaaccaaccagttcataccgtacaac
acatcttcattgggtttaaatcatctctccccgaatttcaccgacttggattgcgcaccggtatacaatgatacc
aaggcttttggcactgttacacacaacagggcagtcccgagcactaatacccagagtgctcagcacggagttcg
tctatggtttcaagtaacaggccaatcactagcacagcttctcctactactcagtatggaggtccgaggactcca
tcccaaaccacccagtacggggggttcatctatggttaccaactcgatggaaatgtttgcttcagctgcacctcag
ggtattatgactacatctggcttgagtggcggttgcaactcagacttgatgcatctgccgaagcgccagcatgct
cactctcttcctcctaccactggcagagatttaactgcatctgaagtggtatctggaaattcgatatcaaacatt
tccggggttggatcttttaacagcagccagaaaagcagtgcatccgtgatgatgtctcctttagctgcttcttct
cacatgcacaaggctgctgctgtatctgaagaacttaagatggcaagtttcaaccctggtccattcgtacctacg
cagaaaaagcagcaacatgagcagcaggatacgatgacctctaatcgtatatgggcggataagaacaacttggga
aaaattagttcatcgcccattccgatcatggggtttgagcagagtcaacagcaatccatgagcaattcctcccct
gttaccagtttggggtttgagcaaaggcaaaaaatgtccatgggtagctctccctccatcacgatcattggattt
gagcaaagacagaagcaacctatgagtagttcttcccccatttcaaacatggtttttgaaccaagacaaaaacag
ccaatgagtagctcttctcctatctctaatattgtctttgagcaaagacaactcccaactgtgggtagctctcct
ccgatttcaatctcaggatttgagccaaagaaacaaccatctttgagcaattctcctcccctctctaatctgggt
tttgagcaaaggctacaacccatgagtaatgcatctcctatttccaacttaccctttgagcaacaaagacaacaa
gcaaccatgagtaacaccagatctgcagaacccgattctgtcgagtctaccacgaagtggcccttgcggatggat
ggtgccataggtggatgtgctggcttaccaagcagtcagaaagctcctgttatcatgcagcctgagactgggact
atgaagtgtcctattccgaggaccatgcccagcaatgctaaggcttgcccagctgtgcagaatgctaattccgta
aacaagcgccctcttacggttgatgacaaggaccaaactggatcgatgaataagaagtcgatgcaaaagttttg
ggacctcaaggttgtagcagacttgaaagtatcagtgctttagctcaccaaaaagtgagtcaaagtacaacaagc
ggtcgtgctctagggcctgctttgaacaccaatctcaagcctcgtgcacgccaagggagtgccaatgatccgcag
agcattgctgctagggtgcgaagagaaagaataagtgagcggctcaaagttttgcaagccttgatacctaacggt
gataaagtggatatggtcaccatgctggagaaggctatcagctacgtgcagtgtttggaatttcagattaagatg
ttaaaaaatgactctttgtggcctaaggcgcttggccctctaccgaacactttgcaagagcttctcgaacttgct
gggccagagtttgccggcatagatggcaagaatactgaggagtcgtcagagaaaccgaagaaatctgctcttgaa
gtaattgagttggacggcaatcagccttctgctgactaa
Physcomitrella RHD SIX LIKE 1 (PpRSL1) nucleotide sequence (SEQ ID NO: 27; EF156393.1 GI: 140084326) 
MNKKPMQKALGPQGCSRLESISALAHQKVSQSASGRALGPALNTNLKPRARQGSANDPQSIAARVRRERISERLK
VLQALIPNGDKVDMVTMLEKAITYVQCLELQIKMLKNDSIWPKALGPLPNTLQELLELAGPEFSGTESKNVEEPP
AKPKKSAPDVIEFDGNQPSADKE*
Physcomitrella RHD SIX LIKE 2 (PpRSL2) amino acid sequence (SEQ ID NO: 28 ABO84931.1 GI: 140084334) 
atgaataagaagcctatgcaaaaagctttggacctcaaggatgcagcaggctagaaagcatcagtgctttagct
catcaaaaagtgagtcagagtgcaagtggtcgtgcactagggcctgctctgaacaccaacctcaagcctcgtgct
cgtcaagggagtgccaatgacccacagagcattgccgctagggttcgaagagaaaggataagtgagcggctgaaa
gttttgcaagccttgatacctaatggtgataaggtagatatggtgaccatgctggagaaggctatcacctacgtg
cagtgtctggaactccagattaagatgttaaagaatgattctatctggcccaaggcgcttggacctctaccaaac
 
actctt caagagcttctggagcttgctggaccagaattttctggaacggaaagcaagaatgtagaggagcccca
gcgaagccaaagaaatcagctcctgacgttattgagttcgacggcaatcaaccttctgccgacaaagagtag
Physcomitrella RHD SIX LIKE 2 (PpRSL2) nucleotide sequence (SEQ ID NO: 29; EF156394.1 GI: 140084333)
   GSATDPQSVYARHRREKINERLKTLQHLVPNGAKVDIVTMLDEAIHYVQFLQLQVTLLKSDEYWMYA
Physcomitrella RHD SIX LIKE 3 (PpRSL3) amino acid sequence (SEQ ID NO: 30; ABO84932.1 GI: 140084346) 
ggttcagcgactgatccgcagagtgtatatgccaggcatagaaggagaagatcaacgagcgcttgaagacatta
cagcacttggtaccaaatggagctaaggtagacatcgtgaccatgcttgacgaagccattcactacgtccaattt
ctgcagctccaagtgacgctgttgaagtcggatgaatattggatgtacgcc
Physcomitrella RHD SIX LIKE 3 (PpRSL3) nucleotide sequence (SEQ ID NO: 31; EF156395.1 GI:140084345) 
MTDLISILESSGSSREEMCPVAVPSSVASSCERLIWEGWTAQPSPVEESTTSKLLPKLLPELETSSYSALTLQQP
DALSSILSVLHPFSHYSSASLELARNPDWSLKSSNPLRESSSEAGIRTSSFEGLYSGQHTTKKIHLGVIPYHLSE
DQRQCAVSPPENECRLLSANSSGSLHWWHSIGPESPSSTLAFHNIGIQHSTFEKCEPRGQSHSSWPAASGTSPTV
QYFHAHSADNEGVEVVKQDDSQISKALATYQPHGDHSLVLNSDRIASTTSHSEDPCGPKPGRRPAASYDTEMILS
PSESFLTTPNMLSTLECVISGASNISDQYMNFVREPQEQRLSSISDLSLIPDSHADPHSIGFISGTFRTDSHGTG
IRKNRIFLSDEESDFLPKKRSKYTVRGDFQMDRFDAVWGNTGLRGSSCPGNSVSQMMAIYEFGPALNRNGRPRVQ
RGSATDPQSVHARARREKIAERLRKLQHLIPNGGKVDIVTMLDEAVHYVQFLKRQVTLLKSDEYWMYATPTSYRS
KFDDCSLVPGENN*
Physcomitrella RHD SIX LIKE 4 (PpRSL4) amino acid sequence (SEQ ID NO: 32; ABO84933.1 GI:140084359) 
ATGACCGATCTGATTTCGATCTTGGAGTCATCAGGGTCATCACGAGAGGAGATGTGCCCTGTTGCTGTGCCAAGC
TCCGTGGCTTCTTCTTGTGAAAGGTTGATATGGAGGGGTGGACTGCACAACCATCTCCTGTCGAAGAAAGCACC
ACCAGCAAGTTACTTCCAAAGCTACTTCCAGAGCTCGAGACATCATCCTACTCTGCACTCACCCTTCAGCAACCT
GATGCGCTCTCCAGCATACTTTCAGTCCTCCACCCTTTTTCTCATTACAGTTCGGCCAGTTTAGAACTCGCTCGC
AATCCTGACTGGAGCTTGAAATCTTCAAATCCTCTGCGGGAAAGCAGCTCGGAGGCTGGCATCCGAACCTCATCT
TTCGAAGGCTTGTACTCTGGTCAGCACACCACCAAAAAGATTCATTTGGGGGTCATACCCTACCACTTGTCCGAA
GATCAGCGCCAGTGCGCTGTCAGTCCTCCGGAAAATGAGTGCCGCCTACTGTCTGCAAATTCCTCTGGATCCCTT
CACTGGTGGCATTCCATAGGCCCCGAGTCTCCTTCCTCTACTCTTGCATTCCATAATATTGGGATCCAACACTCT
ACCTTCGAAAAGTGTGAGCCTAGGGGCCAGTCGCACTCATCATGGCCAGCGGCCAGCGGCACGTCGCCAACAGTT
CAATACTTTCATGCCCATTCTGCAGATAATGAAGGTGTCGAGGTCGTCAAGCAAGATGACTCGCAGATATCCAAG
GCTCTGGCGACCTATCAACCCCACGGCGACCATAGTCTCGTGCTAAATTCAGACCGCATTGCAAGCACAACCAGC
CACTCAGAAGATCCTTGCGGCCCTAAACCTGGACGCAGACCAGCTGCATCATACGACACCGAGATGATTCTTAGC
CCAAGTGAGAGTTTCTTGACAACTCCCAATATGTTATCAACGTTGGAGTGCGTAATATCCGGTGCAAGTAACATA
TCTGATCAGTATATGAACTTCGTCAGAGAACCGCAGGAGCAAAGGCTGTCCTCTATCTCCGATCTGTCCCTTATT
CCTGACAGCCACGCGGATCCGCACAGTATCGGATTTATCTCTGGGACCTTTAGAACAGACTCCCACGGAACTGGA
ATAAGAAAGAACCGCATCTTTCTCAGTGATGAGGAATCCGACTTCTTGCCTAAGAAGCGATCCAAGTACACGGTC
CGCGGCGATTTTCAGATGGATCGCTTCGACGCAGTTTGGGGGAATACCGGTCTTCGGGGATCTAGCTGTCCTGGA
AATTCAGTATCCCAGATGATGGCGATTTACGAATTCGGACCCGCACTGAACAGGAACGGCAGGCCGCGAGTACAA
CGTGGTTCGGCGACTGATCCGCAGAGTGTACACGCCAGGGCGCGGAGGGAGAAAATCGCCGAGCGCTTGAGAAAG
TTGCAGCACCTCATTCCAAACGGCGGGAAGGTGGACATCGTAACCATGCTCGACGAAGCCGTTCACTATGTTCAG
TTTTTGAAGCGACAAGTTACGCTTCTGAAATCCGACGAGTATTGGATGTACGCCACGCCGACCTCGTACCGGAGC
AAATTCGACGACTGCAGTCTGGTTCCCGGCGAGAACAACTGA
Physcomitrella RHD SIX LIKE 4 (PpRSL4) nucleotide sequence (SEQ ID NO: 33; EF156396.1 GI: 140084358) 
MVQLYMSSVEEQRETMVQPYVSSMDSGTSGRQTPSCVVQQGSNTFETSNLWEEWTQASNGDDTVSTSNFLPEIS
SFTSSRLSFQQSDSLTTWMSGFPPLSQTALSPDLSHSSDPVDHPPAFMQEGLGPGDSILDYSPALTEMYPKSSSK
 
HNSSDCLPYPAASAPDKKMTDHELGSAISLAYDRGTVSRQLLRALGPLSPSSPLALQNGLQNPLGDPWDASPSAM
PWPMATTGHAYGPGATRTSIPDHLANAINHLEGIAPSSASHASKPRHTDIFIAPNGTFDSTPGGWTPQYYDGSVT
TDESVKAMKLIASLREAGHAEATIGFCTESKPSFLRGGDRTTSPVDSFFGKCVGAKTSIKQACSGKHPLELEEIV
DSENSELNPTQLKRSKLFENHPNALWSDQSMNGRELRSYSHLVGSSLTASQPMDIIAIGPALNTDGKPRAKRGSA
TDPQSVYARHRREKINERLKSLQNLVPNGAKVDIVTMLDEAIHYVKFLQNQVELLKSDELWIYATPNKYNGMDIS
DLSDMYLQELESRA*
Physcomitrella RHD SIX LIKE 5 (PpRSL5) amino acid sequence (SEQ ID NO: 34: ABO84934.1 GI: 140084368) 
ATGGTGCAGTTATACATGTCCTCAGTTGAAGAGCAGCGGAAACAATGGTACAGCCATACGTCTCAAGCATGGAC
TCAGGCTCAACGTCGGGGCGCCAGACGCCATCTTGCGTCGTTCAGCAGGGAAGTAACACATTTGAGACTTCGAAT
CTGTGGGAGGAATGGACGCAAGCATCGAACGGCGACGATACAGTCTCCACCAGCAATTTCCTCCCCGAAATCAGT
TCCTTCACGTCGAGTCGTCTCTCCTTCCAGCAAAGCGACTCTCTCACCACTTGGATGTCAGGGTTCCCTCCCCTC
TCCCAAACTGCCTTGAGCCCGGATCTTAGTCACTCCTCCGACCCCGTGGATCATCCCCCAGCATTCATGCAGGAG
GGTTTAGGCCCCGGTGATTCTATTCTGGACTATTCCCCCGCTCTCACAGAGATGTACCCGAAAAGTAGCTCCAAA
CATAATTCCTCGGATTGTTTACCTTACCCTGCGGCCAGTGCACCAGACAAAAAAATGACTGATCACGAACTAGGT
TCGGCTATTTCCCTCGCGTATGATAGAGGCACCGTTTCCCGCCAGCTTCTTCGAGCCTTGGGCCCATTGTCGCCT
TCATCGCCTCTAGCATTGCAGAATGGGCTGCAAAACCCGCTTGGGGACCCCTGGGATGCTTCTCCATCTGCAATG
CCGTGGCCAATGGCAACAACCGGTCATGCTTATGGACCAGGCGCCACCAGGACTTCTATTCCAGATCACTTAGCA
AATGCAATTAATCACCTGGAGGGCATTGCACCGTCCAGTGCCAGTCATGCATCGAAACCTCGTCACACTGATATT
TTCATTGCACCCAATGGCACGTTCGATTCGACGCCGGGAGGTTGGACACCGCAGTATTACGATGGGTCCGTGACG
ACAGATGAGTCTGTGAAGGCGATGAAGCTGATTGCGTCCCTACGTGAAGCAGGCCACGCAGAGGCTACAATTGGA
TTCTGTACAGAGAGCAAGCCTAGTTTTCTCAGGGGTGGGACAGAACAACCTCGCCAGTGGACAGCTTCTTCGGC
AAATGTGTAGGGGCCAAAACGAGTATAAAGCAAGCCTGTTCTGGGAAACACCCTCTTGAACTTGAGGAGATCGTT
GATAGTGAAAACAGTGAATTAAATCCCACCCAGCTCAAACGCTCTAAACTTTTGAGAATCATCCGAATGCCTTG
TGGAGCGATCAGAGTATGAATGGAAGAGAACTGAGATCGTACTCTCATTTGGTTGGCAGCAGTCTTACTGCATCG
CAGCCCATGGACATAATTGCAATTGGCCCAGCGCTCAACACTGATGGCAAACCACGAGCAAAGCGGGGTTCAGCA
ACCGATCCTCAGAGTGTTTACGCTAGACATAGGAGAGAAAAAATCAACGAACGATTGAAGAGTTTACAAAACCTA
GTACCTAATGGAGCCAAGGTTGACATAGTAACCATGCTGGACGAAGCTATACATTACGTCAAATTTTTACAAAAT
CAAGTTGAGCTGCTGAAGTCCGACGAGTTGTGGATTTACGCAACACCAAATAAGTACAACGGCATGGACATTTCC
GACCTCTCTGACATGTATTTGCAGGAGCTGGAGTCACGTGCGTGA
Physcomitrella RHD SIX LIKE 5 (PpRSL5) nucleotide sequence (SEQ ID NO: 35; EF156397.1 GI: 140084367) 
MVRFNYMYPVQEQLEAMTDQHTPSMDSVSSAGEKTSSCIVQQGGNASETSNLWEEWTQGSNGDDSVSTSNFLPEL
NSSTSSRLAFHQSDILSTWISGYHPLSQSSLSSEFSHTSDRENHPPAFMQEGLIPSGLILDSDPALTDIYTRSSS
SDSLPYPTARIMDKALTDHELESAVPLAYEKGCVPPQVLRNLGPLSPSSPLAFQNGLLNPLRDPWDSCPSALPWS
NVTTASQTYGQVTTRTFIPDHSASAIDKLEAVATITAGYGASKPQHTDVFIEPNGTFQSTPAGWAPQFYDGSEAT
GLLVKPMRAIASLGEAGCGEATSEFCTKTKPGLLKGGDTITSPVGSLLGDCKKAESSMKQVWPGKHRLELVELVD
GEDTKSSPTQLKRPKHSTDYANVLLSDHILKGAELRSYFHSGDVGLNASQAMDIIVIGPALNTNGKPRAKRGSAT
DPQSVYARHRREKINERLKNLQNLVPNGAKVDIVTMLDEAIHYVKFLQTQVELLKSDEFWMFANPHNYNGIDISD
PSSMHSPELESNI*
Physcomitrella RHD SIX LIKE 6 (PpRSL6) amino acid sequence (SEQ ID NO: 36; ABO84935.1 GI: 140084376) 
ATGGTGCGGTTTAACTACATGTACCCGGTTCAAGAGCAGCTGGAAGCCATGACGGACCAACACACCCCAAGCATG
GATTCGGTCTCGTCGGCCGGAGAGAAGACATCCTCTTGCATCGTCCAGCAGGGAGGAAATGCATCCGAAACTTCA
AACTTGTGGGAAGAATGGACACAAGGGTCGAACGGCGACGATTCTGTCTCTACCAGCAACTTCCTCCCCGAACTG
AATTCCTCCACCTCCAGTCGTCTCGCATTCCACCAAAGCGACATTCTTTCCACTTGGATCTCAGGCTACCACCCA
CTCTCGCAAAGCAGCCTGAGTTCCGAATTCAGCCACACCTCCGACCGCGAGAATCACCCCCCAGCATTCATGCAA
GAGGGTTTAATCCCCAGTGGTTTAATTCTTGACTCTGATCCTGCTCTCACAGATATTTATACGAGAAGCAGCTCC
TCGGACTCTTTGCCATACCCCACGGCTAGGATCATGGACAAAGCATTGACCGATCACGAGCTTGAGTCTGCTGTC
CCACTTGCATATGAAAAAGGCTGCGTTCCTCCCCAGGTTCTGCGTAACCTAGGGCCATTGTCACCTTCTTCGCCT
CTGGCATTCCAGAATGGACTGCTAAACCCCCTCAGGACCCTTGGGATTCGTGTCCATCTGCATTGCCATGGTCA
AATGTGACCACAGCCAGCCAGACTTACGGTCAAGTGACAACCAGGACTTTCATTCCAGATCACTCTGCAAGTGCA
 
ATCGACAAGTTGGAGGCCGTCGCAACGATCACTGCCGGATACGGCGCGTCGAAACCACAACATACTGACGTCTTC
ATAGAACCCAACGGGACGTTTCAGTCGACTCCGGCAGGGTGGGCACCGCAGTTTTACGATGGATCCGAGGCGACG
GGCCTGTTGGTCAAGCCAATGAGGGCCATCGCATCTCTGGGTGAAGCCGGCTGTGGGGAGGCCACTAGTGAATTC
TGCACAAAGACCAAGCCAGGACTTCTCAAAGGTGGGGACACAATAACCTCGCCGGTGGGTAGCCTGTTGGGCGAT
TGCAAAAAAGCTGAGTCAAGTATGAAGCAAGTTTGGCCTGGAAAACACCGTCTTGAACTCGTGGAACTAGTCGAT
GGTGAAGACACCAAATCAAGTCCCACCCAGCTCAAACGGCCGAAACATTCTACGGATTATGCGAATGTCCTGTTG
AGCGATCATATTCTGAAAGGAGCGGAGCTGCGGTCCTACTTCCATTCTGGTGATGTTGGTCTAAATGCATCTCAA
GCGATGGACATTATTGTAATTGGCCCAGCCTTGAATACTAATGGCAAGCCGCGAGCTAAACGGGGTTCAGCCACC
GATCCCCAGAGTGTGTACGCTAGACATAGGCGAGAAAAAATCAACGAACGACTGAAGAATTTACAAAATCTCGTG
CCAAATGGAGCCAAGGTTGACATTGTGACCATGCTAGACGAAGCCATACACTACGTCAAATTCTTGCAAACTCAA
GTTGAGCTGCTGAAATCCGACGAGTTCTGGATGTTCGCAAATCCACACAACTACAACGGCATAGATATCTCCGAT
CCCTCTAGCATGCATTCGCCGGAGCTGGAGTCGAATATTTAG
Physcomitrella RHD SIX LIKE 6 (PpRSL6) nucleotide sequence (SEQ ID NO: 37: EF156398.1 GI: 140084375)
   GSATDPQSVYARHRREKINERLKTLQRLVPNGEQVDIVTMLEEAIHFVKFLEFQLELLRSDDRWMFA
Physcomitrella RHD SIX LIKE 7 (PpRSL7) amino acid sequence (SEQ ID NO: 38; ABO84936.1 GI: 140084384) 
Gggtcagctactgatcctcagagtgtgtacgcaaggcatcgccgggagaagattaacgagcgcctaaagacattg
cagcggttggttcctaacggagaacaggtcgacattgtgaccatgctggaagaagccattcactttgtcaaattt
ttggagttccaactggagctgttgcgatccgatgatcgctggatgttcgcc
Physcomitrella RHD SIX LIKE 7 (PpRSL7) nucleotide sequence (SEQ ID NO: 39; EF156399.1 GI: 140084383) 
LNTNLKPRAKQGCANDPQSIAARQRRERISDRLKILQELIPNGSKVDLVTMLEKAINYVKFLQLQVKVLMNDEYW
PPKGD
Selaginella moelendorfii SmRSLa amino acid sequence (SEQ ID NO: 40) 
CTCAACACTAATCTTAAGCCGCGAGCAAAGCAAGGTTGTGCTAATGATCCACAAAGCATTGCTGCCAGACAACGA
AGAGAACGGATAAGTGACCGGCTTAAAATCCTGCAGGAGCTCATACCAAATGGATCCAAGGTCGATCTGGTAACC
ATGCTGGAGAAGGCCATCAACTACGTCAAGTTCTTGCAATTGCAAGTCAAAGTTCTTATGAACGATGAGTATTGG
CCACCAAAGGGAGAT
Selaginella moelendorfii SmRSLa nucleotide sequence (SEQ ID NO: 41) 
LNTNLKPRAKQGCANDPQSIAARQRRERISDRLKILQELIPNGSKVDLVTMLEKAINYVKFLQLQVKVLMNDEYW
PPKGD
Selaginella moelendorfii SmRSLb amino acid sequence (SEQ ID NO: 42) 
CTCAACACTAATCTTAAGCCGCGAGCAAAGCAAGGTTGTGCTAATGATCCACAAAGCATTGCTGCCAGACAACGA
AGAGAACGGATAAGTGACCGGCTTAAAATCCTGCAGGAGCTCATACCAAATGGATCCAAGGTCGATCTGGTAACC
ATGTTGGAGAAGGCCATCAACTACGTCAAGTTCTTGCAATTGCAAGTCAAAGTTCTTATGAACGATGAGTATTGG
CCACCAAAGGGAGAT
Selaginella moelendorfii SmRSLb nucleotide sequence (SEQ ID NO: 43) 
LNTNFKPRARQGSANDPQSIAARHRRERISDRLKILQELVPNSTKVDLVTMLEKAINYVKFLQLQVKVLTSDDYW
P
Selaginella moelendorfii SmRSLc amino acid sequence (SEQ ID NO: 44) 
CTCAACACCAATTTCAAGCCTCGAGCCAGGCAGGGAAGCGCCAATGATCCCCAGAGCATCGCTGCTAGACATCGC
CGGGAGAGGATCAGTGACAGGCTCAAGATCTTGCAAGAGCTCGTTCCAAACAGCACAAAGGTTGATCTAGTGACG
ATGCTGGAGAAGGCCATCAATTACGTCAAGTTCCTCCAGCTGCAAGTTAAGGTGCTTACGTCGGACGACTACTGG
CCA
Selaginella moelendorfii SmRSLc nucleotide sequence (SEQ ID NO: 45) 
LNTNFKPRARQGSANDPQSIAARHRRERISDRLKILQELVPNSTKVDLVTMLEKAINYVKFLQLQVKVLTSDDYW
P
Selaginella moelendorfii SmRSLd amino acid sequence (SEQ ID NO: 46) 
CTCAACACCAATTTCAAGCCTCGAGCCAGGCAGGGAAGCGCCAATGATCCCCAGAGCATCGCTGCTAGACATCGC
CGGGAGAGGATCAGTGACAGGCTCAAGATCTTGCAAGAGCTCGTTCCAAACAGCACAAAGGTTGATCTAGTGACG
ATGCTGGAGAAGGCCATCAATTACGTCAAGTTCCTCCAGCTGCAAGTTAAGGTGCTTACGTCGGACGACTATTGG
CCA
Selaginella moelendorfii SmRSLd nucleotide sequence (SEQ ID NO: 47) 
LNTDGKPRAKRGSATDPQSIYARQRRERINERLRALQGLVPNGAKVDIVTMLEEAINYVKFLQLQVKLLSSDEYW
MYAPT
Selaginella moelendorfii SmRSLe amino acid sequence (SEQ ID NO: 48) 
CTAAACACCGACGGAAAGCCACGCGCAAAGCGTGGATCTGCCACGGACCCGCAAAGCATCTACGCTCGGCAAAGA
AGAGAAAGGATCAACGAGCGTTTGAGAGCGCTACAAGGACTCGTACCAAACGGAGCGAAGGTTGACATTGTGACG
ATGCTCGAGGAAGCCATCAACTATGTCAAGTTTTTGCAGCTGCAAGTAAAGCTGCTCAGCTCGGACGAGTATTGG
ATGTACGCCCCCACA
Selaginella moelendorfii SmRSLe nucleotide sequence (SEQ ID NO: 49) 
LNTNGKPRAKRGSATDPQSVYARHRRERINERLKTLQHLVPNGAKVDIVTMLEEAIHYVKFLQLQVNMLSSDEYW
IYAPT
Selaginella moelendorfii SmRSLf amino acid sequence (SEQ ID NO: 50) 
CTCAACACGAATGGCAAGCCCAGAGCAAAGCGTGGATCTGCAACAGATCCCCAAAGCGTTTACGCAAGGCACCGG
AGAGAGAGGATCAACGAGAGGCTCAAAACTTTACAACACCTTGTTCCAAATGGTGCAAAGGTTGACATAGTGACA
ATGCTTGAAGAAGCAATACATTACGTGAAGTTTCTACAGCTGCAAGTCAACATGTTAAGCTCTGATGAGTACTGG
ATTTATGCACCCACA
Selaginella moelendorfii SmRSLf nucleotide sequence (SEQ ID NO: 51) 
LNTNGKPRAKRGSATDPQSVYARHRRERINERLKTLQHLVPNGAKVDIVTMLEEAIHYVKFLQLQVNMLSSDEYW
TYAPT
Selaginella moelendorfii SmRSLg amino acid sequence (SEQ ID NO: 52) 
CTCAACACGAATGGCAAGCCCCGAGCAAAGCGTGGATCTGCAACAGATCCCCAAAGCGTTTATGCAAGGCACCGG
AGAGAGAGGATCAACGAGAGGCTCAAAACTTTACAACACCTTGTTCCAAATGGTGCAAAGGTTGACATTGTGACA
ATGCTTGAAGAAGCAATACATTACGTGAAGTTTCTACAGCTGCAAGTCAACATGTTAAGCTCTGATGAGTACTGG
ACTTATGCACCCACA
Selaginella moelendorfii SmRSLg nucleotide sequence (SEQ ID NO: 53) 
LNTDGKPRAKRGSATDPQSIYARQRRERINERLRALQGLVPNGAKVDIVTMLEEAINYVKFLQLQVKLLSSDEYW
MYAPT
Selaginella moelendorfii SmRSLh amino acid sequence (SEQ ID NO: 54) 
CTAAACACCGACGGAAAGCCACGCGCAAAGCGTGGATCTGCCACGGACCCGCAAAGTATCTACGCTCGGCAAAGA
AGAGAAAGGATCAACGAGCGTTTGAGAGCGCTACAAGGACTCGTACCAAACGGAGCGAAGGTTGACATTGTGACG
ATGCTCGAGGAAGCCATCAACTATGTCAAGTTTTTGCAGCTGCAAGTAAAGCTGCTCAGCTCGGACGAGTATTGG
ATGTACGCCCCCACA
Selaginella moelendorfii SmRSLh nucleotide sequence (SEQ ID NO: 55) 
MMAAQASSKRGMLLPREAVLYDDEPSMPLEILGYHGNGVGGGGCVDADYYYSWSGSSSSSSSSVLSFDQAAVGGS
GGGCARQLAFHPGGDDDDCAMWMDAAAGAMVENTSVVAGGGNNYCHRLQFHGGAAGFGLASPGSSVVDNGLEIHE
SNVSKPPPPAAKKRACPSGEARAAGKKQCRKGSKPNKAASASSPSPSPSPSPSPNKEQPQSAAAKVRRERISERL
KVLQDLVPNGTKVDLVTMLEKAINYVKFLQLQVKVLATDEFWPAQGGKAPELSQVKDALDAILSSQHPNK*
Rice (Oryza sativa subsp. Japonica) OsRSLa amino acid sequence (SEQ ID NO: 56; LOC_Os01g02110.1 11971.m06853) 
ATGATGGCAGCTCAGGCAAGCAGCAAGCGCGGCATGCTGCTGCCACGGAGGCGGTGCTCTACGACGACGAGCCC
TCCATGCCGCTGGAGATCTTGGGCTACCACGGCAATGGCGTCGGCGGCGGTGGCTGCGTTGACGCCGATTACTAC
TACAGCTGGTCGGGGTCCAGCTCCAGCTCCAGCTCGTCGGTGCTCAGCTTTGACCAGGCGGCGGTCGGCGGCAGC
GGCGGCGGCTGCGCCCGGCAGCTGGCTTTCCATCCCGGCGGCGACGACGACGACTGCGCCATGTGGATGGACGCC
GCCGCCGGCGCCATGGTCGAGAACACGTCTGTCGTCGCCGGCGGCGGCAACAACTACTGTCATCGCCTGCAGTTC
CACGGCGGCGCCGCCGGTTTCGGACTCGCGAGCCCAGGCTCGTCGGTCGTTGACAACGGCCTCGAAATCCACGAG
AGCAACGTCAGCAAGCCGCCACCGCCGGCAGCCAAGAAGCGCGCATGCCCGAGCGGCGAGGCGAGAGCAGCGGGG
AAGAAGCAGTGCAGGAAAGGGAGCAAGCCAAACAAGGCTGCTTCTGCTTCTTCTCCTTCTCCTTCTCCTTCTCCT
TCTCCTTCTCCTAACAAGGAACAACCTCAAAGCGCCGCTGCAAAGGTAAGAAGAGAGCGGATCAGTGAGAGGCTC
AAAGTTCTTCAGGATCTCGTGCCTAATGGCACAAAGGTAGACTTGGTCACCATGCTAGAAAAGGCGATCAACTAC
GTCAAATTCCTCCAGCTGCAAGTGAAGGTTTTGGCTACTGATGAGTTCTGGCCGGCACAAGGAGGGAAAGCACCA
GAGCTCTCTCAAGTCAAGGACGCCTTGGACGCCATCCTATCTTCTCAGCATCCAAACAAATGA
Rice OsRSLa nucleotide sequence (SEQ ID NO: 57; LOC_Os01g02110.1 11971.m06853) 
MRMALVRERAMVYGGGCDAEAFGGGFESSQMGYGHDALLDIDAAALFGGYEAAASAGCALVQDGAAGWAGAGASS
SVLAFDRAAQAEEAECDAWIEAMDQSYGAGGEAAPYRSTTAVAFDAATGCFSLTERATGGGGGAGGRQFGLLFPS
TSGGGVSPERAAPAPAPRGSQKRAHAESSQAMSPSKKQCGAGRKAGKAKSAPTTPTKDPQSLAAKNRRERISERL
RILQELVPNGTKVDLVTMLEKAISYVKFLQLQVKVLATDEFWPAQGGKAPEISQVKEALDAILSSSSPLMGQLMN
*
Rice OsRSLb amino acid sequence (SEQ ID NO: 58; LOC_Os02g48060.1 11972.m09840)

ATGCGCATGGCGCTGGTGCGGGAGCGCGCGATGGTGTACGGTGGAGGGTGCGACGCCGAGGCGTTCGGCGGCGGG
TTCGAGTCGTCCCAGATGGGGTACGGCCACGACGCGCTGCTCGACATCGACGCGGCGGCGCTGTTCGGGGGGTAC
GAGGCGGCCGCCAGCGCCGGGTGCGCCCTCGTGCAGGACGGCGCCGCGGGGTGGGCGGCGCGGCGCGTCGTCC
TCGGTGCTGGCGTTCGACCGCGCCGCTCAGGCGGAGGAGGCCGAGTGCGACGCGTGGATCGAAGCCATGGACCAG
AGCTACGGCGCCGGCGGCGAGGCGGCGCCGTACCGGTCGACGACGGCCGTCGCCTTCGACGCGGCCACCGGCTGC
TTCAGCCTGACGGAGAGAGCCACCGGCGGCGGCGGCGCGGGTGGGCGGCAGTTCGGGCTGCTGTTCCCGAGC
ACGTCGGGCGGCGGCGTCTCCCCCGAACGCCGCGCCGGCGCCGGCGCCCCGCGGCTCGCAGAAGCGGCCCAC
GCGGAGTCGTCGCAGGCCATGAGCCCTAGCAAGAAGCAGTGCGGCGCCGGCAGGAAGGCGGGCAAGGCCAAGTCG
GCGCCGACCACCCCAACCAAGGACCCGCAAAGCCTCGCGGCCAAGAATCGGCGCGAGAGGATCAGCGAGCGGCTG
CGGATCCTGCAGGAGCTCGTGCCCAACGGCACCAAGGTCGACCTCGTCACCATGCTCGAGAAGGCCATCAGCTAC
GTCAAGTTCCTCCAGCTTCAAGTCAAGGTTCTTGCGACGGACGAGTTCTGGCCGGCGCAGGGAGGGAAGGCGCCG
GAGATATCCCAGGTGAAGGAGGCGCTCGACGCCATCTTGTCGTCGTCGTCGCCGCTGATGGGACAACTCATGAAC
TGA

Rice OsRSLb nucleotide sequence (SEQ ID NO: 59; LOC_Os02g48060.1 11972.m09840) 
MAMVAGDEAMSVPWHDVGVVVDPEAAGTAPFDAGAGYVPSYGQCQYYYYYDDHHHPCSTELIHAGDAGSAVAVA
YDGVDGWVHAAAAATSPSSSSALTFDGHGAEEHSAVSWMDMDMDAHGAAPPLIGYGPTAATSSPSSCFSSGGSGD
SGMVMVTTTTPRSAAASGSQRRARPPPSPLQGSELHEYSKKQRANNKETQSSAAKSRRERISERLRALQELVPSG
GKVDMVTMLDRAISYVKFMQMQLRVLETDAFWPASDGATPDISRVKDALDAIILSSSSPSQKASPPRSG*
Rice OsRSLc amino acid sequence (SEQ ID NO: 60; LOC_Os06g30090.1 11976.m07553) 
ATGGCTATGGTGGCCGGCGACGAGGCGATGTCAGTGCCATGGCACGACGTCGGCGTCGTCGACCCCGAGGCG
GCCGGGACGGCGCCGTTCGACGCCGGCGCCGGCTATGTCCCATCGTACGGTCAGTGCCAATACTACTACTACTAC
GACGACCACCACCACCACCCGTGCAGCACGGAGCTGATCCACGCGGGCGACGCTGGCAGTGCGGTTGCGGTTGCG
TACGACGGCGTCGACGGCTGGGTTCACGCCGCCGCCGCAGCCACCTCCCCGTCCTCGTCATCTGCGCTCACCTTC
GATGGTCACGGCGCCGAGGAGCACAGCGCAGTGTCGTGGATGGACATGGACATGGACGCGCACGGCGCCGCGCCT
CCCCTAATCGGCTACGGCCCGACGGCGGCGACCTCCTCCCCCTCCTCCTGCTTCAGCTCCGGCGGCTCCGGCGAC
AGCGGCATGGTGATGGTGACCACCACCACCCCGAGGAGCGCCGCCGCCTCTGGTTCGCAGAGGCGGGCACGCCCG
CCGCCGTCGCCGTTGCAGGGATCAGAGCTGCACGAGTACTCCAAGAAGCAGCGCGCCAACAACAAGGAGACACAG
AGCTCAGCTGCCAAGAGCCGGCGGGAGAGGATCAGCGAGCGGCTGAGGGCGCTGCAGGAGCTGGTGCCGAGCGGC
GGGAAGGTGGACATGGTGACCATGCTGGACAGGGCCATCAGCTACGTCAAGTTCATGCAGATGCAGCTCAGGGTG
CTGGAGACCGACGCGTTCTGGCCGGCGTCCGACGGCGCCACGCCGGACATCTCCCGGGTCAAGGACGCGCTCGAC
GCCATCATCCTCTCCTCGTCCTCGCCCTCGCAAAAGGCTTCTCCTCCTCGGTCGGGCTAG
Rice OsRSLc nucleotide sequence (SEQ ID NO: 61; LOC_Os06g30090.1 11976.m07553) 
MEDSEAMAQLLGVQYFGNDQEQQQPAAAAPPAMYWPAHDAADQYYGSAPYCYMQQQQHYGCYDGGAMVAGGDFFV
PEEQLVADPSFMVDLNLEFEDQHGGDAGGAGSSAAAAAAATKMTPACKRKVEDHKDESCTDNVARKKARSTAATV
VQKKGNKNAQSKKAQKGACSRSSNQKESNGGGDGGNVQSSSTNYLSDDDSLSLEMTSCSNVSSASKKSSLSSPAT
GHGGAKARAGRGAATDPQSLYARKRRERINERLKILQNLIPNGTKVDISTMLEEAVHYVKFLQLQIKLLSSDDMW
MFAPIAYNGVNVGLDLKISPPQQQ*
Rice OsRSLd amino acid sequence (SEQ ID NO: 62; LOC_Os03g10770.1 11973.m06529) 
ATGGAGGACTCGGAGGCGATGGCGCAGCTGCTCGGCGTGCAGTACTTCGGCAATGACCAGGAGCAGCAGCAGCCG
GCGGCGGCGGCGCCGCCGGCGATGTACTGGCCGGCGCACGACGCGGCCGACCAGTACTACGGCTCGGCGCCATAC
TGCTACATGCAGCAGCAGCAGCATTACGGGTGCTACGACGGCGGCGCGATGGTGGCCGGCGGCGACTTCTTCGTG
CCGGAGGAGCAGCTGGTGGCCGACCCGAGCTTCATGGTGGACCTGAACCTCGAGTTCGAGGACCAGCACGGCGGC
GATGCTGGCGGCGCTGGGAGCAGCGCCGCCGCCGCCGCCGCCACCAAGATGACACCGGCGTGCAAGAGGAAG
GTTGAGGATCACAAGGATGAGAGCTGCACGGACAACGTCGCGAGGAAGAAGGCGCGCTCCACGGCAGCAACAGTG
GTGCAGAAGAAGGGTAATAAGAACGCGCAGTCAAAGAAGGCGCAGAAGGGCGCGTGCAGCCGGAGCAGCAACCAG
AAGGAGAGCAATGGCGGCGGCGACGGCGGCAATGTGCAGAGCTCGAGCACCAACTACCTCTCTGATGACGACTCG
CTGTCGCTGGAGATGACTTCGTGCAGCAACGTGAGCTCGGCGTCCAAGAAGTCGTCGTTGTCATCGCCGGCGACC
GGGCACGGCGGCGCGAAGGCGAGGGCCGGGCGCGGGGCGGCGACCGATCCGCAAAGCCTCTATGCCAGGAAGAGG
AGAGAAAGGATCAATGAACGGCTAAAGATACTGCAGAATCTTATCCCAAATGGAACCAAGGTGGACATCAGCACG
ATGCTTGAAGAAGCAGTTCACTACGTCAAGTTCTTGCAGCTCCAAATCAAGCTTCTGAGCTCGGATGATATGTGG
ATGTTCGCGCCGATCGCGTACAACGGGGTCAACGTCGGGCTCGACCTCAAGATCTCTCCACCGCAGCAGCAATGA
Rice OsRSLd nucleotide sequence (SEQ ID NO: 63; LOC_Os03g10770.1 11973.m06529) 
MESGGVIAEAGWSSLDMSSQAEESEMMAQLLGTCFPSNGEDDHQELPWSVDTPSAYYLHCNGGSSSAYSSTTSS
NSASGSFTLIAPRSEYEGYYVSDSNEAALGISIQEQGAAQFMDAILNRNGDPGFDDLADSSVNLLDSIGASNKRK
IQEQGRLDDQTKSRKSAKKAGSKRGKKAAQCEGEDGSIAVTNRQSLSCCTSENDSIGSQESPVAAKSNGKAQSGH
 
RSATDPQSLYARKRRERINERLKILQNLVPNGTKVDISTMLEEAMHYVKFLQLQIKLLSSDEMWMYAPIAYNGMN
IGIDLNLSQH*
Rice OsRSLe amino acid sequence (SEQ ID NO: 64; LOC_Os03g42100.1 11973.m09268) 
ATGGAGTCCGGAGGGGTGATCGCGGAGGCGGGGTGGAGCTCGCTCGACATGTCGTCGCAGGCCGAGGAGTCGGAG
ATGATGGCGCAGCTGCTTGGAACCTGCTTCCCCTCCAATGGCGAGGATGATCATCACCAAGAGCTTCCTTGGTCG
GTTGACACCCCCAGTGCCTACTACCTCCATTGCAATGGAGGTAGCTCAAGTGCATACAGCTCTACCACTAGCAGC
AACAGTGCTAGTGGTAGCTTCACTCTCATTGCACCAAGATCTGAGTATGAGGGGTACTATGTGAGTGACTCTAAT
GAGGCGGCCCTCGGGATCAGCATCCAGGAGCAAGGTGCAGCTCAGTTCATGGATGCCATTCTCAACCGGAACGGC
GATCCGGGCTTCGATGATCTCGCTGACTCGAGCGTTAATCTGCTGGATTCCATCGGCGCTTCTAACAAGAGAAAG
ATTCAGGAGCAAGGCAGGCTAGATGACCAAACGAAAAGTAGGAAATCTGCGAAGAAGGCTGGCTCGAAGCGGGGA
AAGAAGGCGGCGCAATGTGAAGGTGAAGATGGCAGCATTGCTGTCACCAACAGGCAAAGCTTGAGCTGCTGCACC
TCTGAAAATGATTCGATTGGTTCTCAAGAATCTCCTGTTGCTGCTAAGTCGAATGGCAAGGCTCAATCTGGCCAT
CGGTCAGCAACCGATCCCCAGAGCCTCTATGCAAGGAAAAGAAGAGAGAGGATCAATGAGAGGCTCAAGATTCTG
CAGAACCTTGTACCAAATGGAACCAAAGTAGATATCAGCACTATGCTTGAAGAGGCAATGCATTACGTGAAGTTC
TTGCAGCTTCAAATCAAGCTCCTCAGCTCTGATGAAATGTGGATGTACGCACCGATTGCTTACAACGGGATGAAC
ATCGGGATCGATTTGAACCTCTCTCAGCATTGA
Rice OsRSLe nucleotide sequence (SEQ ID NO: 65; LOC_Os03g42100.1 11973.m09268) 
MDARCANIWSSADARSEESEMIDQLKSMFWSSTDAEINFYSPDSSVNSCVTTSTMPSSLFLPLMDDEGFGTVQLM
HQVITGNKRMFPMDEHFEQQQKKPKKKTRTSRSVSSSSTITDYETSSELVNPSCSSGSSVGEDSIAATDGSVVLK
QSDNSRGHKQCSKDTQSLYAKRRRERINERLRILQQLVPNGTKVDISTMLEEAVQYVKFLQLQIKLLSSDDTWMF
APLAYNGMNMDLGHTLAENQE*
Rice OsRSLf amino acid sequence (SEQ ID NO: 66; LOC_Os11g41640.1 11981.m08005) 
ATGGATGCAAGGTGTGCAAACATCTGGAGCTCTGCTGATGCAAGGAGTGAGGAATCTGAGATGATTGATCAACTA
AAGTCCATGTTCTGGAGCAGCACTGATGCTGAAATCAACTTTTATTCTCCTGACAGTAGTGTAAATTCTTGTGTC
ACAACTAGCACAATGCCTAGCAGCTTGTTTCTTCCTCTGATGGATGATGAGGGATTTGGCACAGTGCAATTGATG
CATCAGGTCATCACTGGGAACAAGAGGATGTTCCCCATGGATGAGCACTTTGAGCAGCAGCAGAAGAAGCCGAAG
AAGAAAACCCGAACTTCTCGCTCGGTATCAAGTAGTTCAACCATTACTGACTATGAGACTAGCTCTGAACTTGTC
AATCCTAGCTGTTCCTCCGGGAGCAGCGTCGGAGAGGATTCAATTGCTGCAACTGATGGATCTGTAGTGCTGAAA
CAAAGTGACAATTCAAGAGGCCATAAGCAGTGCTCCAAGGATACACAAAGCCTCTATGCTAAGAGGAGAAGGGAA
AGGATTAATGAGAGACTGAGAATACTTCAGCAGCTTGTTCCCAATGGCACTAAAGTTGACATCAGCACAATGCTG
GAGGAAGCAGTTCAGTATGTCAAGTTTTTGCAGTTGCAAATAAAGCTATTGAGCTCTGACGACACATGGATGTTT
GCGCCCCTAGCCTATAATGGCATGAACATGGATCTCGGTCATACTCTTGCTGAAAACCAAGAATGA
Rice OsRSLf nucleotide sequence (SEQ ID NO: 67; LOC_Os11g41640.1 11981.m08005) 
MECSSFEAICNESEMIAHLQSLFWSSSDADPCFGSSSFSLISSEGYDTMTTEFVNSSTNVCFDYQDDSFVSAEET
TIGNKRKVQMDTENELMTNRSKEVRTKMSVSKACKHSVSAESSQSYYAKNRRQRINERLRILQELIPNGTKVDIS
TMLEEAIQYVKFLHLQIKLLSSDEMWMYAPLAFDSGNNRLYQNSLSQE*
Rice OsRSLg amino acid sequence (SEQ ID NO: 68; LOC_Os12g32400.1 11982.m07043) 
ATGGAATGCAGCTCCTTTGAAGCAATCTGCAATGAGTCGGAGATGATTGCGCATTTGCAGTCATTGTTCTGGAGC
AGCAGCGATGCTGATCCTTGTTTTGGTAGCTCATCATTTTCTCTCATCAGTAGTGAGGGCTACGACACAATGACC
ACAGAGTTTGTGAATAGCAGCACAAATGTATGTTTTGATTACCAAGATGATAGCTTCGTTTCAGCAGAGGAGACT
ACCATTGGTAACAAGAGAAAAGTTCAGATGGATACTGAGAATGAGCTGATGACGAACCGCAGCAAGGAAGTTCGC
ACCAAGATGTCGGTGTCAAAAGCATGCAAACATTCTGTTTCTGCAGAGAGCTCACAGTCTTATTATGCAAAGAAC
 
AGGAGACAGAGGATCAATGAGAGATTGAGAATACTGCAAGAACTGATCCCTAATGGAACAAAAGTTGACATCAGC
ACAATGTTGGAGGAAGCAATTCAGTATGTCAAGTTTCTACACCTGCAAATCAAGCTCTTGAGCTCTGATGAAATG
TGGATGTATGCGCCCCTTGCTTTTGACAGTGGTAACAACAGGCTCTATCAGAACTCTCTGTCACAAGAGTAG
Rice OsRSLg nucleotide sequence (SEQ ID NO: 69; LOC_Os12g32400.1 11982.m07043) 
MEGGGLIADMSWTVFDLPSHSDESEMMAQLFSAFPIHGEEEGHEQLPWFDQSSNPCYYSCNASSTAYSNSNASSI
PAPSEYEGYCFSDSNEALGVSSSIAPHDLSMVQVQGATEFLNVIPNHSLDSFGNGELGHEDLDSVSGTNKRKQSA
EGEFDGQTRGSKCARKAEPKRAKKAKQTVEKDASVAIPNGSCSISDNDSSSSQEVADAGATSKGKSRAGRGAATD
PQSLYARKRRERINERLKTLQNLVPNGTKVDISTMLEEAVHYVKFLQLQIKLLSSDEMWMYAPIAYNGMNIGLDL
NIDT*
Rice OsRSLh amino acid sequence (SEQ ID NO: 70; LOC_Os12g39850.1 11982.m07769) 
ATGGAGGGTGGAGGACTGATCGCCGATATGAGCTGGACCGTCTTCGACTTGCCATCGCACAGCGATGAGTCGGAG
ATGATGGCGCAGCTCTTCAGTGCATTCCCCATCCATGGTGAGGAGGAAGGCCATGAGCAGCTCCCATGGTTTGAT
CAATCTTCCAATCCATGCTACTATAGCTGCAATGCTAGCAGCACTGCATACAGCAACAGCAATGCTAGTAGCATT
CCTGCTCCATCTGAGTATGAAGGATACTGCTTCAGTGACTCAAATGAGGCCCTGGGTGTCAGCTCCAGCATTGCA
CCACATGACCTGAGCATGGTCCAGGTGCAAGGTGCAACTGAGTTTCTGAATGTGATCCCAAACCATTCCCTTGAT
TCATTCGGTAATGGCGAGCTGGGCCACGAGGATCTTGATTCGGTTAGTGGGACTAACAAGAGAAAACAGTCGGCA
GAAGGAGAATTTGATGGCCAAACAAGAGGTTCAAAATGCGCGAGAAAGGCTGAACCGAAGCGAGCGAAGAAGGCC
AAGCAAACTGTGGAGAAGGATGCAAGTGTTGCCATCCCAAATGGGAGCTGTTCCATTTCTGACAATGATTCCAGT
TCATCCCAGGAGGTTGCAGATGCTGGTGCTACTTCGAAAGGCAAATCCCGGGCTGGCCGCGGAGCAGCCACTGAT
CCCCAGAGCCTCTATGCAAGGAAAAGGAGAGAGAGGATCAATGAGAGGCTCAAGACACTTCAGAACCTTGTGCCC
AATGGCACCAAAGTTGATATCAGCACCATGCTTGAGGAGGCAGTCCACTATGTGAAGTTCCTGCAGCTTCAGATC
AAGCTCCTCAGCTCCGATGAAATGTGGATGTATGCGCCAATTGCGTACAACGGGATGAACATTGGGCTCGATCTG
AACATTGATACATGA
Rice OsRSLh nucleotide sequence (SEQ ID NO: 71; LOC_Os12g39850.1 11982.m07769) 
MAQFLGAHGDHCFTYEQMDESMEAMAAMFLPGLDTDSNSSSGCLNYDVPPQCWPQHGHSSSVTSFPDPAHSYGSF
EFPVMDPFPIADLDAHCAIPYLTEDLISPPHGNHPSARVEEATKVVTPVATKRKSSAAMTASKKSKKAGKKDPIG
SDEGGNTYIDTQSSSSCTSEEGNLEGNAKPSSKKMGTRANRGAATDPQSLYARKRRERINERLRILQNLVPNGTK
VDISTMLEEAVQYVKFLQLQIKLLSSDDTWMYAPIAYNGVNISNIDLNISSLQK*
Rice OsRSLi amino acid sequence (SEQ ID NO: 72; LOC_Os07g39940.1 11977.m08236) 
ATGGCGCAGTTTCTTGGAGCTCATGGTGATCACTGCTTCACCTACGAGCAAATGGATGAGTCCATGGAGGCAATG
GCAGCGATGTTCTTGCCTGGCCTTGACACCGACTCCAATTCTTCTTCTGGTTGTCTCAACTACGATGTGCCTCCA
CAATGCTGGCCTCAGCATGGCCATAGCTCTAGCGTCACCAGCTTCCCTGATCCAGCTCATAGCTATGGAAGCTTT
GAGTTCCCGGTCATGGATCCGTTCCCGATCGCCGATCTCGACGCGCATTGCGCCATCCCCTACCTTACTGAGGAT
CTGATCAGCCCTCCACATGGCAACCATCCATCAGCAAGAGTGGAAGAAGCTACAAAGGTTGTTACACCAGTGGCT
ACCAAGAGGAAGTCTAGTGCTGCCATGACGGCATCAAAGAAGAGCAAGAAGGCTGGCAAAAAAGATCCTATTGGC
AGCGACGAAGGCGGCAACACCTACATTGATACGCAAAGTTCTAGCAGTTGCACCTCAGAGGAAGGAAACCTGGAG
GGCAACGCGAAGCCGAGCTCGAAGAAGATGGGTACTAGGGCCAACCGTGGGGCGGCAACCGATCCCCAGAGTCTC
TATGCAAGGAAGAGGAGAGAGAGGATCAATGAAAGATTGAGGATCCTGCAGAACTTGGTTCCCAATGGAACAAAG
GTTGACATCAGTACAATGCTGGAGGAAGCAGTGCAGTATGTCAAATTTTTGCAACTTCAGATTAAGTTGCTAAGC
TCTGATGACACGTGGATGTATGCACCAATCGCTTACAATGGAGTCAACATCAGCAATATTGATCTGAACATCTCT
TCTCTGCAAAAATAA
Rice OsRSLi nucleotide sequence (SEQ ID NO: 73; LOC_Os07g39940.1 11977.m08236) 
MALAKDRMGSVQTCPYNGNVMGDFSSMGSYGFDEYQKVAFYEEGNSTFEKTSGLMIKNLAMTSSPSSLGSPSSAI
SGELVFQATDHQAEEAHSLISFKGIGFDNIMHNNGSLLSFEQSSRVSQTSSQKDDYSAWEGNLSYNYQWNEMNPK
CNTSPRLMEDFNCFQRAGNFISMTGKENHGDWLYAESTIVADSIQDSATPDASSFHKRPNMGESMQALKKQCNNA
TKKPKPKSAAGPAKDLQSIAAKNRRERISERLKVLQDLVPNGSKVDLVTMLEKAISYVKFLQLQVKVLATDELWP
VQGGKAPDISQVKEAIDALLSSQTKDGNSSSSPK*
Populus trichocarpa PtRSLa amino acid sequence (SEQ ID NO: 74) 
ATGGCACTTGCCAAGGACCGTATGGATCGGTTCAAACTTGCCCCTATAATGGAAATGTGATGGGGATTTTTCC
TCCATGGGGTCTTACGGATTTGATGAATATCAGAAGGTAGCATTTTATGAAGAGGGAAATAGCACCTTTGAGAAA
ACCAGTGGGCTTATGATCAAGAATTTAGCTATGACCTCTTCTCCTTCTTCTCTTGGCAGTCCGAGCAGCGCGATT
TCTGGTGAATTAGTGTTTCAGGCTACTGACCATCAAGCTGAGGAAGCTCATTCTTTGATCAGCTTCAAAGGTATC
GGATTCGATAACATCATGCATAATAATGGATCTTTGCTTAGCTTTGAGCAAAGTAGTAGGGTTTCTCAAACTAGT
AGCCAGAAAGATGACTACTCAGCCTGGGAGGGTAATTTGAGTTACAACTACCAGTGGAACGAAATGAATCCAAAA
TGTAACACAAGTCCTCGGTTGATGGAAGATTTTAATTGCTTTCAAAGAGCTGGCAACTTCATTTCCATGACTGGA
AAGGAAAATCATGGTGATTGGTTATACGCTGAATCCACAATTGTTGCTGATAGCATTCAGGATTCTGCAACACCA
GATGCCAGCAGCTTCCATAAGCGTCCTAATATGGGAGAGAGTATGCAGGCTCTAAAGAAGCAATGCAACAATGCA
ACAAAAAAGCCAAAACCGAAGTCCGCAGCAGGTCCAGCTAAGGATCTACAGAGTATTGCTGCCAAGAATCGACGA
GAGAGGATTAGCGAGAGGCTTAAGGTATTGCAGGATTTAGTCCCTAATGGCTCAAAGGTTGATTTGGTTACTATG
CTAGAGAAAGCCATTAGTTATGTTAAGTTTCTTCAATTGCAAGTAAAGGTGTTAGCCACTGATGAATTATGGCCA
GTTCAAGGTGGTAAAGCTCCTGATATTTCTCAAGTAAAGGAAGCCATCGATGCCCTACTCTCATCTCAGACTAAA
GACGGAAACTCAAGCTCAAGCCCAAAGTAA
Populus trichocarpa PtRSLa nucleotide sequence (SEQ ID NO: 75) 
MALAKDRMDSVQTCALYGNVMGDLSSLGPNYRFDEEGDRNFEKNSALMIKNLAMSPSPPSLGSPSSANSGELVFQ
ATDNQVEEAHSLINFKGTGFDSIMHANGSLISFEQSNRVSQTSSHKDDYSAWEGNLSCNYQWNQINPKCNANPRL
MEDLNCYQSASNFNSITNSAEKENHGDWLYTHESTIVTDSIPDSATPDASSFHKRPNMGESMQALKKQRDSATKK
PKPKSAGPAKDPQSIAAKNRRERISERLKMLQDLVPNGSKVDLVTMLEKAISYVKFLQLQVKVLATDEFWPVQGG
KAPDISQVKGAIDATLSSQTKDRNSNSSSK*
Populus trichocarpa PtRSLb amino acid sequence (SEQ ID NO: 76) 
ATGGCACTTGCCAAGGACCGTATGGATTCGGTTCAAACTTGCGCCCTTTATGGAAATGTGATGGGGGATCTTTCC
TCCTTGGGGCCTAATTATAGATTTGATGAAGAGGGAGATAGGAACTTTGAGAAAAATAGTGCGCTTATGATCAAG
AATTTAGCTATGAGCCCTTCTCCTCCTTCTCTTGGCAGTCCAAGCAGTGCAAATTCTGGTGAACTAGTGTTTCAG
GCTACTGACAATCAAGTTGAGGAAGCTCATTCTTTGATCAACTTCAAAGGTACCGGATTTGATAGTATCATGCAT
GCTAATGGATCTTTGATTAGCTTTGAGCAAAGTAATAGGGTTTCTCAAACTAGTAGTCACAAAGATGACTACTCT
GCTTGGGAGGGTAATTTGAGTTGCAATTACCAGTGGAACCAAATCAATCCAAAATGTAACGCAAATCCTCGGTTG
ATGGAAGATCTTAATTGCTATCAAAGTGCAAGCAACTTCAACTCCATAACCAACAGTGCTGAAAAGGAAAACCAT
GGTGATTGGTTATACACTCATGAATCCACAATTGTTACTGATAGCATTCCCGATTCTGCAACACCAGATGCCAGC
AGCTTCCATAAGCGTCCCAATATGGGAGAGAGTATGCAGGCTCTAAAGAAGCAACGCGACAGCGCCACAAAAAAG
CCGAAACCCAAGTCTGCTGGTCCAGCTAAGGATCCACAAAGTATTGCTGCCAAGAATCGACGAGAGCGGATTAGC
GAGCGCCTTAAGATGTTGCAGGATTTAGTCCCTAACGGCTCCAAGGTTGATTTGGTTACTATGCTAGAGAAAGCC
ATTAGTTATGTTAAGTTTCTTCAATTGCAAGTAAAGGTGTTGGCCACTGATGAATTCTGGCCAGTTCAAGGTGGT
AAAGCTCCTGATATTTCTCAAGTAAAGGGAGCCATTGATGCCACACTCTCATCTCAGACTAAAGACAGAAATTCA
AACTCAAGCTCAAAGTGA
Populus trichocarpa PtRSLb nucleotide sequence (SEQ ID NO: 77) 
MAEGEWSSLGGMYTSEEADFMAQLLGNCPNQVDSSSNFGVPSSFWPNHEPTTDMEGANECLFYSLDFANINLHHF
SQGSSSYSGGSGILFPNTSQDSYYMSDSHPILANNNSSMSMDFCMGDSYLVEGDDCSNQEMSNSNEEPGGNQTVA
ALPENDFRAKREPEMPASELPLEDKSSNPPQISKKRSRNSGDAQKNKRNASSKKSQKVASTSNNDEGSNAGLNGP
ASSGCCSEDESNASHELNRGASSSLSSKGTATLNSSGKTRASRGAATDPQSLYARKRRERINERLRILQTLVPNG
TKVDISTMLEEAVQYVKFLQLQIKLLSSEDLWMYAPIAYNGMDIGLDHLKVTAP*
Populus trichocarpa PtRSLc amino acid sequence (SEQ ID NO: 78) 
ATGGCAGAGGGAGAGTGGAGTTCTCTTGGTGGAATGTACACTAGTGAGGAGGCTGATTTCATGGCACAGTTGCTT
GGTAACTGTCCTAATCAGGTTGATTCAAGTTCAAACTTTGGAGTTCCATCTAGTTTCTGGCCTAACCACGAACCA
ACAACGGACATGGAAGGGGCTAATGAATGTTTATTTTATTCTTTGGATTTTGCTAATATTAATTTGCACCATTTT
TCACAAGGGAGTAGTAGTTATAGTGGTGGCAGTGGCATTCTTTTTCCCAACACAAGCCAAGATAGCTACTACATG
AGTGATTCTCATCCAATTTTGGCTAACAATAATAGCTCAATGTCAATGGATTTTTGCATGGGAGACTCATATCTC
GTTGAAGGCGATGACTGCTCAAACCAAGAAATGAGCAATAGCAATGAGGAGCCTGGTGGAAACCAGACTGTAGCT
GCTCTTCCTGAAAACGATTTTCGGGCCAAGAGAGAACCAGAGATGCCAGCTTCTGAACTACCCCTGGAAGACAAA
AGCAGCAACCCACCTCAGATTTCTAAGAAAAGATCACGAAATTCAGGAGATGCTCAAAAGAACAAGAGGAATGCA
AGTTCAAAGAAGAGCCAGAAGGTTGCCTCGACTAGCAACAATGATGAAGGAAGTAATGCTGGCCTTAATGGGCCT
GCCTCAAGCGGTTGCTGCTCAGAGGATGAATCCAATGCCTCTCATGAGCTCAATAGAGGAGCGAGTTCAAGTTTG
AGCTCGAAAGGGACTGCAACTCTCAACTCAAGTGGCAAAACAAGAGCCAGCAGGGGGGCAGCCACTGATCCCCAG
AGTCTCTATGCAAGGAAAAGAAGAGAAAGAATAAATGAGAGGCTGAGAATTCTACAAACCCTTGTCCCCAACGGA
ACAAAGGTTGACATTAGCACAATGCTTGAAGAAGCTGTCCAGTATGTGAAGTTTTTGCAACTCCAAATTAAGCTG
CTAAGCTCTGAGGACTTGTGGATGTATGCGCCTATCGCTTACAACGGATGGACATCGGTCTTGATCATCTGAAG
GTTACCGCACCATGA
Populus trichocarpa PtRSLc nucleotide sequence (SEQ ID NO: 79) 
MEPIGATAEGEWSSLSGMYTSEEADFMEQLLVNCPPNQVDSSSSFGVPSSFWPNHESTMNMEGANECLLYSLDIA
DTNLYHFSQVSSGYSGELSNGNVEESGGNQTVAALPEPESNLQPKRESKMPASELPLEDKSRKPPENSKKRSRRT
GDAQKNKRNVRSKKSQKVASTGNNDEESNGGLNGPVSSGCCSEDESNASQELNGGASSSLSSKGTTTLNSSGKTR
ASKGAATDPQSLYARKRRERINERLRILQNLVPNGTKVDISTMLEEAVQYVKFLQLQIKLLSSEDLWMYAPIAYN
GMDIGLDHLKLTTPRRL*
Populus trichocarpa PtRSLd amino acid sequence (SEQ ID NO: 80) 
ATGGAGCCTATTGGAGCCACTGCGGAGGGAGAGTGGAGTTCTCTTAGTGGAATGTACACAAGTGAGGAGGCTGAT
TTCATGGAACAGTTGCTTGTCAACTGTCCTCCTAATCAGGTTGATTCAAGTTCAAGCTTTGGAGTTCCATCTAGT
TTTTGGCCTAACCATGAATCAACAATGAACATGGAAGGGGCCAATGAATGTTTATTGTATTCTTTGGATATTGCT
GATACTAATCTGTACCATTTTTCACAAGTGAGCAGTGGTTATAGTGGTGAATTGAGCAATGGAAATGTGGAAGAG
TCTGGTGGAAACCAGACTGTAGCTGCTCTTCCTGAACCTGAAAGCAATTTGCAACCCAAGAGAGAATCAAAGATG
CCAGCATCTGAACTACCCCTGGAAGATAAAAGCAGAAAGCCACCTGAGAATTCCAAGAAAAGATCACGACGTACG
GGAGATGCCCAAAAGAACAAGAGGAATGTAAGGTCAAAGAAGAGCCAGAAGGTTGCCTCGACTGGCAACAATGAT
GAAGAAAGCAATGGTGGCCTTAATGGTCCTGTCTCAAGCGGTTGCTGCTCAGAGGATGAATCCAATGCCTCCCAG
GAGCTCAATGGAGGAGCGAGTTCAAGTTTGAGCTCAAAAGGGACAACAACTCTCAACTCAAGTGGCAAAACAAGA
GCCAGTAAGGGGGCAGCCACTGATCCCCAGAGCCTCTATGCAAGGAAAAGAAGAGAAAGAATAAATGAGAGGCTG
AGAATTCTACAAAACCTTGTCCCCAATGGAACAAAGGTTGACATTAGCACAATGCTTGAAGAGGCTGTCCAGTAT
GTGAAGTTTTTGCAACTCCAAATTAAGCTGCTAAGCTCTGAAGACCTGTGGATGTATGCTCCTATCGCGTACAAT
GGTATGGACATCGGTCTTGATCATCTGAAGCTTACCACACCAAGACGATTGTAG
Populus trichocarpa PtRSLd nucleotide sequence (SEQ ID NO: 81) 
MNTQAMEAFRDGELWNFSRMFSMEEPDCTPELLGQCSFLQDTDEGLHFTIPSAFFPAPESDASMAEDESLFYSWH
TPNPNLHFDSQESSNNSNSSSSVFLPYSSHESYFFNDSNPIQATNNNSMSMDIMDEENIGLFMPLFPEIAMAETA
CMNGDMSGDKTGDLDDNLKPAANDVLAKGLQLKRKLDVPEPIANTLDDMKKKARVTRNVQKTRKVGQSKKNQKNA
PDISHDEEESNAGPDGQSSSSCSSEEDNASQDSDSKVSGVLNSNGKTRATRGAATDPQSLYARKRRERINERLKI
LQNLVPNGTKVDISTMLEEAVHYVNFLQLQIKLLSSDDLWMYAPLAYNGIDIGLNQKLSMFL*
Populus trichocarpa PtRSLe amino acid sequence (SEQ ID NO: 82) 
ATGAATACGCAGGCTATGGAAGCCTTTCGTGATGGAGAATTATGGAACTTCAGCAGAATGTTCTCCATGGAAGAG
CCTGATTGCACCCCAGAATTACTTGGTCAGTGCTCTTTTCTTCAGGATACTGATGAAGGATTGCATTTTACAATC
CCATCAGCTTTCTTCCCTGCTCCTGAATCCGACGCGAGCATGGCTGAGGACGAGAGTTTGTTTTATTCTTGGCAT
ACTCCCAACCCCAATTTGCATTTTGATTCTCAAGAAAGTAGTAATAACAGTAATTCTAGCAGTAGTGTATTTCTT
CCCTATTCCAGCCATGAATCCTACTTCTTCAATGATTCTAATCCCATTCAAGCTACGAACAATAACTCTATGTCC
ATGGATATTATGGATGAGGAAAATATTGGCTTGTTTATGCCACTTTTTCCTGAAATTGCAATGGCAGAAACTGCC
TGTATGAATGGAGATATGAGCGGTGACAAAACAGGAGATTTAGATGATAATCTGAAGCCAGCAGCTAATGATGTT
 
CTGGCCAAGGGATTGCAGCTCAAAAGGAAGCTTGATGTTCCAGAACCAATAGCCAACACATTGGACGACATGAAG
AAAAAAGCCCGGGTTACAAGAAATGTGCAAAAGACTAGGAAGGTTGGACAGTCAAAAAAAAATCAGAAGAACGCA
CCAGATATTAGCCATGATGAAGAAGAGAGTAATGCTGGACCAGACGGACAAAGTTCCAGCAGTTGTAGTTCAGAA
GAGGACAATGCCTCTCAGGATTCTGATTCCAAGGTTTCTGGAGTTCTCAATTCCAATGGAAAAACAAGAGCTACT
AGGGGAGCTGCCACAGACCCCCAGAGCCTTTATGCAAGGAAAAGAAGGGAGAGGATAAACGAGAGACTGAAAATC
TTGCAGAATCTTGTCCCTAACGGAACCAAGGTTGATATCAGCACGATGCTAGAAGAGGCAGTCCATTACGTAAAC
TTTTTGCAGCTTCAAATCAAGCTTTTGAGCTCGGATGATCTATGGATGTATGCACCTCTGGCTTACAATGGAATA
GATATTGGACTCAACCAGAAGCTCTCTATGTTTCTATGA
Populus trichocarpa PtRSLe nucleotide sequence (SEQ ID NO: 83) 
MAQESTWSSFDATMLAEEESRMIAQLLSNYQCFGEQDRDVGCCELPPSSCCSSHAADSCYCWSANENSNPGLCYW
SQSGDESDGAHAIGTVPVFTNHCLVGDQVAVNQTLSIHEPTAAHAEMPKRKIESHASEDDFRRQSSKKKLQAPTN
ALKSVKKARPGRNQKSIVCGDEEENNARSSGRSCCSYSSEEDSQAFQADLNAKTRSNRWPATDPQSLYAKQRRER
INARLRTLQNLVPNGTKVDISTMLEEAVRYVKFLQLQIKLLSSDELWMYAPVVHSGMIDGQVNSEIFVSANTRNE
WF*
Musa acuminata MaRSLa amino acid sequence (SEQ ID NO: 84; GI102139852, ABF70010.1) 
atggctcaggagtcaacttggagctcgtttgatgctacaatgcttgctgaggaggagtcccgaatgatcgcacaa
ttgctcagcaactaccagtgttttggcgagcaagatcgagatgttggatgctgtgaactcccgccatcgtcttgt
tgttcttctcatgcagctgattcatgttactgttggtcagcaaatgagaacagtaacccgggtttgtgctactgg
tctcagagtggagatgaatccgatggagcacatgcaatcggcactgtgccggtcttcacgaaccattgcttggtg
ggagatcaagtcgctgtgaatcaaactttgagcattcacgaacctactgctgctcatgcagagatgccaaagcgc
aagatagagtctcatgcttctgaagatgatttccgtcgtcaaagttctaagaaaaagcttcaggctccgacgaat
gctctgaagagcgtgaagaaggcacgacctgggaggaaccagaagagcattgtgtgtggtgatgaggaagagaac
aatgccaggagcagtggccggagttgctgcagctacagctctgaggaagactcacaagctttccaggctgatctt
aatgcaaaaacacgatcgaatcgatggccagccacagatcctcaaagcctctatgcaaagcaaagaaggaaaga
atcaatgctagattgaggacattgcagaacctggtgcctaatggaactaaagttgacattagcacaatgctcgaa
gaagctgttcgttacgtcaagttcttgcagctgcagataaagcttttgagctcggatgagctgtggatgtacgct
cctgttgtccacagtgggatgattgatggccaagtcaactcagagatatttgtgtctgcaaatactcgtaatgag
tggttctga
*Musa acuminata* MaRSLa nucleotide sequence (SEQ ID NO: 85). 
MEPIGTFPEGEWDFFRKMFASEDHEYYSQQFLDQNSLLLGENDGLNNGTQSTFCTAEIGENERMFYSFDHAHIQN
SNYIPQTQENSYNSNSSASDDTNYYFSYPNHVLENNINNCISNDFRMDENLFASSVPSLNEIVMEENVRMNEDSA
SDDHIVEKNGYNTQIMEPFDLHTKHEMQMKLKRKLDVIEVEVPVEEKINNNPKKKPRVSNDGQGCMKNARSKKNH
KVIASHEEEMTEEINRGSNGNSSSSNISEDDNASQENSGGTTLNSNGKTRASRGSATDPQSLYARKRRERINERL
RVLQNLVPNGTKVDISTMLEEAVNYVKFLQTQIKLLSSDDMWMYAPLAYNGLDLGLNLNLNSSLPL*
*Medicago* truncatula MtRSLa amino acid sequence (SEQ ID NO: 86; AC140548.11 GI:156231148) 
atggaacctataggtactttccctgaaggagaatggatttctttcgcaaatgtttgcaagtgaagatcatgaa
tattactcacaacaatttcttgatcaaaattcacttcttctaggggaaaatgatgggttgaacaatggaacacag
tccacatttgcactgctgaaattggtgaaaatgagcgtatgttttattcttttgatcatgctcatatccaaaac
tctaactatattcctcaaactcaagagaatagttacaatagcaattctagtgctagtgatgatacaaattactat
tttagttatcctaatcatgtactagaaaataatattaataattgtatatccaatgattttcgcatggatgagaat
ttgtttgcttcttctgttccatcccttaatgagattgtaatggaagagaatgtgagaatgaatgaagattctgca
agtgatgatcatattgtggagaaaaatggttacaatactcaaataatggaaccttttgatcttcacaccaagcat
gagatgcaaatgaagctcaaaggaaacttgatgtgatagaagtggaggttcccgttgaagaaaaattaacaac
aatccgaagaaaaaacctcgtgtttcgaatgatggccaaggatgcatgaaaaatgcaaggtcaaagaagaaccac
aaagttattgctagccatgaagaggagatgacagaagagattaatagaggatcaaatggaaatagttctagtagt
aacatttctgaggatgataatgcttctcaagaaaatagtggaggaactactctcaactcaaatgggaagacaaga
gctagtagaggatctgcaacagatccccaaagtctatatgcaaggaaaagaagagagagaataaatgaacgacta
agagtcttacaaaatcttgtaccaaacggaacaaaggttgatatcagtacaatgcttgaagaggcagtcaattat
 
gtgaaatttttacagactcaaatcaagcttttgagctctgatgatatgtggatgtatgcaccacttgcttacaat
ggacttgaccttggactcaatctcaacctcaacagctctctaccactatga
Medicago truncatula nucleotide sequence (SEQ ID NO:87, AC140548.11 GI:156231148) 
XFLCFSQGSSSSTDNSGNNIFSITSSGAYSCDPEANFDSVSMVLCLGDAKFSPHSFQCDDNSNQQINENTDEESS
LDPWKLAIADNNLQAKREYEMMVSEPVEVDRSRNLENLAKRLKSSIEVSKTLRSAKSGKNSKSASVSNDEDDRSL
SLQAQRNSCFSQSDSNAYLEPNGGASKDPAPPNLHRKSRATTGAATDPQSLYARKRRERINERLRILQNLVPNGT
KVDISTMLEEAVQYVKFLQLQIKLLSSDDLWMY
Soybean GmRSLa amino acid sequence (SEQ ID NO: 88)
   (gi|26056905|gb|CA799819.1|CA799819) 
ATTTTTTGTGTTTCTCACAAGGGAGTAGCTCCAGTACTGATAATAGTGGTAATAATATCTTTTCCATTACAAGTA
GTGGAGCCTACTCCTGTGATCCAGAAGCAAACTTTGATTCTGTGTCCATGGTTTTGTGCCTTGGAGATGCCAAAT
TTAGTCCCCATAGTTTTCAATGTGATGACAACTCAAACCAACAGATAAATGAAAACACTGATGAAGAGTCAAGTC
TAGACCCATGGAAGTTGGCTATAGCTGACAATAATTTGCAGGCTAAGAGGGAGTATGAAATGATGGTTTCTGAAC
CTGTAGAAGTGGATAGAAGCAGAAACCTGGAGAACCTAGCAAAAAGACTAAAGAGTTCAATAGAGGTTTCAAAAA
CATTGAGGAGTGCTAAATCAGGGAAAAATTCAAAATCTGCTTCAGTGAGCAACGATGAAGATGATAGAAGCTTGA
GCCTCCAAGCCCAAAGGAATAGCTGTTTTTCACAGAGTGACTCTAATGCTTATCTGGAGCCAAATGGAGGGGCAT
CAAAAGATCCTGCACCTCCCAATTTGCATAGAAAATCAAGAGCAACTACCGGTGCTGCCACTGATCCACAGAGCC
TCTATGCAAGAAAGAGAAGAGAAAGAATAAATGAAAGGTTGAGAATACTGCAAAATCTTGTTCCCAACGGAACTA
AGGTGGATATCAGCACCATGCTTGAGGAAGCTGTCCAATACGTGAAGTTTTTACAGCTCCAAATTAAGCTTCTGA
GCTCTGACGATCTGTGGATGTAT
Soybean GmRSLa nucleotide sequence (SEQ ID NO: 89)
   (gi|26056905|gb|CA799819.1|CA799819) 
XNLENLPKRLKSSIEVPKTSRNAKSRKNSKSASTSNDEDDRSLSLQVQRNNSCFSQSDSNAYLEPNGGASKDPAP
PNLDRKSRATTSAAADPQSLYARKRRERINERLRILQNLVPNGTKVDISTMLEEAVQYVKFLQLQIKLLSSEDLW
MYAPIVYNGINIGLDLGISPTKGRSM*
Soybean GmRSLb amino acid sequence (SEQ ID NO: 90)
   (gi|15663066|gb|BI700437.1|BI700437) 
GAAACCTGGAGAACCTACCAAAAGACTAAAGAGCTCAATAGAGGTCCCAAAAACATCGAGGAATGCTAAATCAA
GGAAAAATTCAAAATCTGCTTCAACTAGCAACGATGAAGATGATAGAAGCTTGAGCCTCCAAGTCCAAAGGAATA
ATAGCTGTTTTTCACAGAGTGACTCTAATGCTTATCTTGAGCCAAATGGAGGGCATCAAAAGATCCTGCACCTC
CTAATTTGGATAGAAAATCAAGAGCAACTACCAGTGCCGCCGCTGATCCACAGAGCCTCTATGCAAGAAAGAGAA
GAGAAAGAATAAATGAAAGGCTGAGAATACTGCAAAATCTTGTCCCCAACGGAACTAAGGTGGATATCAGCACCA
TGCTTGAAGAAGCTGTCCAATACGTTAAGTTTTTACAGCTCCAAATTAAGCTTCTGAGCTCTGAAGATTTGTGGA
TGTATGCTCCAATTGTTTACAATGGAATAAACATTGGACTAGACCTCGGTATTTCTCCAACCAAAGGAAGATCAA
TGTGATAGCATAGCAATTAAAGAGGATATAATATTTCATTAACTTA
Soybean GmRSLb nucleotide sequence (SEQ ID NO: 91).
   (gi|15663066|gb|BI700437.1|BI700437) 
XRSKEAEILSSNGKRKASRGSATDPQSVYARKRRERINERLRILQNLVPNGTKVDISTMLEEAVEYVKFLQLQIK
LLSSDDMWMYAPIAYDGMDIGLHSTTIPSSSTR*
Lettuce saligna LsRSLa amino acid sequence (SEQ ID NO: 92)
   (gi|83790803|gb|DW051020.1|DW051020 CLLX3812.b1_H18.ab1) 
TGAGATCAAAAGAGGCTGAAATTCTGAGCTCAAATGGCAAGAGAAAAGCAAGTAGGGGGTCAGCAACTGATCCAC
AAAGTGTCTATGCACGGAAAAGAAGAGAAAGAATTAACGAACGTTTAAGAATATTACAAAATCTTGTTCCTAATG
GTACAAAGGTTGATATAAGCACAATGCTTGAAGAGGCTGTTGAGTACGTGAAGTTTTTGCAGCTTCAAATCAAGC
TCTTGAGCTCCGATGATATGTGGATGTATGCTCCGATTGCATACGATGGAATGGACATTGGGCTTCATTCAACAA
 
CCATCCCATCATCGTCAACAAGATAATGCAAAGTTGGGCTATCCATATTGTCACATTTTTGTTGAATAAAAGGCA
ATCGATAACAAAATTCAAAGTTTATAAAGAGTACACATTTATGC
Lettuce saligna LsRSLa nucleotide sequence (SEQ ID NO:93)
   (gi|83790803|gb|DW051020.1|DW051020 CLLX3812.b1_H18.ab1) 
MASKRATTRELRAMYDDEPSSMSLELFGYHGVVVDGDDENDDTATALPQLSFVDNFKGGCGSAADYYSWAYNASG
GTPGASSSSTSSVLSFEHAGGAGHQLAYNSGTGDDDCALWMDSMADHQHGAARFGFMNPGSADVVPEIQESSIKQ
PAKSAQKRSSSGGEAQAAAKKQCGGGRKSKAKVVPTKDPQSAVAKVRRERISERLKVLQDLVPNGTKVDMVTMLE
KAITYVKFLQLQVKVLATDEFWPVQGGKAPELSQVKTALDAILSSQQQP*
Triticum aestivum TaRSLa amino acid sequence (SEQ ID NO: 94)
   (gi|25232820|gb|CA654295.1|CA654295) 
ATGGCGAGCAAGCGGGCCACCACGCGGGAGCTCCGGGCGATGTACGACGACGAGCCCTCCTCCATGTCCCTCGAG
CTCTTCGGCTACCATGGCGTGGTCGTCGACGGTGACGATGAAAACGACGACACTGCCACCGCCCTGCCCCAGCTC
TCCTTCGTCGACAACTTCAAAGGTGGGTGCGGGTCGGCGGCGGACTACTACAGCTGGGCGTACAACGCCTCCGGC
GGGACGCCGGGCGCCTCCTCCAGCTCCACCTCGTCGGTGCTCAGCTTTGAGCATGCCGGCGGTGCCGGTCATCAG
CTGGCTTATAATTCCGGCACAGGCGACGATGACTGCGCGCTCTGGATGGACAGCATGGCCGATCATCAGCACGGC
GCGGCCAGGTTTGGGTTCATGAACCCAGGGTCGGCCGATGTCGTCCCAGAAATCCAGGAGAGCAGCATCAAGCAG
CCGGCCAAGTCTGCGCAGAAGCGCTCGAGCTCGGGTGGTGAGGCGCAAGCAGCGGCGAAGAAGCAGTGTGGAGGA
GGCAGGAAGAGCAAGGCCAAAGTTGTCCCTACCAAGGATCCTCAGAGCGCTGTTGCAAAGGTCCGAAGAGAGCGC
ATCAGTGAGAGGCTCAAAGTTCTGCAGGATCTTGTACCCAACGGCACGAAGGTGGACATGGTCACCATGCTCGAG
AAGGCAATCACCTATGTCAAGTTCCTGCAGCTGCAAGTCAAGGTGTTGGCGACCGACGAGTTCTGGCCGGTGCAA
GGAGGGAAGGCGCCGGAGCTCTCCCAAGTGAAGACCGCGCTGGACGCCATCCTTTCTTCCCAGCAGCAACCCTAG
Triticum aestivum TaRSLa nucleotide sequence (SEQ ID NO:95)
   (gi|25232820|gb|CA654295.1|CA654295) 
DSQIIHPMPCDELHKSLI*LYHIRRRYPYWVFTDGESTSFARPLLNDSRIRGELLLTLSTTKHCKVTASSMRRSY
SMMHDHEKS*KIQRRKSQKLVSKGNESEADHDAVFGQIMKMCGSDNDSNWPRESSTSPRPKEAANLNSNGKTKAN
RGSATDPQSVYARKRRERINERLRILQSLVPNGTKVDISTMLEDAVQYVKFLQLQIKPLSSDDLWMYAPIAYNGM
ETGLDSTIPSPR*RLSKVAASFFLKKGKPGA
Safflower Carthamus tinctorius CtRSLa amino acid sequence (SEQ ID NO: 96)
   (gi|125399878|gb|EL411863.1|EL411863 CFFS9477.b1_I18.ab1) 
GATTCACAGATAATCCACCCTATGCCGTGTGATGAACTCCACAAATCCTTAATTTAATTGTACCACATCAGGCGA
CGTTATCCATATTGGGTGTTCACTGATGGTGAAAGCACATCTTTCGCGCGACCTCTACTCAATGACTCAAGAATT
AGAGGTGAACTATTGCTTACACTATCTACTACTAAACATTGTAAAGTGACTGCCAGTTCTATGAGACGTTCGTAT
AGCATGATGCATGATCATGAGAAAAGCTAAAAGATACAGCGCAGAAAGAGCCAGAAGCTCGTTTCTAAAGGCAAC
GAAAGTGAAGCTGACCATGATGCAGTTTTTGGGCAAATAATGAAAATGTGTGGATCTGACAATGACTCGAATTGG
CCTCGGGAGTCGAGCACAAGTCCAAGACCAAAAGAGGCTGCAAATCTGAACTCAAATGGGAAGACAAAAGCAAAT
AGGGGGTCAGCAACGGATCCACAAAGTGTCTACGCACGGAAGAGAAGAGAACGAATTAATGAACGGTTAAGAATA
CTACAGAGTCTGGTTCCTAATGGTACAAAGGTTGATATAAGCACAATGCTTGAAGATGCTGTCCAGTATGTGAAA
TTTTTGCAGCTCCAAATCAAGCCGTTGAGCTCTGATGATCTGTGGATGTATGCCCCCATCGCGTACAACGGGATG
GAGACGGGGCTTGATTCTACGATCCCCTCGCCAAGGTGAAGACTATCCAAAGTTGCCGCATCTTTTTCTTGAAA
AAAGGGAAGCCTGGGGCAA
Safflower Carthamus tinctorius CtRSLa nucleotide sequence (SEQ ID NO: 97)
   (gi|125399878|gb|EL411863.1|EL411863 CFFS9477.b1_I18.ab1) 
MALVREPMVLYDGGFDASEASAFDSIGCFGHGHGHDALLGGVDAAALFGGYAHDEPAGASASAYVKDGSHWAGVG
ASVLAFDRAARGHGAQAMATAAAQEEEECDAWIDAMDEDNGEAAPAPSIGFDPATGCFSLTQRPGAGARRPFGLL
FPSASGGAPSPDSAAPAPASRGSQKRPSAGIARAQDAEPRASKKQCGASRKTTAKAKSPAPAITSPKDPQSLAAK
NRREKISERLRTLQEMVPNGTKVDMVTMLEKAISYVKFLQLQVKVLATDEFWPAQGGMAPEISQVKEALDAILSS
QRGQFNCSS*
BdRSLa amino acid sequence (SEQ ID NO: 98) 
ATGGCATTAGTGCGGGAGCCGATGGTACTGTATGACGGCGGTTTCGACGCCTCGGAGGCGTCGGCATTCGACTCC
ATCGGCTGCTTCGGCCACGGCCACGGCCACGACGCGCTCCTAGGCGGCGTCGACGCGGCCGCGCTGTTCGGGGGC
TACGCGCACGACGAGCCGGCCGGCGCCAGCGCCAGCGCCTACGTGAAGGACGGCTCGCACTGGGCCGGCGTGGGT
GCGTCCGTGCTCGCGTTCGACCGTGCCGCTCGGGGCCACGGCGCGCAGGCCATGGCGACCGCGGCCGCTCAGGAG
GAGGAAGAATGCGACGCGTGGATCGACGCCATGGACGAGGACAATGGCGAGGCGGCGCCGGCGCCGTCCATCGGC
TTCGACCCGGCCACGGGCTGCTTCAGCCTCACGCAGCGGCCCGGCGCCGGCGCGCGGCGCCCGTTCGGGCTCCTG
TTCCCGAGCGCGTCCGGTGGCGCGCCCTCGCCCGACAGCGCCGCGCCAGCGCCGGCATCCCGCGGTTCCCAGAAG
CGGCCATCCGCCGGGATTGCGCGCGCGCAGGACGCGGAGCCGCGGGCCAGCAAGAAGCAGTGCGGCGCGAGCAGG
AAGACGACGGCCAAGGCGAAGTCGCCTGCGCCTGCCATCACCTCGCCCAAGGACCCGCAGAGCCTCGCTGCAAAG
AACCGGAGGGAGAAGATCAGCGAGCGGCTCCGGACGTTGCAGGAGATGGTGCCCAACGGCACCAAGGTGGACATG
GTCACCATGCTCGAGAAGGCCATCAGCTACGTCAAGTTCCTGCAGCTGCAAGTCAAGGTGCTCGCGACGGACGAG
TTCTGGCCGGCGCAGGGAGGGATGGCGCCGGAGATCTCCCAGGTGAAGGAGGCGCTCGACGCCATCCTGTCGTCG
CAGAGGGGGCAATTCAACTGCTCCAGCTAG
BdRSLa nucleotide sequence (SEQ ID NO: 99) 
MASRHATTREPHLRTMYDDEPSMSLELFGYHGVVVDGDDDGDTATDLPQLTFVDNFKGGCGSADYYGWAYSASGG
ASGACSSSSSSSVLSFEQAGGAGHQLAYNAGTGDDDCALWMDGMADQHDTAKFGFMDPGMSDVSLEIQESSMKPPA
KMAQKRACQGGETQAAAKKQCGGSKKSKAKAAPAKDPQSAVAKVRRERISERLKVLQDLVPNGTKVDMVTMLEKA
ITYVKFLQLQVKVLATDDFWPVQGGKAPELSQVKDALDAILSSQNQS*
BdRSLb amino acid sequence (SEQ ID NO: 100) 
ATGGCAAGCAGGCACGCCACTACACGGAGCCACACCTCCGGACCATGTACGACGACGAGCCATCCATGTCCCTC
GAGCTCTTCGGCTACCATGGCGTCGTCGTCGACGGTGACGACGATGGCGACACCGCCACCGACCTTCCCCAGCTC
ACCTTTGTTGACAACTTCAAAGGCGGGTGTGGGTCAGCCGACTACTACGGCTGGGCGTACAGCGCCTCCGGTGGT
GCGTCAGGCGCCTGCTCCAGCTCCAGCTCGTCGGTGCTCAGCTTTGAGCAGGCGGGTGGTGCCGGTCATCAGCTG
GCTTATAACGCCGGCACAGGTGACGATGACTGCGCGCTCTGGATGGACGGCATGGCTGACCAGCATGACACAGCC
AAGTTTGGGTTCATGGACCCAGGCATGTCTGATGTCAGCCTAGAAATCCAGGAGAGCAGCATGAAACCGCCGGCC
AAGATGGCACAGAAGCGCGCTTGCCAGGGTGGTGAGACGCAAGCAGCGGCGAAGAAGCAGTGTGGAGGAAGCAAG
AAGAGCAAGGCAAAAGCTGCCCCTGCCAAGGATCCTCAAAGCGCCGTTGCAAAGGTCCGAAGAGAGCGCATCAGC
GAGAGGCTCAAAGTTCTGCAGGATCTCGTGCCCAATGGCACAAAGGTTGACATGGTCACCATGCTCGAAAAGGCA
ATCACCTATGTCAAGTTCCTGCAGCTGCAAGTCAAGGTATTGGCGACTGATGACTTCTGGCCGGTGCAAGGAGGG
AAAGCTCCGGAGCTCTCCCAAGTGAAGGACGCTCTGGACGCGATCCTGTCTTCCCAGAATCAATCCTAG
BdRSLb nucleotide sequence (SEQ ID NO: 101) 
MALVGQATKLCYDGFAGDGVPPFMDAACLAFDHGYDYNNPHAWEFPTGAEPGNSSAFDVAWTGVSSTSPVLTFDA
AEWMDATATDRLSSYSPSAATVPASYKRPRAHVQPQQEAEEQESITPNPKKQCGDGKVVIKSSAAATGTSPRKEP
QSQAAKSRRERIGERLRALQELVPNGSKVDMVTMLDKAITYVKFMQLQLTVLETDAFWPAQGGAAPEISQVKAAL
DAIILSSSQKPRQWS*
BdRSLc amino acid sequence (SEQ ID NO: 102) 
ATGGCTCTAGTGGGTCAGGCAACGAAGCTCTGCTACGACGGCTTCGCCGGAGACGGTGTGCCGCCGTTCATGGAC
GCAGCTTGTCTGGCATTCGACCACGGGTATGATTACAACAATCCCCACGCATGGGAATTCCCCACCGGCGCCGAG
CCAGGCAACAGCAGCGCGTTCGACGTTGCCTGGACCGGCGTCTCCTCCACTTCTCCGGTGCTCACATTCGACGCC
GCCGAGTGGATGGACGCCACGGCCACGGACCGGCTGAGCTCCTACAGCCCGTCTGCGGCCACCGTGCCGGCCTCT
TACAAGCGGCCTCGTGCGCACGTGCAGCCACAGCAGGAAGCAGAAGAACAGGAAAGCATTACTCCCAATCCCAAG
AAGCAGTGCGGCGATGGGAAAGTAGTTATCAAGTCATCGGCGGCGGCTACCGGCACCAGTCCACGCAAGGAACCC
CAAAGCCAAGCTGCCAAGAGCCGTCGTGAGCGGATCGGCGAGCGGCTGAGAGCGCTGCAGGAGCTGGTGCCCAAC
GGCAGCAAGGTGGACATGGTCACCATGCTCGACAAGGCCATCACTTATGTCAAGTTCATGCAGCTCCAGCTCACG
GTGCTCGAGACAGACGCGTTCTGGCCTGCGCAGGGTGGCGCGGCGCCGGAGATCTCCCAGGTGAAGGCGGCGCTC
GACGCCATCATCCTCTCCTCGTCGCAGAAGCCTCGTCAGTGGAGCTAG
BdRSLc nucleotide sequence (SEQ ID NO: 103) 
MEAGGLISEAGWTMFDFPSQGEESEIMSQLLGAFPSHLEEGHQDLPWYQASDPSYYDCNLNTSSESNASSLAVPS
ECMGYYLGDSSESLDLSSCIAPNDLNLVQEQDATEFLNMTPNLSLDLRGNGESSCEDLTSVGPTNKRKHSSAEEG
IDCQARGQKFARKAEPKRTKKTKQSGWEVAVATRNGSTASCCTSDDDSNASQESADTGVCPKGKARAARGASTDP
QSLYARKRRERINERLKTLQTLVPNGTKVDMSTMLEEAVHYVKFLQLQIKVLSSDDMWMYAPLAYNGMNIGLDLN
IYTPERWRTASAAPSTEGREYAGVDRISDLPDGILGDIVSLLPTAEGARTQILKRRWRHIWRCSAPLNLDCCTLV
ARGGGREAEDELVGLIPSILSSHQGTGRRFHVPSSRHSDRAATIEAWLQSAALDNLQELDLWCTHTYLYDYVPLP
PAVFRFSATVRVVTIANCNLRDSAVQGLQFPQLKQLGFKDIIIMEDSLHHMIAACPDLECLMIERSLGFACVRIN
SLSLRSIGVSTDHPHPHELQFVELVIDNAPCLKRLLHLEMCYHLDMHITVISAPKLETLSCCSSVSRSSTKLSFG
SAAIQGLHIDSLTTVVRTVQILAVEMHSLCLDTIIDFMKCFPCLQKLYIKSFVSGNNWWQRKHRNVIKSLDIRLK
TIALESYGGNQSDINFVTFFVLNARVLELMTFDVCSEHYTVEFLAEQYRKLQLDKRASRAARFHFTSNRCVRGIP
YIGRAELFLPIKCSHVDTSPNLSSFRLSAVFSVCITRNLLRLKKAMWVISLYYSPEFTKQVAVHNPNEMPF*
BdRSLd amino acid sequence (SEQ ID NO: 104) 
ATGGAGGCTGGAGGGCTGATTTCTGAGGCTGGCTGGACCATGTTTGACTTCCCGTCGCAAGGCGAGGAATCAGAG
ATCATGTCGCAGCTGCTAGGCGCCTTCCCCTCCCATCTTGAGGAAGGCCATCAGGATCTGCCTTGGTACCAGGCT
TCTGACCCATCCTACTATGACTGTAATCTTAATACAAGTAGTGAAAGCAATGCTAGTAGTCTTGCTGTTCCATCC
GAGTGTATGGGCTACTATTTGGGTGATTCAAGTGAGTCCCTGGACCTGAGCTCCTGCATTGCACCAAATGACCTG
AACTTGGTCCAGGAGCAAGATGCAACTGAGTTTCTGAATATGACACCAAATCTTTCCCTTGATTTACGTGGAAT
GGTGAGTCGAGCTGCGAGGATCTCACTTCGGTCGGTCCTACTAACAAGCGAAAGCACTCCTCGGCAGAAGAAGGA
ATCGACTGCCAAGCAAGAGGCCAGAAATTCGCCAGAAAGGCTGAACCGAAGCGAACAAAGAAGACCAAGCAAAGC
GGATGGGAGGTTGCTGTTGCCACCAGGAATGGAAGCACAGCGAGCTGCTGCACCTCTGATGATGACTCAAACGCT
TCTCAAGAATCTGCAGATACCGGTGTTTGTCCGAAAGGCAAGGCTCGGGCTGCCCGTGGCGCATCAACTGATCCC
CAGAGCCTCTATGCAAGGAAAAGGAGGGAAAGGATCAATGAGAGACTGAAGACACTGCAGACCCTTGTGCCCAAT
GGAACCAAAGTAGATATGAGCACCATGCTTGAGGAGGCAGTCCACTACGTGAAGTTCCTGCAGCTTCAGATCAAG
GTCTTGAGCTCTGATGATATGTGGATGTATGCGCCGCTAGCATACAACGGGATGAACATTGGGCTTGATCTGAAC
ATATATACTCCGGAGAGGTGGAGGACAGCGTCCGCGGCGCCCTCAACCGAAGGGCGTGAATACGCCGGCGTCGAC
CGCATCAGCGACCTCCCCGACGGCATCCTCGGCGACATCGTCTCGTTGCTCCCCACCGCCGAAGGAGCCGCACC
CAGATCCTCAAGCGCAGGTGGCGCCACATCTGGCGCTGCTCCGCCCCTCTCAACCTCGATTGCTGTACCTTGGTC
GCCCGTGGCGGCGGCCGTGAGGCTGAAGATGAACTCGTCGGTCTCATACCGTCCATCCTTTCTTCTCACCAAGGC
ACCGGCCGCCGCTTCCACGTCCCCTCGTCGCGCCACTCTGACCGAGCTGCTACCATTGAAGCCTGGCTCCAATCT
GCTGCCCTCGACAATCTCCAGGAGCTCGATTTATGGTGCACCCACACCTATCTTTACGACTATGTTCCGCTGCCA
CCCGCCGTCTTTCGCTTCTCCGCCACCGTCCGTGTTGTCACCATCGCAAATTGTAACCTCCGTGACAGCGCCGTC
CAAGGCCTTCAATTCCCACAACTTAAACAGCTCGGATTCAAAGATATCATCATCATGGAGGATTCGCTGCACCAC
ATGATTGCTGCGTGTCCAGATCTCGAGTGCTTGATGATTGAAAGGAGCTTAGGTTTTGCTTGCGTCCGGATCAAT
TCCCTTAGTCTTAGAAGCATCGGTGTGAGCACTGACCACCCTCACCCACATGAGCTCCAGTTTGTGGAACTCGTC
ATTGATAATGCACCTTGTCTTAAGAGATTGCTCCATCTTGAAATGTGTTATCACCTTGACATGCATATAACAGTA
ATCTCCGCGCCTAAACTGGAGACCTTGAGCTGCTGTTCTTCTGTGAGTCGCTCCTCCACCAAACTCTCGTTTGGC
TCCGCGGCCATTCAGGGATTGCACATTGATAGCCTAACAACAGTGGTGCGCACTGTCCAAATTTTAGCTGTAGAG
ATGCATTCTCTTTGTCTAGACACAATTATTGACTTCATGAAATGCTTTCCATGTCTGCAGAAGTTGTACATTAAG
TCATTTGTAAGTGGAAACAATTGGTGGCAACGTAAACACCGGAACGTTATCAAATCCCTTGACATCCGTCTCAAG
ACAATAGCGTTGGAAAGTTATGGGGGCAATCAGTCTGACATCAACTTTGTCACATTCTTTGTCTTGAACGCGAGA
GTGCTAGAGTTGATGACATTTGACGTTTGTTCTGAGCATTACACTGTGGAGTTCTTGGCAGAGCAATATAGGAAG
CTTCAGCTAGATAAGAGGGCTTCAAGAGCCGCTCGGTTCCATTTTACAAGTAACCGATGTGTCCGTGGTATTCCG
TATATCGGACGTGCCGAGCTATTCTTGCCTATCAAATGTTCTCATGTTGACACCAGTCCAAACTTGAGTAGTTTC
CGTTTGTCTGCAGTATTTTCAGTTTGTATTACCCGGAACCTTTTGCGTTTAAAAAAAGCTATGTGGGTCATTAGT
TTGTATTATTCTCCAGAATTTACAAAACAAGTGGCCGTGCACAATCCCAATGAAATGCCGTTTTAG
BdRSLd nucleotide sequence (SEQ ID NO: 105) 
MEAKCGAIWSSIDARSEDSEMIAHLQSMFWSNSDVALNLCSSNTSGNSCVTASTLPSSLFLPLVDNESYGAAPSV
DTGMDSCFDHQHQSITGHKRISHMDEQMKKTRKKSRTVPSVSKALGSSLVDNQMNADIFNQSSSCCSSGEDSIGT
SEKSIVANQSDNTSGCKRPSKNMQSLYAKKRRERINEKLRVLQQLIPNGTKVDISTMLEEAVQYVKFLQLQIKVL
SSDETWMYAPLAYNGMDIGLTLALRTAANQE*
BdRSLe amino acid sequence (SEQ ID NO: 106) 
ATGGAGGCCAAGTGTGGAGCTATTTGGAGCTCTATCGATGCGAGGAGCGAGGACTCTGAGATGATTGCTCACCTG
CAGTCCATGTTCTGGAGCAACAGTGATGTTGCTCTCAACCTCTGTTCGTCAAACACCAGTGGCAATTCTTGTGTC
ACAGCTAGCACATTGCCTAGCAGCTTGTTCCTTCCTCTTGTCGATAATGAGAGCTATGGTGCAGCGCCATCGGTG
GACACCGGCATGGATTCATGCTTTGATCACCAGCATCAGAGCATTACTGGTCACAAGAGGATATCGCACATGGAT
GAGCAGATGAAGAAGACGAGAAAGAAGTCCCGGACTGTTCCATCGGTATCAAAGGCTCTGGGTTCCAGCCTAGTC
GATAATCAGATGAATGCTGACATTTTCAATCAGAGCTCCTCCTGCTGCAGCTCGGGAGAAGATTCAATTGGAACA
TCTGAGAAATCCATTGTTGCAAACCAGAGTGACAATACGAGTGGTTGTAAGCGGCCTTCAAAGAATATGCAAAGC
CTTTATGCAAAGAAGAGAAGAGAGAGGATCAACGAGAAGTTGAGAGTACTGCAGCAGCTGATTCCCAATGGCACC
AAAGTTGACATCAGCACAATGTTGGAGGAAGCAGTTCAGTATGTCAAGTTTCTGCAGCTGCAAATAAAGGTCTTA
AGCTCTGACGAGACATGGATGTATGCGCCCCTCGCCTACAATGGTATGGACATCGGTCTCACTCTCGCTCTGAGA
ACTGCTGCAAACCAAGAGTGA
BdRSLe nucleotide sequence (SEQ ID NO: 107) 
MALVREHGGYYGGFDSVEAAAFDTLGYGHGASLGFDASSALFGEGGYAAGGGDAWAGAGASTVLAFNRTTAAAAV
GVEEEEEECDAWIDAMDEDDQSSGPAAAAPEARHALTASVGFDASTGCFTLTERASSSSGGAGRPFGLLFPSTSS
SGGTPERTAPVRVPQKRTYQAVSPNKKHCGAGRKASKAKLASTAPTKDPQSLAAKQNRRERISERLRALQELVPN
GTKVDLVTMLEKAISYVKFLQLQVKVLATDEFWPAQGGKAPEISQVREALDAILSSAS
Zea mays ZmRSLa amino acid sequence (AZM4_60871: SEQ ID NO: 108) 
ATGGCGTTGGTGAGGGAGCACGGTGGGTACTACGGAGGCTTCGACAGCGTCGAGGCGGCGGCCTTCGACACGCTC
GGCTACGGCCACGGCGCGTCGCTGGGCTTTGACGCGTCGTCGGCGCTGTTCGGGGAAGGCGGTTATGCGGCGGGC
GGCGGGGACGCCTGGGCGGCGCGGGGGCGTCGACCGTCCTGGCGTTCAACCGCACAACGGCAGCGGCGGCCGTG
GGTGTGGAAGAGGAGGAGGAGGAGTGCGACGCGTGGATCGACGCTATGGACGAGGACGACCAGAGCTCCGGCCCC
GCCGCGGCGGCGCCAGAGGCGCGCCACGCGCTGACGGCCTCCGTGGGTTTCGACGCCTCCACGGGGTGCTTCACC
CTGACGGAGAGGGCGTCGTCGTCGTCAGGCGGAGCGGGGCGCCCGTTCGGCCTGCTGTTCCCGAGCACGTCGTCG
TCGGGCGGCACGCCCGAGCGCACGGCGCCGGTGCGCGTCCCGCAGAAACGGACCTACCAGGCTGTGAGCCCCAAC
AAGAAGCACTGCGGCGCGGGCAGGAAGGCGAGCAAGGCCAAGCTCGCGTCCACAGCCCCAACCAAAGATCCCCAG
AGCCTCGCGGCCAAGCAGAACCGGCGCGAGCGGATCAGCGAGCGGCTGCGGGCGCTGCAGGAGCTGGTGCCCAAC
GGCACCAAGGTCGACCTGGTCACCATGCTCGAGAAGGCCATCAGCTACGTTAAGTTCCTCCAGTTGCAAGTCAAG
GTTCTGGCAACAGACGAATTCTGGCCGGCACAGGGAGGGAAGGCGCCGGAGATCTCCCAGGTGAGGGAGGCGCTC
GACGCCATCTTGTCGTCGGCGTCG
Zea mays ZmRSLa nucleotide sequence (AZM4_60871: SEQ ID NO: 109) 
MAQFLGAADDHCFTYEYEHVDESMEAIAALFLPTLDTDSANFSSSCFNYAVPPQCWPQPDHSSSVTSLLDPAENF
EFPVRDPLPPSGFDPHCAVAYLTEDSSPLHGKRSSVIEEEAANAAPAAKKRKAGAAMQGSKKSRKASKKDNIGDA
DDDGGYACVDTQSSSSCTSEDGNFEGNTNSSSKKTCARASRGAATEPQSLYARKRRERINERLRILQNLVPNGTK
VDISTMLEEAAQYVKFLQLQIKLLSCDDTWMYAPIAYNGINIGNVDLNIYSLQK*
Zea mays ZmRSLb amino acid sequence (AZM4_70092: SEQ ID NO: 110) 
ATGGCTCAGTTTCTTGGGGCGGCTGATGATCACTGCTTCACCTACGAGTATGAGCATGTGGATGAGTCCATGGAA
GCAATAGCAGCCCTGTTCTTGCCTACCCTTGACACCGACTCCGCCAACTTCTCCTCTAGCTGTTTCAACTATGCT
GTCCCTCCACAGTGCTGGCCTCAGCCAGACCATAGCTCTAGCGTTACCAGTTTGCTTGATCCAGCCGAGAACTTT
GAGTTTCCAGTCAGGGACCCGCTCCCCCCAAGCGGCTTCGATCCACATTGCGCTGTCGCCTACCTCACTGAGGAT
TCGAGCCCTCTGCATGGCAAACGTTCATCAGTCATTGAGGAAGAAGCAGCCAACGCCGCACCTGCTGCTAAGAAG
AGGAAGGCTGGTGCTGCAATGCAGGGATCAAAGAAATCCAGGAAGGCGAGCAAAAAGGATAACATCGGCGACGCC
GACGATGATGGCGGCTATGCCTGTGTTGACACGCAAAGCTCCAGTAGCTGCACCTCCGAGGACGGAACTTCGAA
GGAAATACGAATTCAAGCTCCAAGAAGACCTGCGCCAGGGCCAGCCGCGGAGCAGCAACTGAACCTCAGAGTCTC
TATGCAAGGAAGAGGAGAGAGAGGATCAACGAAAGGTTGAGAATCTTGCAGAACTTGGTTCCAAATGGAACAAAA
GTAGACATTAGCACGATGCTCGAGGAAGCGGCGCAGTATGTCAAGTTTTTACAGCTCCAGATTAAGCTGTTGAGC
TGTGACGACACATGGATGTATGCGCCAATCGCGTACAATGGAATTAACATCGGCAATGTTGATCTGAACATCTAC
TCTCTGCAAAAGTAA
Zea mays ZmRSLb nucleotide sequence (AZM4_70092: SEQ ID NO: 111) 
MEDGGLXSEAGAWAELGTGGDESEELVAQLLGAFFRSHGEEGRHQLLWSDDQASSDDVHGDGSLAVPLAYDGCCG
YLSYSGSNSDELPLGSSSRAAPAGGPPEELLGAAETEYLNNVAAADHPFFKWCGNGEGLDGPTSVVGTLGLGSGR
KRARKKSGDEDEDPSTAIASGSGPTSCCTTSDSDSNASPLESADAGARRPKGNENARAAGRGAAAATTTTAEPQS
IYARVRRERINERLKVLQSLVPNGTKVDMSTMLEEAVHYVKFLQLQIRVLQLLSSDDTWMYAPIAYNGMGIGIDL
RMHGQDR*
Zea mays ZmRSLc amino acid sequence (AZM4_91750: SEQ ID NO: 112) 
ATGGAGGACGGAGGGTTGRTCAGCGAGGCCGGCGCCTGGGCCGAGCTCGGCACCGGCGGCGACGAGTCGGAGGAG
CTGGTGGCGCAGCTGCTGGGCGCCTTCTTCCGGTCCCACGGCGAGGAAGGCCGGCACCAGCTGCTTTGGTCTGAC
GACCAAGCTTCTTCCGACGACGTGCACGGCGACGGCAGCCTTGCCGTGCCGCTCGCATACGACGGCTGCTGCGGC
TATCTGAGCTACTCAGGTAGCAACTCGGACGAGCTCCCCCTCGGGAGCAGCTCCCGCGCTGCGCCAGCAGGTGGC
CCACCGGAGGAGCTGCTCGGTGCAGCTGAGACTGAGTACCTGAATAATGTGGCCGCCGCAGACCATCCCTTCTTC
AAATGGTGTGGGAATGGTGAGGGTCTGGATGGTCCGACGAGCGTCGTGGGCACGCTTGGGCTTGGCTCGGGCCGG
AAACGCGCGCGCAAGAAGAGCGGGGACGAAGACGAAGACCCGAGCACGGCCATCGCCAGCGGAAGCGGCCCCACG
AGCTGCTGCACTACCTCCGACAGCGACTCAAACGCGTCTCCTCTGGAGTCCGCGGACGCCGGCGCTCGTCGCCCC
AAGGGCAACGAGAATGCCCGGGCAGCTGGCCGCGGCGCGGCGGCGGCGACGACGACGACAGCGGAGCCCCAGAGC
ATCTACGCAAGGGTACGGAGGGAGCGGATCAACGAGAGGCTCAAGGTGCTGCAGAGCCTGGTGCCCAACGGCACC
AAGGTGGACATGAGCACCATGCTCGAGGAGGCCGTCCACTACGTCAAGTTCCTGCAGCTTCAGATCAGGGTGCTG
CAGCTCCTGAGCTCCGACGACACGTGGATGTACGCGCCCATCGCGTACAACGGGATGGGCATCGGGATCGACCTC
CGCATGCATGGACAGGACAGATGA
Zea mays ZmRSLc nucleotide sequence (AZM4_91750: SEQ ID NO: 113) 
SKKSRKASKKDCIVDDDDVYVDPQSSGSCTSEEGNFEGNTYSSAKKTCTRASRGGATDPQSLYARKRRERINERL
RILQNLVPNGTKVDISTMLEEAAQYVKFLQLQIKLLSSDDMWMYAPIAYNGINISNVDLNIPALQK*
Zea mays amino acid sequence (AZM4_86104: SEQ ID NO: 114) 
TCAAAGAAATCCAGGAAGGCGAGCAAAAAAGATTGTATTGTCGATGACGACGATGTCTATGTTGACCCGCAAAGC
TCCGGTAGCTGCACCTCCGAGGAGGGGAATTTTGAAGGGAATACGTATTCAAGCGCGAAAAAGACCTGCACCAGG
GCCAGCCGCGGAGGAGCAACTGATCCTCAGAGTCTCTATGCAAGGAAGAGGAGAGAGAGGATCAATGAAAGGTTG
AGAATCTTGCAGAACTTGGTCCCCAATGGAACAAAGGTTGACATTAGTACGATGCTCGAGGAAGCAGCACAGTAT
GTCAAATTTTTACAGCTTCAGATTAAGCTGTTGAGCTCTGACGACATGTGGATGTATGCGCCAATCGCGTACAAT
GGGATCAACATCAGCAATGTTGATCTGAACATCCCTGCA
Zea mays ZmRSLd nucleotide sequence (AZM4_86104: SEQ ID NO: 115)

## Claims

1. A method for increasing the length and/or longevity of root hairs and/or the number of root hairs in a plant comprising:
increasing expression of an RHD6-related polypeptide by incorporating a heterologous nucleic acid which encodes a RHD6-related polypeptide comprising an amino acid sequence having at least 90% sequence identity to SEQ ID NO: 1
into a plant cell by means of transformation, expressing said heterologous nucleic acid within cells of said plant and
regenerating the plant from one or more transformed cells wherein the length and/or longevity of root hairs and/or the number of root hairs is increased relative to control plants in which expression of the RHD6-related polypeptide is not increased.

2. A method according to claim 1 wherein said RHD6-related polypeptide comprises SEQ ID NO: 1.

3. A method according to claim 1 wherein the plant has increased tolerance to nutrient-deficient conditions relative to the control plants.

4. A method according to claim 3 wherein the nutrient-deficient conditions are selected from the group consisting of nitrate-deficient, phosphate-deficient or iron-deficient conditions.

5. A method according to claim 1 wherein the plant has increased production or secretion of a root-secreted phytochemical relative to the control plants.

6. A method according to claims 1-5 wherein the heterologous nucleic acid comprises a nucleotide sequence which has at least 90% sequence identity with any one of SEQ ID NO: 2.

7. A method according to any one of claims 1 to 6 wherein the heterologous nucleic acid is operably linked to a promoter.

8. A method according to claim 7 wherein the promoter is a constitutive promoter.

9. A method according to claim 7 wherein the promoter is a root-specific promoter.

10. A method according to claim 7 wherein the promoter is an inducible promoter.

11. A method according to any one of claims 1 to 10 wherein the heterologous nucleic acid is comprised in a vector.

12. A method according to a preceding claim wherein the plant is an agricultural plant selected from the group consisting of *Lithospermum erythrorhizon, Taxus* spp, tobacco, cucurbits, carrot, vegetable brassica, melons, capsicums, grape vines, lettuce, strawberry, oilseed brassica, sugar beet, wheat, barley, maize, rice, soyabeans, peas, sorghum, sunflower, tomato, potato, pepper, chrysanthemum, carnation, linseed, hemp and rye.

## Patentansprüche

1. Verfahren zum Vergrößern der Länge und/oder Langlebigkeit von Wurzelhaaren und/oder der Anzahl von Wurzelhaaren in einer Pflanze, umfassend:
Erhöhen der Expression eines mit RHD6 verwandten Polypeptids durch Einbau einer heterologen Nucleinsäure, die für ein mit RHD6 verwandtes Polypeptid codiert, das eine Aminosäuresequenz mit einer Sequenzidentität von mindestens 90% zu SEQ ID Nr.: 1 umfasst, mittels Transformation in eine Pflanzezelle, Exprimieren der heterologen Nucleinsäure innerhalb von Zellen der Pflanze und Regenerieren der Pflanze aus einer oder mehreren transformierten Zellen, wobei die Länge und/oder Langlebigkeit von Wurzelhaaren und/oder die Anzahl von Wurzelhaaren gegenüber Kontrollpflanzen erhöht wird, in denen die Expression des mit RHD6 verwandten Polypeptids nicht erhöht ist.

2. Verfahren nach Anspruch 1, wobei das mit RHD6 verwandte Polypeptid SEQ ID Nr.: 1 umfasst.

3. Verfahren nach Anspruch 1, wobei die Pflanze bezüglich der Kontrollpflanzen erhöhte Toleranz gegenüber nährstoffarmen Bedingungen aufweist.

4. Verfahren nach Anspruch 3, wobei die nährstoffarmen Bedingungen aus der Gruppe ausgewählt sind, die aus nitratarmen, phosphatarmen oder eisenarmen Bedingungen besteht.

5. Verfahren nach Anspruch 1, wobei die Pflanze bezüglich der Kontrollpflanzen eine erhöhte Produktion oder Sekretion einer wurzel-sezernierten Phytochemikalie aufweist.

6. Verfahren nach einem der Ansprüche 1-5, wobei die heterologe Nucleinsäure eine Nucleotidsequenz aufweist, die eine Sequenzidentität von mindestens 90% mit einer der Sequenzen SEQ ID Nr.: 2 umfasst.

7. Verfahren nach einem der Ansprüche 1-6, wobei die heterologe Nucleinsäure funktionsfähig mit einem Promotor verbunden ist.

8. Verfahren nach Anspruch 7, wobei der Promotor ein konstitutiver Promotor ist.

9. Verfahren nach Anspruch 7, wobei der Promotor ein wurzelspezifischer Promotor ist.

10. Verfahren nach Anspruch 7, wobei der Promotor ein induzierbarer Promotor ist.

11. Verfahren nach einem der Ansprüche 1 bis 10, wobei die heterologe Nucleinsäure in einem Vektor enthalten ist.

12. Verfahren nach einem der vorstehenden Ansprüche, wobei die Pflanze eine landwirtschaftliche Pflanze ist, die aus der Gruppe ausgewählt ist, die aus Lithospermum erythrorhizon, Taxus spp, Tabak, Kürbisgewächsen, Karotte, Gemüsekohl, Melonen, Paprika, Weinreben, Kopfsalat, Erdbeere, Raps, Zuckerrüben, Weizen, Gerste, Mais, Reis, Sojabohnen, Erbsen, Sorghum, Sonnenblume, Tomate, Kartoffel, Pfeffer, Chrysantheme, Gartennelke, Leinsaat, Hanf und Roggen besteht.

## Revendications

1. Procédé pour l'augmentation de la longueur et/ou de la longévité de poils racinaires et/ou du nombre de poils racinaires dans une plante comprenant:
l'augmentation de l'expression d'un polypeptide apparenté à RHD6 par l'incorporation d'un acide nucléique hétérologue qui code pour un polypeptide apparenté à RHD6 comprenant une séquence d'acides aminés possédant au moins 90% d'identité de séquence avec la SEQ ID NO:1 dans une cellule de plante au moyen d'une transformation, l'expression dudit acide nucléique hétérologue dans des cellules de ladite plante et la régénération de la plante à partir d'une ou de plusieurs cellules transformées, où la longueur et/ou la longévité de poils racinaires et/ou le nombre de poils racinaires est augmenté relativement à des plantes témoins dans lesquelles l'expression du polypeptide apparenté à RHD6 n'est pas augmentée.

2. Procédé selon la revendication 1, dans lequel ledit polypeptide apparenté à RHD6 comprend la SEQ ID NO:1.

3. Procédé selon la revendication 1, dans lequel la plante a une tolérance accrue à des conditions de carence nutritive relativement aux plantes témoins.

4. Procédé selon la revendication 3, dans lequel les conditions de carence nutritive sont choisies dans le groupe constitué de conditions de carence en nitrate, de carence en phosphate ou de carence en fer.

5. Procédé selon la revendication 1, dans lequel la plante a une production ou une sécrétion accrue d'un composé phytochimique sécrété par les racines relativement aux plantes témoins.

6. Procédé selon les revendications 1-5, dans lequel l'acide nucléique hétérologue comprend une séquence de nucléotides qui possède au moins 90% d'identité de séquence avec l'une quelconque de la SEQ ID NO:2.

7. Procédé selon l'une quelconque des revendications 1 à 6, dans lequel l'acide nucléique hétérologue est lié de manière fonctionnelle à un promoteur.

8. Procédé selon la revendication 7, dans lequel le promoteur est un promoteur constitutif.

9. Procédé selon la revendication 7, dans lequel le promoteur est un promoteur spécifique aux racines.

10. Procédé selon la revendication 7, dans lequel le promoteur est un promoteur inductible.

11. Procédé selon l'une quelconque des revendications 1 à 10, dans lequel l'acide nucléique hétérologue est compris dans un vecteur.

12. Procédé selon une revendication précédente, dans lequel la plante est une plante agricole choisie dans le groupe constitué de *Lithospermum erythrorhizon, Taxus* spp., tabac, cucurbitacées, carotte, légume brassica, melons, piments secs, vignes, laitue, fraise, espèce brassica oléagineuse, betterave à sucre, blé, orge, maïs, riz, soja, pois, sorgho, tournesol, tomate, pomme de terre, poivre, chrysanthème, oeillet, lin, chanvre et seigle.
